# EUROPEAN PATENT APPLICATION

(11) **EP 3 904 337 A1**
(43) Date of publication of application: **03.11.2021**
(21) Application number: 19905826.4
(22) Date of filing: 26.12.2019
(51) Int. Cl.: C07C 309/04, C07C 309/05, C07C 309/06, C07C 309/29, C07F 3/02, H01M 6/16, H01M 10/054, H01M 10/0567, H01M 10/0568, H01M 10/0569

(54) **SULFUR-BASED ELECTROLYTE SOLUTION FOR MAGNESIUM CELL**

(30) Priority: 27.12.2018 JP 2018246327
(71) Applicant: FUJIFILM Wako Pure Chemical Corporation, Osaka 540-8605 (JP)
(72) Inventor: MORI Goro, Tokyo 103-0023 (JP); SATO Kazuhiko, Kawagoe-shi, Saitama 350-1101 (JP); INOMATA Satoru, Kawagoe-shi, Saitama 350-1101 (JP); MIZUTA Hironori, Kawagoe-shi, Saitama 350-1101 (JP); OKAMOTO Kuniaki, Kawagoe-shi, Saitama 350-1101 (JP)
(74) Representative: Patentanwaltskanzlei Hinkelmann
(86) International application number: PCT/JP2019/051330
(87) International publication number: WO 2020/138377

(57) **Abstract**

An object of the present invention is to provide a highly practical electrolyte solution which has high oxidation resistance and enables dissolution-precipitation of magnesium to proceed repeatedly and stably.

The present invention relates to an electrolyte solution for a magnesium battery comprising a mixture of a compound represented by the general formula [1], a Lewis acid or a compound represented by the general formula [4], and a solvent; an electrochemical device containing the electrolyte solution; and a compound represented by the general formula [1]. 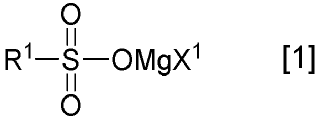 [In the general formula [1], X¹ represents a halogeno group, and R¹ represents an alkyl group having 1 to 10 carbon atoms, which may have a group represented by - SO₃MgX² (X² represents a halogeno group); a haloalkyl group having 1 to 10 carbon atoms, which may have a group represented by -SO₃MgX² (X² is the same as described above); an aryl group having 6 to 14 carbon atoms, which may have an alkyl group having 1 to 6 carbon atoms, an alkoxy group having 1 to 6 carbon atoms, a halogeno group, or a group represented by -SO₃MgX² (X² is the same as described above); or a biphenyl group which may have a group represented by -SO₃MgX² (X² is the same as described above).]

Mg [N(SO₂R⁴)₂]₂ [4]

(In the general formula [4], four R⁴'s each independently represent an alkyl group having 1 to 6 carbon atoms, a perfluoroalkyl group having 1 to 6 carbon atoms, a phenyl group, or a perfluorophenyl group.)

## Description

### TECHNICAL FIELD

The present invention relates to an electrolyte solution containing magnesium and an electrochemical device containing the electrolyte solution.

### BACKGROUND ART

Magnesium ions are polyvalent ions, and accordingly, the electric capacity of magnesium per unit volume is high. In addition, magnesium is safer as compared to lithium due to its high melting point, and in addition, resources thereof are relatively evenly distributed on earth and magnesium is inexpensive since the resources are abundant on earth. Therefore, a magnesium battery adopting metallic magnesium as a negative electrode has been paid attention as a next-generation battery replacing a lithium ion battery.

However, in a magnesium battery using metallic magnesium as a negative electrode, magnesium is reacted with an electrolyte solution due to its high reducing property to form a passivating surface film on the electrode. As a result, the reversible dissolution-precipitation of magnesium is inhibited, and thus, the negative electrode reaction is difficult to occur.

As an electrolyte solution that does not form such a passivation film, an electrolyte solution dissolved a Grignard reagent RMgX (R represents an alkyl group or an aryl group, and X represents chlorine or bromine) in tetrahydrofuran (THF) is known, and the reversible dissolution-precipitation of magnesium has been confirmed. However, since the Grignard reagent RMgX is strongly basic, it has a problem in safety, and further, the Grignard reagent also has a problem of being impractical due to its low oxidation resistance.

Therefore, an electrolyte solution improved safety and performance has been developed by mixing a strongly basic Grignard reagent or an organic magnesium reagent with a Lewis acid having aluminum. For example, a THF solution of Mg(AlCl₂BuEt)₂ in which dibutylmagnesium (Bu₂Mg) and ethylaluminum dichloride (EtAlCl₂) are mixed has been reported by Aurbach, et al. (Non-Patent Literature 1). However, the electrolyte solution containing a nucleophilic magnesium salt has had a problem that it was sensitive to air and water, and was not practical.

As an electrolyte solution that has solved such a problem, for example, an electrolyte solution in which a non-nucleophilic thiolate-based magnesium salt and aluminum chloride are mixed has been reported by Wang et al. (Non-Patent Literature 2).

It should be noted that the present inventors have reported an electrolyte solution for a magnesium battery comprising a mixture of a magnesium salt having a siloxy group and a Lewis acid in a solvent (Patent Literature 1); an electrolyte solution for a magnesium battery comprising a mixture of a magnesium salt of boronic acid, a Lewis acid, and the like in a solvent (Patent Literature 2); an electrolyte solution for a magnesium battery comprising a mixture of a magnesium salt having a phosphinyl group, a Lewis acid, and the like in a solvent (Patent Literature 3); and the like.

### CITATION LIST

### PATENT LITERATURE

Patent Literature 1: International Publication WO2016/084924
Patent Literature 2: International Publication WO2017/170976
Patent Literature 3: International Publication WO2017/204322

### NON-PATENT LITERATURE

Non-Patent Literature 1: Nature, 407, p. 724-727 (2000)
Non-Patent Literature 2: Electrochimica Acta 121, p. 258-263 (2014)

### SUMMARY OF INVENTION

### TECHNICAL PROBLEM

The electrolyte solution reported in Non-Patent Literature 2 exhibits reversible dissolution-precipitation behavior of magnesium, but has only oxidation resistance of about 2.5 V, and thus, the oxidation resistance of the electrolyte solution was insufficient to put a magnesium battery into practical use as a substitute for a lithium ion battery.

The present invention has been made in view of such a situation, and has an object to provide an electrolyte solution having high practicality, which has high oxidation resistance, enables dissolution-precipitation of magnesium to proceed repeatedly and stably, and is easy to prepare. As a result of repeated diligent studies, the present inventors have found that an electrolyte solution comprising a mixture of a sulfonic acid-based magnesium salt having a higher degree of oxidation than a thiolate-based magnesium salt, a Lewis acid, and the like in a solvent has excellent oxidative stability (also referred to as a decomposition potential) of 3 V or more with respect to magnesium, thereby leading to completion of the present invention.

### SOLUTION TO PROBLEM

The present invention has the following configurations.
(1) An electrolyte solution for a magnesium battery comprising a mixture of a compound represented by the general formula [1], a Lewis acid or a compound represented by the general formula [4], and a solvent (hereinafter sometimes simply referred to as the electrolyte solution of an embodiment of the present invention): 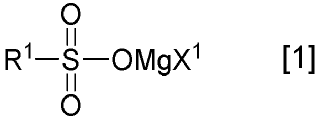 in the general formula [1], X¹ represents a halogeno group, and R¹ represents an alkyl group having 1 to 10 carbon atoms, which may have a group represented by - SO₃MgX² (X² represents a halogeno group); a haloalkyl group having 1 to 10 carbon atoms, which may have a group represented by -SO₃MgX² (X² is the same as described above); an aryl group having 6 to 14 carbon atoms, which may have an alkyl group having 1 to 6 carbon atoms, an alkoxy group having 1 to 6 carbon atoms, a halogeno group, or a group represented by -SO₃MgX² (X² is the same as described above); or a biphenyl group which may have a group represented by -SO₃MgX² (X² is the same as described above);

   Mg[N(SO₂R⁴)₂]₂ [4]

   in the general formula [4], four R⁴'s each independently represent an alkyl group having 1 to 6 carbon atoms, a perfluoroalkyl group having 1 to 6 carbon atoms, a phenyl group, or a perfluorophenyl group.
(2) The electrolyte solution as described in (1), wherein the electrolyte solution for a magnesium battery comprises a mixture of the compound represented by the general formula [1], the Lewis acid, and the solvent.
(3) The electrolyte solution as described in (1) or (2), wherein the compound represented by the general formula [1] is a compound represented by the general formula [2] or the general formula [3]: 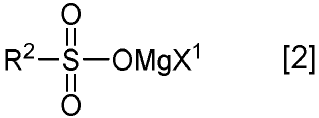 in the general formula [2], R² represents an alkyl group having 1 to 10 carbon atoms; a haloalkyl group having 1 to 10 carbon atoms; an aryl group having 6 to 14 carbon atoms, which may have an alkyl group having 1 to 6 carbon atoms, an alkoxy group having 1 to 6 carbon atoms, or a halogeno group; or a biphenyl group, and X¹ is the same as described above; 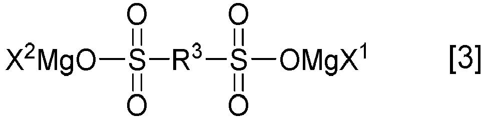 in the general formula [3], R³ represents an alkylene group having 1 to 10 carbon atoms; a haloalkylene group having 1 to 10 carbon atoms; an arylene group having 6 to 14 carbon atoms, which may have an alkyl group having 1 to 6 carbon atoms, an alkoxy group having 1 to 6 carbon atoms, or a halogeno group; or a biphenylene group, and X¹ and X² are each the same as described above.
(4) The electrolyte solution as described in any one of (1) to (3), wherein the compound represented by the general formula [1] is a compound represented by the general formula [2']: 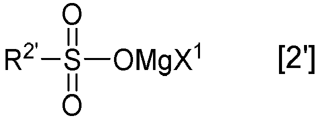 in the general formula [2'], R^{2'} represents an alkyl group having 1 to 10 carbon atoms; a fluoroalkyl group having 1 to 10 carbon atoms; an aryl group having 6 to 14 carbon atoms, which may have an alkyl group having 1 to 6 carbon atoms, an alkoxy group having 1 to 6 carbon atoms, or a fluoro group; or a biphenyl group, and X¹ is the same as described above.
(5) The electrolyte solution as described in any one of (1) to (4), wherein the Lewis acid is a Lewis acid containing beryllium, boron, aluminum, silicon, titanium, chromium, iron, cobalt, or tin as an element.
(6) The electrolyte solution as described in any one of (1) to (4), wherein the Lewis acid is a Lewis acid containing aluminum as an element.
(7) The electrolyte solution as described in any one of (1) to (4), wherein the Lewis acid is aluminum chloride.
(8) The electrolyte solution as described in any one of (1) to (3), wherein X¹ and X² in the compound represented by the general formula [1] are each a chloro group or a bromo group.
(9) The electrolyte solution as described in any one of (1) to (3), wherein X¹ and X² in the compound represented by the general formula [1] are a chloro group.
(10) The electrolyte solution as described in any one of (1) to (9), wherein the solvent is an ether-based solvent, a carbonate-based solvent, a halogenated hydrocarbon-based solvent, a nitrile-based solvent, or a sulfone-based solvent.
(11) The electrolyte solution as described in any one of (1) to (9), wherein the solvent is an ether-based solvent.
(12) An electrochemical device comprising: the electrolyte solution as described in any one of (1) to (11); a positive electrode; and a negative electrode.
(13) A compound represented by the general formula [1]: 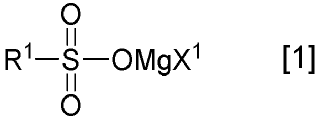 in the general formula [1], X¹ represents a halogeno group, and R¹ represents an alkyl group having 1 to 10 carbon atoms, which may have a group represented by - SO₃MgX² (X² represents a halogeno group); a haloalkyl group having 1 to 10 carbon atoms, which may have a group represented by -SO₃MgX² (X² is the same as described above); an aryl group having 6 to 14 carbon atoms, which may have an alkyl group having 1 to 6 carbon atoms, an alkoxy group having 1 to 6 carbon atoms, a halogeno group, or a group represented by -SO₃MgX² (X² is the same as described above); or a biphenyl group which may have a group represented by -SO₃MgX² (X² is the same as described above).
(14) The compound as described in (13), wherein the compound represented by the general formula [1] is a compound represented by the general formula [2] or the general formula [3]: 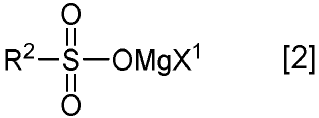 in the general formula [2], R² represents an alkyl group having 1 to 10 carbon atoms; a haloalkyl group having 1 to 10 carbon atoms; an aryl group having 6 to 14 carbon atoms, which may have an alkyl group having 1 to 6 carbon atoms, an alkoxy group having 1 to 6 carbon atoms, or a halogeno group; or a biphenyl group, and X¹ is the same as described above; 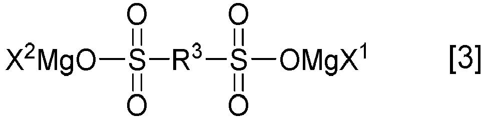 in the general formula [3], R³ represents an alkylene group having 1 to 10 carbon atoms; a haloalkylene group having 1 to 10 carbon atoms; an arylene group having 6 to 14 carbon atoms, which may have an alkyl group having 1 to 6 carbon atoms, an alkoxy group having 1 to 6 carbon atoms, or a halogeno group; or a biphenylene group, and X¹ and X² are each the same as described above.
(15) The compound described in (13), wherein the compound represented by the general formula [1] is a compound represented by the general formula [2']: 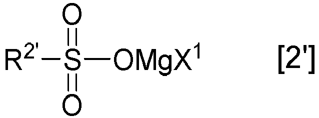 in the general formula [2'], R^{2'} represents an alkyl group having 1 to 10 carbon atoms; a fluoroalkyl group having 1 to 10 carbon atoms; an aryl group having 6 to 14 carbon atoms, which may have an alkyl group having 1 to 6 carbon atoms, an alkoxy group having 1 to 6 carbon atoms, or a fluoro group; or a biphenyl group, and X¹ is the same as described above.

### ADVANTAGEOUS EFFECTS OF INVENTION

Since the electrolyte solution of the embodiment of the present invention has higher oxidation resistance than an electrolyte solution in the related art, it can be used as an electrolyte solution for a high-voltage magnesium battery. Therefore, the electrolyte solution of the embodiment of the present invention has an effect that dissolution-precipitation of magnesium can be stabilized and repeated in a case where the electrolyte solution is used as an electrolyte solution of a magnesium battery.

### BRIEF DESCRIPTION OF DRAWINGS

Fig. 1 shows the results of cyclic voltammetry (CV) measurement at the 10^{th} cycle, using an electrolyte solution 1 [methanesulfonic acid magnesium chloride salt-aluminum chloride / triethylene glycol dimethyl ether solution] in Evaluation Example 1.
Fig. 2 shows the results of CV measurement at the 10^{th} cycle, using an electrolyte solution 2 [methanesulfonic acid magnesium chloride salt-aluminum chloride / triethylene glycol dimethyl ether-1,4-dioxane solution] in Evaluation Example 1.
Fig. 3 shows the results of CV measurement at the 10^{th} cycle, using an electrolyte solution 3 [trifluoromethanesulfonic acid magnesium chloride salt-aluminum chloride / triethylene glycol dimethyl ether solution] in Evaluation Example 1.
Fig. 4 shows the results of CV measurement at the 10^{th} cycle, using an electrolyte solution 4 [trifluoromethanesulfonic acid magnesium chloride salt-aluminum chloride / tetrahydrofuran solution] in Evaluation Example 1.
Fig. 5 shows the results of CV measurement at the 10^{th} cycle, using an electrolyte solution 5 [nonafluoro-1-butanesulfonic acid magnesium chloride salt-aluminum chloride / triethylene glycol dimethyl ether solution] in Evaluation Example 1.
Fig. 6 shows the results of CV measurement at the 10^{th} cycle, using an electrolyte solution 6 [benzenesulfonic acid magnesium chloride salt-aluminum chloride / triethylene glycol dimethyl ether solution] in Evaluation Example 1.
Fig. 7 shows the results of CV measurement at the 10^{th} cycle, using an electrolyte solution 7 [ethanesulfonic acid magnesium chloride salt-aluminum chloride / triethylene glycol dimethyl ether solution] in Evaluation Example 1.
Fig. 8 shows the results of CV measurement at the 10^{th} cycle, using an electrolyte solution 8 [ethanedisulfonic acid magnesium chloride salt-aluminum chloride / triethylene glycol dimethyl ether solution] in Evaluation Example 1.
Fig. 9 shows the results of CV measurement in the 10^{th} cycle, using a comparative electrolyte solution 1 [n-C₄H₉SMgCl-aluminum chloride / tetrahydrofuran solution] in Evaluation Example 1.
Fig. 10 shows the results of CV measurement in the 10^{th} cycle, using a comparative electrolyte solution 2 [(n-C₄H₉SMgCl)₂-aluminum chloride / tetrahydrofuran solution] in Evaluation Example 1.
Fig. 11 shows the results of CV measurement in the 10^{th} cycle, using a comparative electrolyte solution 3 [benzenesulfinic acid magnesium chloride salt-aluminum chloride / triethylene glycol dimethyl ether solution] in Evaluation Example 1.
Fig. 12 shows the results of CV measurement in the 10^{th} cycle, using a comparative electrolyte solution 4 [benzenesulfinic acid magnesium chloride salt-aluminum dichloride / triethylene glycol dimethyl ether solution] in Evaluation Example 1.
Fig. 13 shows the results of CV measurement in the 10^{th} cycle, using a comparative electrolyte solution 5 [p-i-C₃H₇-C₆H₄SMgCl-aluminum chloride / tetrahydrofuran solution] in Evaluation Example 1.
Fig. 14 shows the results (charge-discharge curve) of the constant current charge-discharge test of the 1^{st} cycle (solid line), the 5^{th} cycle (wavy line), and the 10^{th} cycle (dotted line), using an electrolyte solution 8 [ethanedisulfonic acid magnesium chloride salt-aluminum chloride / triethylene glycol dimethyl ether solution] in Evaluation Example 2.

### DESCRIPTION OF EMBODIMENTS

### - Compound Represented by General Formula [1] -

The compound represented by the general formula [1] according to the electrolyte solution of the embodiment of the present invention (hereinafter sometimes simply referred to as the compound represented by the general formula [1] of an embodiment of the present invention) is represented by the following general formula. 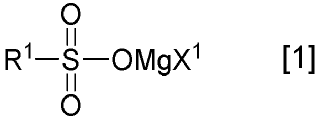 [In the general formula [1], X¹ represents a halogeno group, and R¹ represents an alkyl group having 1 to 10 carbon atoms, which may have a group represented by - SO₃MgX² (X² represents a halogeno group); a haloalkyl group having 1 to 10 carbon atoms, which may have a group represented by -SO₃MgX² (X² is the same as described above); an aryl group having 6 to 14 carbon atoms, which may have an alkyl group having 1 to 6 carbon atoms, an alkoxy group having 1 to 6 carbon atoms, a halogeno group, or a group represented by -SO₃MgX² (X² is the same as described above); or a biphenyl group which may have a group represented by -SO₃MgX² (X² is the same as described above).]

Specific examples of the halogeno group represented by X¹ and X² in the group represented by -SO₃MgX² in the general formula [1] include a fluoro group, a chloro group, a bromo group, and an iodo group, and among these, the chloro group and the bromo group are preferable, and the chloro group is more preferable.

X¹ and X² may be the same halogeno group or different halogeno groups, and among these, they are preferably the same halogeno group.

As the alkyl group having 1 to 10 carbon atoms in the "alkyl group having 1 to 10 carbon atoms, which may have a group represented by -SO₃MgX²", represented by R¹ in the general formula [1], an alkyl group having 1 to 6 carbon atoms is preferable, an alkyl group having 1 to 4 carbon atoms is more preferable, and an alkyl group having 1 or 2 carbon atoms is still more preferable. In addition, the alkyl group may be linear, branched, or cyclic, and among these, the alkyl group is preferably linear. Specific examples of such an alkyl group include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a tert-pentyl group, a neopentyl group, a 2-methylbutyl group, a 1,2-dimethylpropyl group, a 1-ethylpropyl group, a cyclopentyl group, an n-hexyl group, an isohexyl group, a sec-hexyl group, a tert-hexyl group, a neohexyl group, a 2-methylpentyl group, a 1,2-dimethylbutyl group, 2,3-dimethylbutyl group, a 1-ethylbutyl group, a cyclohexyl group, an n-heptyl group, a cycloheptyl group, an n-octyl group, a cyclooctyl group, an n-nonyl group, a cyclononyl group, an n-decyl group, a cyclodecyl group, a norbornyl group (norbornan-x-yl group), a bornyl group (bornan-x-yl group), a menthyl group (mentha-x-yl group), an adamantyl group, and a decahydronaphthyl group. Among these alkyl groups, the linear, branched, or cyclic alkyl group having 1 to 6 carbon atoms is preferable, the linear or branched alkyl group having 1 to 4 carbon atoms is more preferable, and the methyl group and the ethyl group are more preferable.

Examples of the haloalkyl group having 1 to 10 carbon atoms in the "haloalkyl group having 1 to 10 carbon atoms, which may have a group represented by - SO₃MgX²", represented by R¹ in the general formula [1] include a fluoroalkyl group having 1 to 10 carbon atoms, a chloroalkyl group having 1 to 10 carbon atoms, a bromoalkyl group having 1 to 10 carbon atoms, and an iodoalkyl group having 1 to 10 carbon atoms, and the haloalkyl group represents a group in which one or more halogeno groups are bonded to an alkyl group. Among these haloalkyl groups, the fluoroalkyl group having 1 to 10 carbon atoms is preferable, a perfluoroalkyl group having 1 to 10 carbon atoms is more preferable, a perfluoroalkyl group having 1 to 6 carbon atoms is still more preferable, and a perfluoroalkyl group having 1 to 4 carbon atoms is particularly preferable. In addition, the haloalkyl group may be linear, branched, or cyclic, and among these, the haloalkyl group is preferably linear. Specific examples of such a haloalkyl group include a fluoroalkyl group having 1 to 10 carbon atoms, such as a fluoromethyl group, a difluoromethyl group, a trifluoromethyl group, a fluoroethyl group, a difluoroethyl group, a trifluoroethyl group, a tetrafluoroethyl group, a pentafluoroethyl group, a heptafluoro-n-propyl group, a heptafluoroisopropyl group, a heptafluorocyclopropyl group, a perfluoro-n-butyl group, a perfluoroisobutyl group, a perfluoro-sec-butyl group, a perfluoro-tert-butyl group, a perfluorocyclobutyl group, a perfluoro-n-pentyl group, a perfluoroisopentyl group, a perfluoro-sec-pentyl group, a perfluoro-tert-pentyl group, a perfluoroneopentyl group, a perfluoro-2-methylbutyl group, a perfluoro-1,2-dimethylpropyl group, a perfluoro-1-ethylpropyl group, a perfluorocyclopentyl group, a perfluoro-n-hexyl group, a perfluoroisohexyl group, a perfluoro-sec-hexyl group, a perfluoro-tert-hexyl group, a perfluoroneohexyl group, a perfluoro-2-methylpentyl group, a perfluoro-1,2-dimethylbutyl group, a perfluoro-2,3-dimethylbutyl group, a perfluoro-1-ethylbutyl group, a perfluorocyclohexyl group, a perfluoro-n-heptyl group, a perfluorocycloheptyl group, a perfluoro-n-octyl group, a perfluorocyclooctyl group, a perfluoro-n-nonyl group, a perfluorocyclononyl group, a perfluoro-n-decyl group, a perfluorocyclodecyl group, a perfluoronorbornyl group (perfluoronorbornan-χ-yl group), a perfluorobornyl group (perfluorobornan-χ-yl group), a perfluoromenthyl group (perfluoromentha)-χ-yl group), a perfluoroadamantyl group, and a perfluorodecahydronaphthyl group; a chloroalkyl group having 1 to 10 carbon atoms, such as a chloromethyl group, a dichloromethyl group, a trichloromethyl group, a chloroethyl group, a dichloroethyl group, a trichloroethyl group, a tetrachloroethyl group, a pentachloroethyl group, a heptachloro-n-propyl group, a perchloro-n-butyl group, a perchloro-n-pentyl group, a perchloro-n-hexyl group, a perchloro-n-heptyl group, a perchloro-n-octyl group, a perchloro-n-nonyl group, and a perchloro-n-decyl group; bromoalkyl group having 1 to 10 carbon atoms, such as a bromomethyl group, a dibromomethyl group, a tribromomethyl group, a bromoethyl group, a dibromoethyl group, a tribromoethyl group, a tetrabromoethyl group, a pentabromoethyl group, a heptabromo-n-propyl group, a perbromo-n-butyl group, a perbromo-n-pentyl group, a perbromo-n-hexyl group, a perbromo-n-heptyl group, a perbromo-n-octyl group, a perbromo-n-nonyl group, and a perbromo-n-decyl group; and an iodoalkyl group having 1 to 10 carbon atoms, such as an iodomethyl group, a diiodomethyl group, a triiodomethyl group, an iodoethyl group, a diiodoethyl group, a triiodoethyl group, a tetraiodoethyl group, a pentaiodoethyl group, a heptaiodo-n-propyl group, a periodo-n-butyl group, a periodo-n-pentyl group, a periodo-n-hexyl group, a periodo-n-heptyl group, a periodo-n-octyl group, a periodo-n-nonyl group, and a periodo-n-decyl group. Among these haloalkyl groups, the linear, branched, or cyclic fluoroalkyl group having 1 to 10 carbon atoms is preferable, the linear, branched, or cyclic perfluoroalkyl group having 1 to 10 carbon atoms is more preferable, the linear, branched, or cyclic perfluoroalkyl group having 1 to 6 carbon atoms is still more preferable, the linear or branched perfluoroalkyl group having 1 to 4 carbon atoms is particularly preferable, and the linear perfluoroalkyl group having 1 to 4 carbon atoms is the most preferable.

Specific examples of the group represented by -SO₃MgX² in the "alkyl group having 1 to 10 carbon atoms, which may have a group represented by -SO₃MgX²", and the "haloalkyl group having 1 to 10 carbon atoms, which may have a group represented by -SO₃MgX²", represented by R¹ in the general formula [1] include -SO₃MgF, - SO₃MgCl, -SO₃MgBr, and -SO₃MgI, and among these, -SO₃MgCl and -SO₃MgBr are preferable, and -SO₃MgCl is more preferable.

In the "alkyl group having 1 to 10 carbon atoms, which may have a group represented by -SO₃MgX²", and the "haloalkyl group having 1 to 10 carbon atoms, which may have a group represented by -SO₃MgX²", represented by R¹ in the general formula [1], the alkyl group to which at least one group represented by -SO₃MgX² is bonded is denoted as an "alkyl group having 1 to 10 carbon atoms, having a group represented by -SO₃MgX²", and the haloalkyl group to which at least one group represented by -SO₃MgX² is bonded is denoted as a "haloalkyl group having 1 to 10 carbon atoms, having a group represented by -SO₃MgX²".

The group represented by -SO₃MgX² in the "alkyl group having 1 to 10 carbon atoms, which has a group represented by -SO₃MgX²" and the "haloalkyl group having 1 to 10 carbon atoms, which has a group represented by -SO₃MgX²", represented by R¹ in the general formula [1], may be bonded to any of carbon atoms constituting the alkyl group or the haloalkyl group. In addition, the group represented by -SO₃MgX² may have one bond to a carbon atom constituting an alkyl group or a haloalkyl group, or may have a plurality of bonds. Among these, in a case where a carbon atom directly bonded to the sulfonyl group bonded to R¹ in the general formula [1] is set to the 1-position in the linear alkyl group or haloalkyl group, the linear alkyl group or haloalkyl group is preferably an alkyl group or haloalkyl group in which one group represented by -SO₃MgX² is bonded to the carbon atom at the ω-position is preferable.

As the "alkyl group having 1 to 10 carbon atoms, having a group represented by - SO₃MgX²", represented by R¹ in the general formula [1], a linear alkyl group having 1 to 10 carbon atoms, which has one group represented by -SO₃MgX² at the ω-position, is preferable, a linear alkyl group having 1 to 6 carbon atoms, having one group represented by -SO₃MgX² at the ω-position, is more preferable, a linear alkyl group having 1 to 4 carbon atoms, having one group represented by -SO₃MgX² at the ω-position, is still more preferable, and an alkyl group having 1 or 2 carbon atoms, which has one group represented by -SO₃MgX² at the ω-position, is particularly preferable.

As the "haloalkyl group having 1 to 10 carbon atoms, having a group represented by -SO₃MgX²", represented by R¹ in the general formula [1], a linear haloalkyl group having 1 to 10 carbon atoms, which has one group represented by -SO₃MgX² at the ω-position, is preferable, a linear haloalkyl group having 1 to 6 carbon atoms, which has one group represented by -SO₃MgX² at the w-position, is more preferable, and a linear haloalkyl group having 1 to 4 carbon atoms, which has one group represented by - SO₃MgX² at the w-position, is still more preferable.

As the "haloalkyl group having 1 to 10 carbon atoms, which may have a group represented by -SO₃MgX²", represented by R¹ in the general formula [1], a haloalkyl group having 1 to 10 carbon atoms, which has no group represented by -SO₃MgX², that is, an unsubstituted haloalkyl group having 1 to 10 carbon atoms is preferable.

As the aryl group having 6 to 14 carbon atoms in the "aryl group having 6 to 14 carbon atoms, which may have an alkyl group having 1 to 6 carbon atoms, an alkoxy group having 1 to 6 carbon atoms, a halogeno group, or a group represented by - SO₃MgX²", represented by R¹ in the general formula [1], an aryl group having 6 to 10 carbon atoms is preferable, and an aryl group having 6 carbon atoms is more preferable. Specific examples of such an aryl group include a phenyl group, a naphthyl group, and an anthracenyl group. Among these aryl groups, the phenyl group and the naphthyl group are preferable, and the phenyl group is more preferable.

As the alkyl group having 1 to 6 carbon atoms in the "aryl group having 6 to 14 carbon atoms, which may have an alkyl group having 1 to 6 carbon atoms, an alkoxy group having 1 to 6 carbon atoms, a halogeno group, or a group represented by - SO₃MgX²", represented by R¹ in the general formula [1], an alkyl group having 1 to 4 carbon atoms is preferable, and an alkyl group having 1 or 2 carbon atoms is more preferable. In addition, the alkyl group may be linear, branched, or cyclic. Specific examples of such an alkyl group include the same groups as the specific examples of the alkyl group having 1 to 6 carbon atoms, which is a preferred alkyl group in the "alkyl group having 1 to 10 carbon atoms, which may have a group represented by - SO₃MgX²", represented by R¹ in the general formula [1]. Specific examples of the alkyl group having 1 to 4 carbon atoms or the alkyl group having 1 or 2 carbon atoms also include the same groups as the specific examples of the alkyl group having 1 to 4 carbon atoms, which is a more preferable alkyl group, and the specific examples of the alkyl group having 1 or 2 carbon atoms, which is a still more preferable alkyl group, in the "alkyl group having 1 to 10 carbon atoms, which may have a group represented by -SO₃MgX²", represented by R¹ in the general formula [1].

As the alkoxy group having 1 to 6 carbon atoms in the "aryl group having 6 to 14 carbon atoms, which may have an alkyl group having 1 to 6 carbon atoms, an alkoxy group having 1 to 6 carbon atoms, a halogeno group, or a group represented by - SO₃MgX²", represented by R¹ in the general formula [1], an alkoxy group having 1 to 4 carbon atoms is preferable, and an alkoxy group having 1 or 2 carbon atoms is more preferable. In addition, the alkoxy group may be linear, branched, or cyclic. Specific examples of such an alkoxy group include a methoxy group, an ethoxy group, an n-propoxy group, an isopropoxy group, a cyclopropoxy group, an n-butoxy group, an isobutoxy group, a sec-butoxy group, a tert-butoxy group, a cyclobutoxy group, an n-pentyloxy group, an isopentyloxy group, a sec-pentyloxy group, a tert-pentyloxy group, a neopentyloxy group, a 2-methylbutoxy group, a 1,2-dimethylpropoxy group, a 1-ethylpropoxy group, a cyclopentyloxy group, an n-hexyloxy group, an isohexyloxy group, a sec-hexyloxy group, a tert-hexyloxy group, a neohexyloxy group, a 2-methylpentyloxy group, a 1,2-dimethylbutoxy group, a 2,3-dimethylbutoxy group, a 1-ethylbutoxy group, and a cyclohexyloxy group. Among these alkoxy groups, a linear or branched alkoxy group having 1 to 4 carbon atoms is more preferable, and a methoxy group and an ethoxy group are still more preferable.

Specific examples of the halogeno group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1] include a fluoro group, a chloro group, a bromo group, and an iodo group, and the fluoro group is preferable.

Specific examples of the group represented by -SO₃MgX² in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1] include the same groups as the specific examples of the group represented by -SO₃MgX² in the "alkyl group having 1 to 10 carbon atoms, which may have a group represented by -SO₃MgX²", and the "haloalkyl group having 1 to 10 carbon atoms, which may have a group represented by -SO₃MgX²", represented by R¹ in the general formula [1], and preferred specific examples thereof also include the same groups.

Specific examples of the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1] include groups represented by the general formulae [I] to [III]. 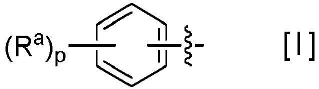 (In the general formula [I], p pieces of R^{a}'s each independently represent an alkyl group having 1 to 6 carbon atoms, an alkoxy group having 1 to 6 carbon atoms, a halogeno group, or a group represented by -SO₃MgX² (X² is the same as described above), and p represents an integer of 0 to 5.) 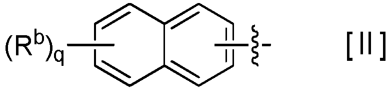 (In the general formula [II], q pieces of R^{b}'s each independently represent an alkyl group having 1 to 6 carbon atoms, an alkoxy group having 1 to 6 carbon atoms, a halogeno group, or a group represented by -SO₃MgX² (X² is the same as described above), and q represents an integer from 0 to 7.) 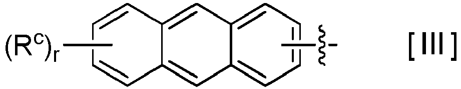 (In the general formula [III], r pieces of R^{c}'s each independently represent an alkyl group having 1 to 6 carbon atoms, an alkoxy group having 1 to 6 carbon atoms, a halogeno group, or a group represented by -SO₃MgX² (X² is the same as described above), and r represents an integer from 0 to 9.)

Specific examples and preferred specific examples of the alkyl group having 1 to 6 carbon atoms, the alkoxy group having 1 to 6 carbon atoms, the halogeno group, or the group represented by -SO₃MgX², represented by R^{a}, R^{b}, and R^{c} in the general formulae [I] to [III], include the same groups as the specific examples of the alkyl group having 1 to 6 carbon atoms, the alkoxy group having 1 to 6 carbon atoms, the halogeno group, or the group represented by -SO₃MgX² in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1].

As R^{a}, R^{b}, and R^{c} in the general formulae [I] to [III], the alkyl group having 1 to 6 carbon atoms, the alkoxy group having 1 to 6 carbon atoms, the fluoro group, and the group represented by -SO₃MgX² are preferable, and among these, the group represented by -SO₃MgX² is more preferable, -SO₃MgCl and -SO₃MgBr are still more preferable, and -SO₃MgCl is particularly preferable.

p in the general formula [I] represents an integer of 0 to 5, and among these, p is preferably an integer of 0 to 3, more preferably an integer of 0 or 1, and still more preferably 0.

q in the general formula [II] represents an integer of 0 to 7, and among these, q is preferably an integer of 0 to 3, more preferably an integer of 0 or 1, and still more preferably 0.

r in the general formula [III] represents an integer of 0 to 9, and among these, r is preferably an integer of 0 to 3, more preferably an integer of 0 or 1, and still more preferably 0.

A case where p, q, and r in the general formulae [I] to [III] are "0" indicates that any of an alkyl group having 1 to 6 carbon atoms, an alkoxy group having 1 to 6 carbon atoms, a halogeno group, and a group represented by -SO₃MgX² is not contained.

In a case where p, q, and r in the general formulae [I] to [III] are integers of 2 or more, the plurality of R^{a}'s, R^{b}'s, and R^{c}'s may be the same groups or different groups.

p pieces of R^{a}'s in the general formula [I] may be bonded to any of carbon atoms on a phenyl group, and among these, it is preferable that at least one of R^{a}'s is bonded to a carbon atom at the 4-position on a phenyl group.

q pieces of R^{b}'s in the general formula [II] may be bonded to any of carbon atoms on the naphthyl group.

r pieces of R^{c}'s in the general formula [III] may be bonded to any of carbon atoms on the anthracenyl group.

Specific examples of the group represented by the general formula [I] include a phenyl group; a phenyl group having an alkyl group having 1 to 6 carbon atoms, such as a tolyl group, a xylyl group, a mesityl group, an ethylphenyl group, a diethylphenyl group, a triethylphenyl group, an n-propylphenyl group, a di-n-propylphenyl group, a tri-n-propylphenyl group, an isopropylphenyl group, a diisopropylphenyl group, a triisopropylphenyl group, an n-butylphenyl group, a di-n-butylphenyl group, a tri-n-butylphenyl group, a tert-butylphenyl group, a di-tert-butylphenyl group, a tri-tert-butylphenyl group, an n-pentylphenyl group, a di-n-pentylphenyl group, a tri-n-pentylphenyl group, an n-hexylphenyl group, a di-n-hexylphenyl group, and a tri-n-hexylphenyl group; a phenyl group having an alkoxy group having 1 to 6 carbon atoms, such as a methoxyphenyl group, a dimethoxyphenyl group, a trimethoxyphenyl group, an ethoxyphenyl group, a diethoxyphenyl group, a triethoxyphenyl group, an n-propoxyphenyl group, a di-n-propoxyphenyl group, a tri-n-propoxyphenyl group, an isopropoxyphenyl group, a diisopropoxyphenyl group, a triisopropoxyphenyl group, an n-butoxyphenyl group, a di-n-butoxyphenyl group, a tri-n-butoxyphenyl group, a tert-butoxyphenyl group, a di-tert-butoxyphenyl group, a tri-tert-butoxyphenyl group, an n-pentyloxyphenyl group, a di-n-pentyloxyphenyl group, a tri-n-pentyloxyphenyl group, an n-hexyloxyphenyl group, a di-n-hexyloxyphenyl group, and a tri-n-hexyloxyphenyl group; a phenyl group having a halogeno group, such as a fluorophenyl group, a difluorophenyl group, a trifluorophenyl group, a tetrafluorophenyl group, a perfluorophenyl group, a chlorophenyl group, a dichlorophenyl group, a trichlorophenyl group, a tetrachlorophenyl group, a perchlorophenyl group, a bromophenyl group, a dibromophenyl group, a tribromophenyl group, a tetrabromophenyl group, a perbromophenyl group, an iodophenyl group, a diiodophenyl group, a triiodophenyl group, a tetraiodophenyl group, and a periodophenyl group; and a group represented by -C₆H₄-SO₃MgX^{2.}, such as a group represented by -C₆H₄-SO₃MgF, a group represented by -C₆H₄-SO₃MgCl, a group represented by -C₆H₄-SO₃MgBr, and a group represented by -C₆H₄-SO₃MgI. Among these groups, the phenyl group, the phenyl group having an alkyl group having 1 to 6 carbon atoms, the phenyl group having an alkoxy group having 1 to 6 carbon atoms, the phenyl group having a fluoro group, and the group represented by -C₆H₄-SO₃MgX² are preferable, and among these, the phenyl group is more preferable.

Specific examples of the group represented by the general formula [II] include a naphthyl group; a naphthyl having an alkyl group having 1 to 6 carbon atoms, such as a methylnaphthyl group, a dimethylnaphthyl group, a trimethylnaphthyl group, an ethylnaphthyl group, a diethylnaphthyl group, a triethylnaphthyl group, an n-propylnaphthyl group, a di-n-propylnaphthyl group, a tri-n-propylnaphthyl group, an isopropylnaphthyl group, a diisopropylnaphthyl group, a triisopropylnaphthyl group, an n-butylnaphthyl group, a di-n-butylnaphthyl group, a tri-n-butylnaphthyl group, a tert-butylnaphthyl group, a di-tert-butylnaphthyl group, a tri-tert-butylnaphthyl group, an n-pentylnaphthyl group, a di-n-pentylnaphthyl group, a tri-n-pentylnaphthyl group, an n-hexylnaphthyl group, a di-n-hexylnaphthyl group, and a tri-n-hexylnaphthyl group; a naphthyl having an alkoxy group having 1 to 6 carbon atoms, such as a methoxynaphthyl group, a dimethoxynaphthyl group, a trimethoxynaphthyl group, an ethoxynaphthyl group, a diethoxynaphthyl group, a triethoxynaphthyl group, an n-propoxynaphthyl group, a di-n-propoxynaphthyl group, a tri-n-propoxynaphthyl group, an isopropoxynaphthyl group, a diisopropoxynaphthyl group, a triisopropoxynaphthyl group, an n-butoxynaphthyl group, a di-n-butoxynaphthyl group, a tri-n-butoxynaphthyl group, a tert-butoxynaphthyl group, a di-tert-butoxynaphthyl group, a tri-tert-butoxynaphthyl group, an n-pentyloxynaphthyl group, a di-n-pentyloxynaphthyl group, a tri-n-pentyloxynaphthyl group, an n-hexyloxynaphthyl group, a di-n-hexyloxynaphthyl group, and a tri-n-hexyloxynaphthyl group; a naphthyl having a halogeno group, such as a fluoronaphthyl group, a difluoronaphthyl group, a trifluoronaphthyl group, a tetrafluoronaphthyl group, a perfluoronaphthyl group, a chloronaphthyl group, a dichloronaphthyl group, a trichloronaphthyl group, a tetrachloronaphthyl group, a perchloronaphthyl group, a bromonaphthyl group, a dibromonaphthyl group, a tribromonaphthyl group, a tetrabromonaphthyl group, a perbromonaphthyl group, an iodonaphthyl group, a diiodonaphthyl group, a triiodonaphthyl group, a tetraiodonaphthyl group, and a periodonaphthyl group; and a group represented by - C₁₀H₆-SO₃MgX², such as a group represented by -C₁₀H₆-SO₃MgF, a group represented by -C₁₀H₆-SO₃MgCl, a group represented by -C₁₀H₆-SO₃MgBr, and a group represented by -C₁₀H₆-SO₃MgI. Among these groups, the naphthyl group, the naphthyl group having an alkyl group having 1 to 6 carbon atoms, the naphthyl group having an alkoxy group having 1 to 6 carbon atoms, the naphthyl group having a fluoro group, and the group represented by -C₁₀H₆-SO₃MgX² are preferable, and the naphthyl group is more preferable.

Specific examples of the group represented by the general formula [III] include an anthracenyl group; an anthracenyl group having an alkyl group having 1 to 6 carbon atoms, such as a methylanthracenyl group, a dimethylanthracenyl group, a trimethylanthracenyl group, an ethylanthracenyl group, a diethylanthracenyl group, a triethylanthracenyl group, an n-propylanthracenyl group, a di-n-propylanthracenyl group, a tri-n-propylanthracenyl group, an isopropylanthracenyl group, a diisopropylanthracenyl group, a triisopropylanthracenyl group, an n-butylanthracenyl group, a di-n-butylanthracenyl group, a tri-n-butylanthracenyl group, a tert-butylanthracenyl group, a di-tert-butylanthracenyl group, a tri-tert-butylanthracenyl group, an n-pentylanthracenyl group, a di-n-pentylanthracenyl group, a tri-n-pentylanthracenyl group, an n-hexylanthracenyl group, a di-n-hexylanthracenyl group, and a tri-n-hexylanthracenyl group; an anthracenyl group having an alkoxy group having 1 to 6 carbon atoms, such as a methoxyanthracenyl group, a dimethoxyanthracenyl group, a trimethoxyanthracenyl group, an ethoxyanthracenyl group, a diethoxyanthracenyl group, a triethoxyanthracenyl group, an n-propoxyanthracenyl group, a di-n-propoxyanthracenyl group, a tri-n-propoxyanthracenyl group, an isopropoxyanthracenyl group, a diisopropoxyanthracenyl group, a triisopropoxyanthracenyl group, an n-butoxyanthracenyl group, a di-n-butoxyanthracenyl group, a tri-n-butoxyanthracenyl group, a tert-butoxyanthracenyl group, a di-tert-butoxyanthracenyl group, a tri-tert-butoxyanthracenyl group, an n-pentyloxyanthracenyl group, a di-n-pentyloxyanthracenyl group, a tri-n-pentyloxyanthracenyl group, an n-hexyloxyanthracenyl group, a di-n-hexyloxyanthracenyl group, and a tri-n-hexyloxyanthracenyl group; an anthracenyl group having a halogeno group, such as a fluoroanthracenyl group, a difluoroanthracenyl group, a trifluoroanthracenyl group, a tetrafluoroanthracenyl group, a perfluoroanthracenyl group, a chloroanthracenyl group, a dichloroanthracenyl group, a trichloroanthracenyl group, a tetrachloroanthracenyl group, a perchloroanthracenyl group, bromoanthracenyl group, a dibromoanthracenyl group, a tribromoanthracenyl group, a tetrabromoanthracenyl group, a perbromoanthracenyl group, an iodoanthracenyl group, a diiodoanthracenyl group, a triiodoanthracenyl group, a tetraiodoanthracenyl group, and a periodoanthracenyl group; and a group represented by -C₁₄H₈-SO₃MgX², such as a group represented by -C₁₄H₈-SO₃MgF, a group represented by -C₁₄H₈-SO₃MgCl, a group represented by - C₁₄H₈-SO₃MgBr, and a group represented by -C₁₄H₈-SO₃MgI. Among these groups, the anthracenyl group, the anthracenyl group having an alkyl group having 1 to 6 carbon atoms, the anthracenyl group having an alkoxy group having 1 to 6 carbon atoms, the anthracenyl group having a fluoro group, and the group represented by -C₁₄H₈-SO₃MgX² are preferable, and among these, the anthracenyl group is more preferable.

As the groups represented by the general formulae [I] to [III], the groups represented by the general formulae [I] and [II] are preferable, and the group represented by the general formula [I] is more preferable.

Specific examples of the group represented by -SO₃MgX² in the "biphenyl group which may have a group represented by -SO₃MgX²", represented by R¹ in the general formula [1], include the same groups as the specific examples of the group represented by -SO₃MgX² in the "alkyl group having 1 to 10 carbon atoms, which may have a group represented by -SO₃MgX²", and the "haloalkyl group having 1 to 10 carbon atoms, which may have a group represented by -SO₃MgX²", represented by R¹ in the general formula [1], and preferred specific examples also include the same groups.

As the "biphenyl group which may have a group represented by -SO₃MgX²", represented by R¹ in the general formula [1], a biphenyl group having no group represented by -SO₃MgX², that is, a (unsubstituted) biphenyl group is preferable.

As R¹ in the general formula [1], the alkyl group having 1 to 10 carbon atoms, which may have a group represented by -SO₃MgX²; the fluoroalkyl group having 1 to 10 carbon atoms, which may have a group represented by -SO₃MgX²; the aryl group having 6 to 14 carbon atoms, which may have an alkyl group having 1 to 6 carbon atoms, an alkoxy group having 1 to 6 carbon atoms, a fluoro group, or a group represented by -SO₃MgX²; and the biphenyl group which may have a group represented by -SO₃MgX² are preferable, and among these, the alkyl group having 1 to 10 carbon atoms, which may have a group represented by -SO₃MgX²; the fluoroalkyl group having 1 to 10 carbon atoms, which may have a group represented by -SO₃MgX²; and the aryl group having 6 to 14 carbon atoms, which may have an alkyl group having 1 to 6 carbon atoms, an alkoxy group having 1 to 6 carbon atoms, a fluoro group, or a group represented by -SO₃MgX², are more preferable.

Preferred specific examples of the compound represented by the general formula [1] include compounds represented by the general formulae [2] and [3]. 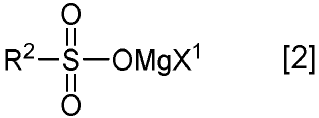 (In the general formula [2], R² represents an alkyl group having 1 to 10 carbon atoms; a haloalkyl group having 1 to 10 carbon atoms; an aryl group having 6 to 14 carbon atoms which may have an alkyl group having 1 to 6 carbon atoms, an alkoxy group having 1 to 6 carbon atoms, or a halogeno group; or a biphenyl group, and X¹ is the same as described above.) 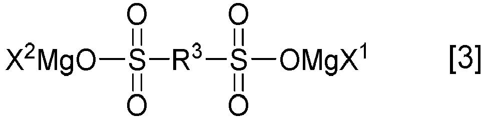 (In the general formula [3], R³ represents an alkylene group having 1 to 10 carbon atoms; a haloalkylene group having 1 to 10 carbon atoms; an arylene group having 6 to 14 carbon atoms which may have an alkyl group having 1 to 6 carbon atoms, an alkoxy group having 1 to 6 carbon atoms, or a halogeno group; or a biphenylene group, and X¹ and X² are each the same as described above.)

Specific examples of the alkyl group having 1 to 10 carbon atoms, represented by R² in the general formula [2], include the same groups as the specific examples of the alkyl group in the "alkyl group having 1 to 10 carbon atoms, which may have a group represented by -SO₃MgX²", represented by R¹ in the general formula [1], and preferred specific examples and the like thereof are also the same.

Specific examples of the haloalkyl group having 1 to 10 carbon atoms, represented by R² in the general formula [2], include the same groups as the specific examples of the haloalkyl group in the "haloalkyl group having 1 to 10 carbon atoms, which may have a group represented by -SO₃MgX²", represented by R¹ in the general formula [1], and preferred specific examples and the like thereof are also the same.

Specific examples of the aryl group having 6 to 14 carbon atoms in the "aryl group having 6 to 14 carbon atoms, which may have an alkyl group having 1 to 6 carbon atoms, an alkoxy group having 1 to 6 carbon atoms, or a halogeno group", represented by R² in the general formula [2] include the same groups as the specific examples of the aryl group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the alkyl group having 1 to 6 carbon atoms, the alkoxy group having 1 to 6 carbon atoms, or the halogeno group in the "aryl group having 6 to 14 carbon atoms, which may have an alkyl group having 1 to 6 carbon atoms, an alkoxy group having 1 to 6 carbon atoms, or a halogeno group", represented by R² in the general formula [2] include the same groups as the specific examples of the alkyl group, the alkoxy group, or the halogeno group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the "aryl group having 6 to 14 carbon atoms, which may have an alkyl group having 1 to 6 carbon atoms, an alkoxy group having 1 to 6 carbon atoms, or a halogeno group", represented by R² in the general formula [2] include groups represented by the general formulae [I-I] to [III-I]. 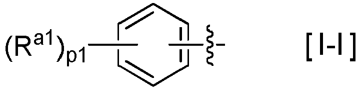 (In the general formula [I-I], p1 pieces of R^{a1}'s each independently represent an alkyl group having 1 to 6 carbon atoms, an alkoxy group having 1 to 6 carbon atoms, or a halogeno group, and p1 represents an integer of 0 to 5.) 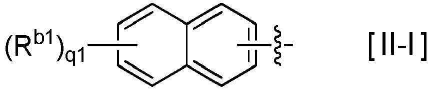 (In the general formula [II-I], q1 pieces of R^{b1}'s each independently represent an alkyl group having 1 to 6 carbon atoms, an alkoxy group having 1 to 6 carbon atoms, or a halogeno group, and q1 represents an integer of 0 to 7.) 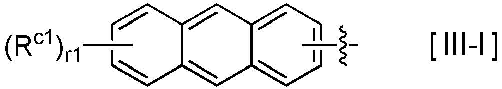 (In the general formula [III-I], r1 pieces of R^{c1}'s each independently represent an alkyl group having 1 to 6 carbon atoms, an alkoxy group having 1 to 6 carbon atoms, or a halogeno group, and r1 represents an integer of 0 to 9.)

Specific examples and preferred specific examples of the alkyl group having 1 to 6 carbon atoms, the alkoxy group having 1 to 6 carbon atoms, and the halogeno group, represented by R^{a1}, R^{b1}, and R^{c1} in the general formulae [I-I] to [III-I], include the same groups as the specific examples of the alkyl group having 1 to 6 carbon atoms, the alkoxy group having 1 to 6 carbon atoms, and the halogeno group in the "aryl group having 6 to 14 carbon atoms, which may have an alkyl group having 1 to 6 carbon atoms, an alkoxy group having 1 to 6 carbon atoms, or a halogeno group", represented by R² in the general formula [2].

As R^{a1}, R^{b1}, and R^{c1} in the general formulae [I-I] to [III-I], the alkyl group having 1 to 6 carbon atoms, the alkoxy group having 1 to 6 carbon atoms, and the fluoro group are preferable.

p1 in the general formula [I-I] represents an integer of 0 to 5, and among these, p1 is preferably an integer of 0 to 3, more preferably an integer of 0 or 1, and still more preferably 0.

q1 in the general formula [II-I] represents an integer of 0 to 7, and among these, q1 is preferably an integer of 0 to 3, more preferably an integer of 0 or 1, and still more preferably 0.

r1 in the general formula [III-I] represents an integer of 0 to 9, and among these, r1 is preferably an integer of 0 to 3, more preferably an integer of 0 or 1, and still more preferably 0.

A case where p1, q1, and r1 in the general formulae [I-I] to [III-I] are "0" indicates that any of an alkyl group having 1 to 6 carbon atoms, an alkoxy group having 1 to 6 carbon atoms, and a halogeno group is not contained.

In a case where p1, q1, and r1 in the general formulae [I-I] to [III-I] are integers of 2 or more, the plurality of R^{a1}'s, R^{b1}'s, and R^{c1}'s may be the same groups or different groups.

p1 pieces of R^{a1}'s in the general formula [I-I] may be bonded to any of carbon atoms on a phenyl group, and among these, it is preferable that at least one of R^{a1}'s is bonded to a carbon atom at the 4-position on a phenyl group.

q1 pieces of R^{b1}'s in the general formula [II-I] may be bonded to any of carbon atoms on the naphthyl group.

r1 pieces of R^{c1}'s in the general formula [III-I] may be bonded to any of carbon atoms on the anthracenyl group.

Specific examples of the group represented by the general formula [I-I] include a phenyl group, a phenyl group having an alkyl group having 1 to 6 carbon atoms, a phenyl group having an alkoxy group having 1 to 6 carbon atoms, and a phenyl group having a halogeno group. Specific examples of the phenyl group having an alkyl group having 1 to 6 carbon atoms, the phenyl group having an alkoxy group having 1 to 6 carbon atoms, and the phenyl group having a halogeno group include the same groups of the specific example of the group represented by the general formula [I]. Among these groups, the phenyl group, the phenyl group having an alkyl group having 1 to 6 carbon atoms, the phenyl group having an alkoxy group having 1 to 6 carbon atoms, and the phenyl group having a fluoro group are preferable, and among these, the phenyl group is more preferable.

Specific examples of the group represented by the general formula [II-I] include a naphthyl group, a naphthyl group having an alkyl group having 1 to 6 carbon atoms, a naphthyl group having an alkoxy group having 1 to 6 carbon atoms, and a naphthyl group having a halogeno group. Specific examples of such the naphthyl group having an alkyl group having 1 to 6 carbon atoms, the naphthyl group having an alkoxy group having 1 to 6 carbon atoms, and the naphthyl group having a halogeno group include the same groups of the specific example of the group represented by the general formula [II]. Among these groups, the naphthyl group, the naphthyl group having an alkyl group having 1 to 6 carbon atoms, the naphthyl group having an alkoxy group having 1 to 6 carbon atoms, and the naphthyl group having a fluoro group are preferable, and the naphthyl group is more preferable.

Specific examples of the group represented by the general formula [III-I] include an anthracenyl group, an anthracenyl group having an alkyl group having 1 to 6 carbon atoms, an anthracenyl group having an alkoxy group having 1 to 6 carbon atoms, and an anthracenyl group having a halogeno group. Specific examples of the anthracenyl group having an alkyl group having 1 to 6 carbon atoms, the anthracenyl group having an alkoxy group having 1 to 6 carbon atoms, and the anthracenyl group having a halogeno group include the same groups as the specific example of the group represented by the general formula [III]. Among these groups, the anthracenyl group, an anthracenyl group having an alkyl group having 1 to 6 carbon atoms, the anthracenyl group having an alkoxy group having 1 to 6 carbon atoms, and the anthracenyl group having a fluoro group are preferable, and among these, the anthracenyl group is particularly preferable.

As the groups represented by the general formulae [I-I] to [III-I], the groups represented by the general formulae [I-I] and [II-I] are preferable, and among these, the group represented by the general formula [I-I] is more preferable.

As R² in the general formula [2], the alkyl group having 1 to 10 carbon atoms; the fluoroalkyl group having 1 to 10 carbon atoms; the aryl group having 6 to 14 carbon atoms, which may have an alkyl group having 1 to 6 carbon atoms, an alkoxy group having 1 to 6 carbon atoms, or a fluoro group; and the biphenyl group are preferable, and among these, the alkyl group having 1 to 10 carbon atoms; the fluoroalkyl group having 1 to 10 carbon atoms; and the aryl group having 6 to 14 carbon atoms, which may have an alkyl group having 1 to 6 carbon atoms, an alkoxy group having 1 to 6 carbon atoms, or a fluoro group are more preferable.

As the alkylene group having 1 to 10 carbon atoms, represented by R³ in the general formula [3], an alkylene group having 1 to 6 carbon atoms is preferable, an alkylene group having 1 to 4 carbon atoms is more preferable, and an alkylene group having 1 or 2 carbon atoms is still more preferable. In addition, the alkylene group may be linear, branched, or cyclic. Specific examples of such an alkylene group include a methylene group, a dimethylene group (ethylene group), a trimethylene group (propane-1,3-diyl group), a propylene group (propane-1,2-diyl group), a tetramethylene group (butane-1,4-diyl group), a 1-methyltrimethylene group, a 2-methyltrimethylene group, a 1,2-dimethyldimethylene group (1,2-dimethylethylene group), a 1,1-dimethyldimethylene group (1,1-dimethylethylene group), an ethyldimethylene group (ethylethylene group), a pentamethylene group (pentane-1,5-diyl group), a 1,3-cyclopentylene group (cyclopentane-1,3-diyl group), a hexamethylene group (hexane-1,6-diyl group), a 1,4-cyclohexylene group (cyclohexane-1,4-diyl group), a heptamethylene group (heptane-1,7-diyl group), an octamethylene group (octane-1,8-diyl group), and a decamethylene group (decane-1,10-diyl group). Among these alkylene groups, the linear, branched, or cyclic alkylene group having 1 to 6 carbon atoms is preferable, the linear or branched alkylene group having 1 to 4 carbon atoms is more preferable, and the methylene group and the dimethylene group (ethylene group) are more preferable.

Examples of the haloalkylene group having 1 to 10 carbon atoms, represented by R³ in the general formula [3], include a fluoroalkylene group having 1 to 10 carbon atoms, a chloroalkylene group having 1 to 10 carbon atoms, a bromoalkylene group having 1 to 10 carbon atoms, and an iodoalkylene group having 1 to 10 carbon atoms, and represents a group in which one or a plurality of halogeno groups are bonded on the alkylene group. Among these haloalkylene groups, a fluoroalkylene group having 1 to 10 carbon atoms is preferable, a perfluoroalkylene group having 1 to 10 carbon atoms is more preferable, a perfluoroalkylene group having 1 to 6 carbon atoms is still more preferable, and a perfluoroalkylene group having 1 to 4 carbon atoms is particularly preferable. In addition, the haloalkylene group may be linear, branched, or cyclic. Specific examples of such a haloalkylene group include fluoroalkylene group having 1 to 10 carbon atoms, such as a fluoromethylene group, a difluoromethylene group, a fluorodimethylene group (fluoroethylene group), a difluorodimethylene group (difluoroethylene group), a trifluorodimethylene group (trifluoroethylene group), a tetrafluorodimethylene group (tetrafluoroethylene group), a pentafluorotrimethylene group (pentafluoropropane-1,3-diyl group), a pentafluoropropylene group (pentafluoropropane-1,2-diyl group), a perfluorotetramethylene group (perfluorobutane-1,4-diyl group), a perfluoro-1-methyltrimethylene group, a perfluoro-2-methyltrimethylene group, a perfluoro-1,2-dimethyldimethylene group (perfluoro-1,2-dimethylethylene group), a perfluoro-1,1-dimethyldimethylene group (perfluoro-1,1-dimethylethylene group), a perfluoroethyldimethylene group (perfluoroethylethylene group), a perfluoropentamethylene group (perfluoropentane-1,5-diyl group), a perfluoro-1,3-cyclopentylene group (perfluorocyclopentane-1,3-diyl group), a perfluorohexamethylene group (perfluorohexane-1,6-diyl group), a perfluoro-1,4-cyclohexylene group (perfluorocyclohexane-1,4-diyl group), a perfluoroheptamethylene group (perfluoroheptane-1,7-diyl group), a perfluorooctamethylene group (perfluorooctane-1,8-diyl group), a perfluorononamethylene group (perfluorononane-1,9-diyl group), and a perfluorodecamethylene group (perfluorodecane-1,10-diyl group); a chloroalkylene group having 1 to 10 carbon atoms, such as a chloromethylene group, a dichloromethylene group, a chlorodimethylene group (chloroethylene group), a dichlorodimethylene group (dichloroethylene group), a trichlorodimethylene group (trichloroethylene group), a tetrachlorodimethylene group (tetrachloroethylene group), a pentachlorotrimethylene group (pentachloropropane-1,3-diyl group), a pentachloropropylene group (pentachloropropane-1,2-diyl group), a perchlorotetramethylene group (perchlorobutane-1,4-diyl group), a perchloro-1-methyltrimethylene group, a perchloro-2-methyltrimethylene group, a perchloro-1,2-dimethyldimethylene group (perchloro-1,2-dimethylethylene group), a perchloro-1,1-dimethyldimethylene group (perchloro-1,1-dimethylethylene group), a perchloroethyldimethylene group (perchloroethylethylene group), a perchloropentamethylene group (perchloropentane-1,5-diyl group), a perchloro-1,3-cyclopentylene group (perchlorocyclopentane-1,3-diyl group), a perchlorohexamethylene group (perchlorohexane-1,6-diyl group), a perchloro-1,4-cyclohexylene group (perchlorocyclohexane-1,4-diyl group), a perchloroheptamethylene group (perchloroheptane-1,7)-diyl group), a perchlorooctamethylene group (perchlorooctane-1,8-diyl group), and a perchlorodecamethylene group (perchlorodecane-1,10-diyl group); bromoalkylene group having 1 to 10 carbon atoms, such as a bromomethylene group, a dibromomethylene group, a bromodimethylene group (bromoethylene group), a dibromodimethylene group (dibromoethylene group), a tribromodimethylene group (tribromoethylene group), a tetrabromodimethylene group (tetrabromoethylene group), a pentabromotrimethylene group (pentabromopropane-1,3-diyl group), a pentabromopropylene group (pentabromopropane-1,2-diyl group), a perbromotetramethylene group (perbromobutane-1,4-diyl group), a perbromo-1-methyltrimethylene group, a perbromo-2-methyltrimethylene group, a perbromo-1,2-dimethyldimethylene group (perbromo-1,2-dimethylethylene group), a perbromo-1,1-dimethyldimethylene group (perbromo-1,1-dimethylethylene group), a perbromoethyldimethylene group (perbromoethylethylene group), a perbromopentamethylene group (perbromopentane-1,5-diyl group), a perbromo-1,3-cyclopentylene group (perbromocyclopentane-1,3-diyl group), a perbromohexamethylene group (perbromohexane-1,6-diyl group), a perbromo-1,4-cyclohexylene group (perbromocyclohexane-1,4-diyl group), a perbromoheptamethylene group (perbromoheptane-1,7-diyl group), a perbromooctamethylene group (perbromooctane-1,8-diyl group), and a perbromodecamethylene group (perbromodecane-1,10-diyl group); and an iodoalkylene group having 1 to 10 carbon atoms, such as an iodomethylene group, a diiodomethylene group, an iododimethylene group (iodoethylene group), a diiododimethylene group (diiodoethylene group), a triiododimethylene group (triiodoethylene group), a tetraiododimethylene group (tetraiodoethylene group), a pentaiodotrimethylene group (pentaiodopropane-1,3-diyl group), a pentaiodopropylene group (pentaiodopropane-1,2-diyl group), a periodotetramethylene group (periodobutane-1,4-diyl group), a periodo-1-methyltrimethylene group, a periodo-2-methyltrimethylene group, a periodo-1,2-dimethyldimethylene group (periodo-1,2-dimethylethylene group), a periodo-1,1-dimethyldimethylene group (periodo-1,1-dimethylethylene group), a periodoethyldimethylene group (periodoethylethylene group), a periodopentamethylene group (periodopentane-1,5-diyl group), a periodo-1,3-cyclopentylene group (periodocyclopentane-1,3-diyl group), a periodohexamethylene group (periodohexane-1,6-diyl group), a periodo-1,4-cyclohexylene group (periodocyclohexane-1,4-diyl group), a periodoheptamethylene group (periodoheptane-1,7-diyl group), a periodooctamethylene group (periodooctane-1,8-diyl group), and a periododecamethylene group (periododecane-1,10-diyl group). Among these haloalkylene groups, the linear, branched, or cyclic fluoroalkylene group having 1 to 10 carbon atoms is preferable, the linear, branched, or cyclic perfluoroalkylene group having 1 to 10 carbon atoms is more preferable, the linear, branched, or cyclic perfluoroalkylene group having 1 to 6 carbon atoms is still more preferable, the linear or branched perfluoroalkylene group having 1 to 4 carbon atoms is particularly preferable, and the linear perfluoroalkylene group having 1 to 4 carbon atoms is the most preferable.

As the arylene group having 6 to 14 carbon atoms in the "arylene group having 6 to 14 carbon atoms, which may have an alkyl group having 1 to 6 carbon atoms, an alkoxy group having 1 to 6 carbon atoms, or a halogeno group", represented by R³ in the general formula [3], an arylene group having 6 to 10 carbon atoms is preferable, and an arylene group having 6 carbon atoms is more preferable. Specific examples of such an arylene group include a phenylene group, a naphthylene group, and an anthracenylene group. Among these arylene groups, the phenylene group and the naphthylene group are preferable, and the phenylene group is more preferable.

Specific examples of the alkyl group having 1 to 6 carbon atoms, the alkoxy group having 1 to 6 carbon atoms, or the halogeno group in the "arylene group having 6 to 14 carbon atoms, which may have an alkyl group having 1 to 6 carbon atoms, an alkoxy group having 1 to 6 carbon atoms, or a halogeno group", represented by R³ in the general formula [3] include the same groups as the specific examples of the alkyl group, the alkoxy group, or the halogeno group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the "arylene group having 6 to 14 carbon atoms, which may have an alkyl group having 1 to 6 carbon atoms, an alkoxy group having 1 to 6 carbon atoms, or a halogeno group", represented by R³ in the general formula [3] include groups represented by the general formulae [I-II] to [III-II]. 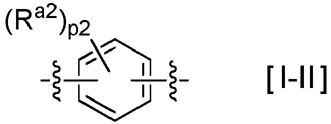 (In the general formula [I-II], p2 pieces of R^{a2}'s each independently represent an alkyl group having 1 to 6 carbon atoms, an alkoxy group having 1 to 6 carbon atoms, or a halogeno group, and p2 represents an integer of 0 to 4.) 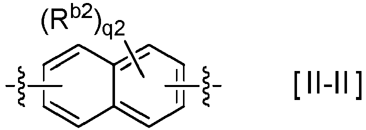 (In the general formula [II-II], q2 pieces of R^{b2}'s each independently represent an alkyl group having 1 to 6 carbon atoms, an alkoxy group having 1 to 6 carbon atoms, or a halogeno group, and q2 represents an integer of 0 to 6.) 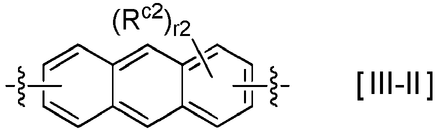 (In the general formula [III-II], r2 pieces of R^{c2}'s each independently represent an alkyl group having 1 to 6 carbon atoms, an alkoxy group having 1 to 6 carbon atoms, or a halogeno group, and r2 represents an integer of 0 to 8.)

Specific examples and preferred specific examples of the alkyl group having 1 to 6 carbon atoms, the alkoxy group having 1 to 6 carbon atoms, and the halogeno group, represented by R^{a2}, R^{b2}, and R^{c2} in the general formulae [I-II] to [III-II], include the same groups as the specific examples of the alkyl group having 1 to 6 carbon atoms, the alkoxy group having 1 to 6 carbon atoms, and the halogeno group in the "arylene group having 6 to 14 carbon atoms, which may have an alkyl group having 1 to 6 carbon atoms, an alkoxy group having 1 to 6 carbon atoms, or a halogeno group", represented by R³ in the general formula [3].

As R^{a2}, R^{b2}, and R^{c2} in the general formulae [I-II] to [III-II], the alkyl group having 1 to 6 carbon atoms, the alkoxy group having 1 to 6 carbon atoms, and the fluoro group are preferable.

p2 in the general formula [I-II] represents an integer of 0 to 4, and among these, p2 is preferably an integer of 0 to 2, more preferably an integer of 0 or 1, and still more preferably 0.

q2 in the general formula [II-II] represents an integer of 0 to 6, and among these, q2 is preferably an integer of 0 to 4, more preferably an integer of 0 to 2, and still more preferably 0.

r2 in the general formula [III-II] represents an integer of 0 to 8, and among these, r2 is preferably an integer of 0 to 4, more preferably an integer of 0 to 2, and still more preferably 0.

A case where p2, q2, and r2 in the general formulae [I-II] to [III-II] are "0" indicates that any of an alkyl group having 1 to 6 carbon atoms, an alkoxy group having 1 to 6 carbon atoms, and a halogeno group is not contained.

In a case where p2, q2, and r2 in the general formulae [I-II] to [III-II] are integers of 2 or more, the plurality of R^{a2}'s, R^{b2}'s, and R^{c2}'s may be the same groups or different groups.

In the general formula [I-II], the two bonds may be bonded to any of the carbon atoms constituting the benzene ring.

In the general formula [II-II], the two bonds may be bonded to any of the carbon atoms constituting the naphthalene ring.

In the general formula [III-II], the two bonds may be bonded to any of the carbon atoms constituting the anthracene ring.

p2 pieces of R^{a2}'s in the general formula [I-II] may be bonded to any of carbon atoms on the phenylene group.

q2 pieces of R^{b2}'s in the general formula [II-II] may be bonded to any of carbon atoms on the naphthylene group.

r2 pieces of R^{c2}'s in the general formula [III-II] may be bonded to any of carbon atoms on the anthracenylene group.

Specific examples of the group represented by the general formula [I-II] include a phenylene group; a phenylene group having an alkyl group having 1 to 6 carbon atoms, such as a methylphenylene group, a dimethylphenylene group, a trimethylphenylene group, an ethylphenylene group, a diethylphenylene group, a triethylphenylene group, an n-propylphenylene group, a di-n-propylphenylene group, a tri-n-propylphenylene group, an isopropylphenylene group, a diisopropylphenylene group, a triisopropylphenylene group, an n-butylphenylene group, a di-n-butylphenylene group, a tri-n-butylphenylene group, a tert-butylphenylene group, a di-tert-butylphenylene group, a tri-tert-butylphenylene group, an n-pentylphenylene group, a di-n-pentylphenylene group, a tri-n-pentylphenylene group, a n-hexylphenylene group, a din-hexylphenylene group, and a tri-n-hexylphenylene group; a phenylene group having an alkoxy group having 1 to 6 carbon atoms, such as a methoxyphenylene group, a dimethoxyphenylene group, a trimethoxyphenylene group, an ethoxyphenylene group, a diethoxyphenylene group, a triethoxyphenylene group, an n-propoxyphenylene group, a di-n-propoxyphenylene group, a tri-n-propoxyphenylene group, an isopropoxyphenylene group, a diisopropoxyphenylene group, a triisopropoxyphenylene group, an n-butoxyphenylene group, a di-n-butoxyphenylene group, a tri-n-butoxyphenylene group, a tert-butoxyphenylene group, a di-tert-butoxyphenylene group, a tri-tert-butoxyphenylene group, an n-pentyloxyphenylene group, a di-n-pentyloxyphenylene group, a tri-n-pentyloxyphenylene group, an n-hexyloxyphenylene group, a di-n-hexyloxyphenylene group, and a tri-n-hexyloxyphenylene group; and a phenylene group having a halogeno group, such as a fluorophenylene group, a difluorophenylene group, a trifluorophenylene group, a tetrafluorophenylene group, a chlorophenylene group, a dichlorophenylene group, a trichlorophenylene group, a tetrachlorophenylene group, a bromophenylene group, a dibromophenylene group, a tribromophenylene group, a tetrabromophenylene group, an iodophenylene group, a diiodophenylene group, a triiodophenylene group, and a tetraiodophenylene group. Among these groups, the phenylene group, the phenylene group having an alkyl group having 1 to 6 carbon atoms, the phenylene group having an alkoxy group having 1 to 6 carbon atoms, and the phenylene group having a fluoro group are preferable, and among these, the phenylene group is more preferable.

Specific examples of the group represented by the general formula [II-II] include a naphthylene group; a naphthylene group having an alkyl group having 1 to 6 carbon atoms, such as a methylnaphthylene group, a dimethylnaphthylene group, a trimethylnaphthylene group, an ethylnaphthylene group, a diethylnaphthylene group, a triethylnaphthylene group, an n-propylnaphthylene group, a di-n-propylnaphthylene group, a tri-n-propylnaphthylene group, an isopropylnaphthylene group, a diisopropylnaphthylene group, a triisopropylnaphthylene group, an n-butylnaphthylene group, a di-n-butylnaphthylene group, a tri-n-butylnaphthylene group, a tert-butylnaphthylene group, a di-tert-butylnaphthylene group, a tri-tert-butylnaphthylene group, an n-pentylnaphthylene group, a di-n-pentylnaphthylene group, a tri-n-pentylnaphthylene group, an n-hexylnaphthylene group, a di-n-hexylnaphthylene group, and a tri-n-hexylnaphthylene group; a naphthylene group having an alkoxy group having 1 to 6 carbon atoms, such as a methoxynaphthylene group, a dimethoxynaphthylene group, a trimethoxynaphthylene group, an ethoxynaphthylene group, a diethoxynaphthylene group, a triethoxynaphthylene group, an n-propoxynaphthylene group, a di-n-propoxynaphthylene group, a tri-n-propoxynaphthylene group, an isopropoxynaphthylene group, a diisopropoxynaphthylene group, a triisopropoxynaphthylene group, an n-butoxynaphthylene group, a di-n-butoxynaphthylene group, a tri-n-butoxynaphthylene group, a tert-butoxynaphthylene group, a di-tert-butoxynaphthylene group, a tri-tert-butoxynaphthylene group, an n-pentyloxynaphthylene group, a di-n-pentyloxynaphthylene group, a tri-n-pentyloxynaphthylene group, an n-hexyloxynaphthylene group, a di-n-hexyloxynaphthylene group, and a tri-n-hexyloxynaphthylene group; a naphthylene group having a halogeno group, such as a fluoronaphthylene group, a difluoronaphthylene group, a trifluoronaphthylene group, a tetrafluoronaphthylene group, a perfluoronaphthylene group, a chloronaphthylene group, a dichloronaphthylene group, a trichloronaphthylene group, a tetrachloronaphthylene group, a perchloronaphthylene group, a bromonaphthylene group, a dibromonaphthylene group, a tribromonaphthylene group, a tetrabromonaphthylene group, a perbromonaphthylene group, an iodonaphthylene group, a diiodonaphthylene group, a triiodonaphthylene group, a tetraiodnonaphthylene group, and a periodonaphthylene group. Among these groups, the naphthylene group, the naphthylene group having an alkyl group having 1 to 6 carbon atoms, the naphthylene group having an alkoxy group having 1 to 6 carbon atoms, and the naphthylene group having a fluoro group are preferable, and among these, the naphthylene group is more preferable.

Specific examples of the group represented by the general formula [III-II] include an anthracenylene group; an anthracenylene group having an alkyl group having 1 to 6 carbon atoms, such as a methylanthracenylene group, a dimethylanthracenylene group, a trimethylanthracenylene group, an ethylanthracenylene group, a diethylanthracenylene group, a triethylanthracenylene group, an n-propylanthracenylene group, a di-n-propylanthracenylene group, a tri-n-propylanthracenylene group, an isopropylanthracenylene group, a diisopropylanthracenylene group, a triisopropylanthracenylene group, an n-butylanthracenylene group, a di-n-butylanthracenylene group, a tri-n-butylanthracenylene group, a tert-butylanthracenylene group, a di-tert-butylanthracenylene group, a tri-tert-butylanthracenylene group, an n-pentylanthracenylene group, a di-n-pentylanthracenylene group, a tri-n-pentylanthracenylene group, an n-hexylanthracenylene group, a di-n-hexylanthracenylene group, and a tri-n-hexylanthracenylene group; an anthracenylene group having an alkoxy group having 1 to 6 carbon atoms, such as a methoxyanthracenylene group, a dimethoxyanthracenylene group, a trimethoxyanthracenylene group, an ethoxyanthracenylene group, a diethoxyanthracenylene group, a triethoxyanthracenylene group, an n-propoxyanthracenylene group, a di-n-propoxyanthracenylene group, a tri-n-propoxyanthracenylene group, an isopropoxyanthracenylene group, a diisopropoxyanthracenylene group, a triisopropoxyanthracenylene group, an n-butoxyanthracenylene group, a di-n-butoxyanthracenylene group, a tri-n-butoxyanthracenylene group, a tert-butoxyanthracenylene group, a di-tert-butoxyanthracenylene group, a tri-tert-butoxyanthracenylene group, an n-pentyloxyanthracenylene group, a di-n-pentyloxyanthracenylene group, a tri-n-pentyloxyanthracenylene group, an n-hexyloxyanthracenylene group, a di-n-hexyloxyanthracenylene group, and a tri-n-hexyloxyanthracenylene group; and an anthracenylene group having a halogeno group, such as a fluoroanthracenylene group, a difluoroanthracenylene group, a trifluoroanthracenylene group, a tetrafluoroanthracenylene group, a perfluoroanthracenylene group, a chloroanthracenylene group, a dichloroanthracenylene group, a trichloroanthracenylene group, a tetrachloroanthracenylene group, a perchloroanthracenylene group, bromoanthracenylene group, a dibromoanthracenylene group, a tribromoanthracenylene group, a tetrabromoanthracenylene group, a perbromoanthracenylene group, an iodoanthracenylene group, a diiodoanthracenylene group, a triiodoanthracenylene group, a tetraiodoanthracenylene group, and a periodoanthracenylene group. Among these groups, the anthracenylene group, the anthracenylene group having an alkyl group having 1 to 6 carbon atoms, the anthracenylene group having an alkoxy group having 1 to 6 carbon atoms, and the anthracenylene group having a fluoro group are preferable, and among these, the anthracenylene group is particularly preferable.

As the groups represented by the general formulae [I-II] to [III-II], the groups represented by the general formulae [I-II] and [II-II] are preferable, and among these, the group represented by the general formula [I-II] is more preferable.

As R³ in the general formula [3], the alkylene group having 1 to 10 carbon atoms; the fluoroalkylene group having 1 to 10 carbon atoms; the arylene group having 6 to 14 carbon atoms, which may have an alkyl group having 1 to 6 carbon atoms, an alkoxy group having 1 to 6 carbon atoms, or a fluoro group; and the biphenylene group are preferable, and among these, the alkylene group having 1 to 10 carbon atoms; the fluoroalkylene group having 1 to 10 carbon atoms; and the arylene group having 6 to 14 carbon atoms, which may have an alkyl group having 1 to 6 carbon atoms, an alkoxy group having 1 to 6 carbon atoms, or a fluoro group are more preferable, and the alkylene group having 1 to 10 carbon atoms is still more preferable.

Preferred specific examples of the compound represented by the general formula [2] include a compound represented by the general formula [2']. 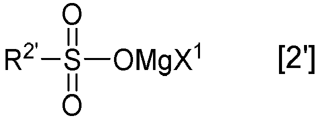 (In the general formula [2'], R^{2'} represents an alkyl group having 1 to 10 carbon atoms; a fluoroalkyl group having 1 to 10 carbon atoms; an aryl group having 6 to 14 carbon atoms, which may have an alkyl group having 1 to 6 carbon atoms, an alkoxy group having 1 to 6 carbon atoms, or a fluoro group; or a biphenyl group, and X¹ is the same as described above.)

Specific examples of the alkyl group having 1 to 10 carbon atoms, represented by R^{2'} in the general formula [2'], include the same groups as the specific examples of the alkyl group in the "alkyl group having 1 to 10 carbon atoms, which may have a group represented by -SO₃MgX²", represented by R¹ in the general formula [1], and preferred specific examples and the like thereof are also the same.

The fluoroalkyl group having 1 to 10 carbon atoms, represented by R^{2'} in the general formula [2'], represents a group in which one or a plurality of fluoro groups are bonded to the alkyl group. Among these fluoroalkyl groups, a perfluoroalkyl group having 1 to 10 carbon atoms is preferable, a perfluoroalkyl group having 1 to 6 carbon atoms is more preferable, and a perfluoroalkyl group having 1 to 4 carbon atoms is still more preferable. In addition, the fluoroalkyl group may be linear, branched, or cyclic. Specific examples of such a fluoroalkyl group include the same groups as the specific examples of the fluoroalkyl group having 1 to 10 carbon atoms in the haloalkyl group having 1 to 10 carbon atoms in the "haloalkyl group having 1 to 10 carbon atoms, which may have a group represented by -SO₃MgX²", represented by R¹ in the general formula [1], and preferred specific examples and the like thereof are also the same.

Specific examples of the aryl group having 6 to 14 carbon atoms in the "aryl group having 6 to 14 carbon atoms, which may have an alkyl group having 1 to 6 carbon atoms, an alkoxy group having 1 to 6 carbon atoms, or a fluoro group", represented by R^{2'} in the general formula [2'] include the same groups as the specific examples of the aryl group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the alkyl group having 1 to 6 carbon atoms and the alkoxy group having 1 to 6 carbon atoms in the "aryl group having 6 to 14 carbon atoms, which may have an alkyl group having 1 to 6 carbon atoms, an alkoxy group having 1 to 6 carbon atoms, or a fluoro group", represented by R^{2'} in the general formula [2'] include the same groups as the specific examples of the alkyl group and the alkoxy group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the "aryl group having 6 to 14 carbon atoms, which may have an alkyl group having 1 to 6 carbon atoms, an alkoxy group having 1 to 6 carbon atoms, or a fluoro group", represented by R^{2'} in the general formula [2'] include groups represented by the general formulae [I-I'] to [III-I']. 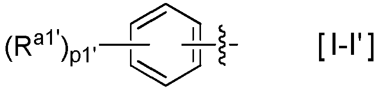 (In the general formula [I-I'], p1' pieces of R^{a1'}'s each independently represent an alkyl group having 1 to 6 carbon atoms, an alkoxy group having 1 to 6 carbon atoms, or a fluoro group, and p1' represents an integer of 0 to 5.) 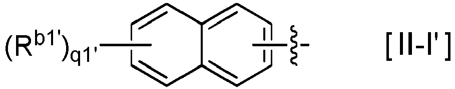 (In the general formula [II-I'], q1' pieces of R^{b1'}'s each independently represent an alkyl group having 1 to 6 carbon atoms, an alkoxy group having 1 to 6 carbon atoms, or a fluoro group, and q1' represents an integer of 0 to 7.) 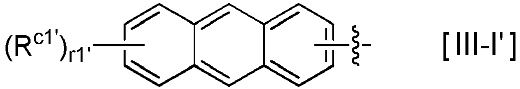 (In the general formula [III-I'], r1' pieces of R^{c1'}'s each independently represent an alkyl group having 1 to 6 carbon atoms, an alkoxy group having 1 to 6 carbon atoms, or a fluoro group, and r1' represents an integer of 0 to 9.)

Specific examples and preferred specific examples of the alkyl group having 1 to 6 carbon atoms and the alkoxy group having 1 to 6 carbon atoms, represented by R^{a1'}, R^{b1'}, and R^{c1'} in the general formulae [I-I'] to [III-I'], include the same groups as the specific examples of the alkyl group having 1 to 6 carbon atoms and the alkoxy group having 1 to 6 carbon atoms in the "aryl group having 6 to 14 carbon atoms, which may have an alkyl group having 1 to 6 carbon atoms and an alkoxy group having 1 to 6 carbon atoms, or a halogeno group", represented by R² in the general formula [2].

p1' in the general formula [I-I'] represents an integer of 0 to 5, and among these, p1' is preferably an integer of 0 to 3, more preferably an integer of 0 or 1, and still more preferably 0.

q1' in the general formula [II-I'] represents an integer of 0 to 7, and among these, q1' is preferably an integer of 0 to 3, more preferably an integer of 0 or 1, and still more preferably 0.

r1' in the general formula [III-I'] represents an integer of 0 to 9, and among these, r1' is preferably an integer of 0 to 3, more preferably an integer of 0 or 1, and still more preferably 0.

A case where p1', q1', and r1' in the general formulae [I-I'] to [III-I'] are "0" indicates that any of an alkyl group having 1 to 6 carbon atoms, an alkoxy group having 1 to 6 carbon atoms, and a fluoro group is not contained.

In a case where p1', q1', and r1' in the general formulae [I-I'] to [III-I'] are integers of 2 or more, the plurality of R^{a1'}'s, R^{b1'}'s, and R^{c1'}'s may be the same groups or different groups.

p1' pieces of R^{a1'}'s in the general formula [I-I'] may be bonded to any carbon atom on a phenyl group, and among these, it is preferable that at least one of R^{a1'}'s is bonded to a carbon atom at the 4-position on a phenyl group.

q1' pieces of R^{b1'}'s in the general formula [II-I'] may be bonded to any of carbon atoms on the naphthyl group.

r1' pieces of R^{c1'}'s in the general formula [III-I'] may be bonded to any of carbon atoms on the anthracenyl group.

Specific examples of the group represented by the general formula [I-I'] include a phenyl group, a phenyl group having an alkyl group having 1 to 6 carbon atoms, a phenyl group having an alkoxy group having 1 to 6 carbon atoms, and a phenyl group having a fluoro group. Specific examples of the phenyl group having a fluoro group include a phenyl group having a fluoro group, such as a fluorophenyl group, a difluorophenyl group, a trifluorophenyl group, a tetrafluorophenyl group, and a perfluorophenyl group. In addition, specific examples of the phenyl group having an alkyl group having 1 to 6 carbon atoms and the phenyl group having an alkoxy group having 1 to 6 carbon atoms include the same groups of the specific example of the group represented by the general formula [I]. Among these groups, the phenyl group is preferable.

Specific examples of the group represented by the general formula [II-I'] include a naphthyl group, a naphthyl group having an alkyl group having 1 to 6 carbon atoms, a naphthyl group having an alkoxy group having 1 to 6 carbon atoms, and a naphthyl group having a fluoro group. Specific examples of the naphthyl group having a fluoro group include a fluoronaphthyl group, a difluoronaphthyl group, a trifluoronaphthyl group, a tetrafluoronaphthyl group, and a perfluoronaphthyl group. In addition, specific examples of the naphthyl group having an alkyl group having 1 to 6 carbon atoms and the naphthyl group having an alkoxy group having 1 to 6 carbon atoms include the same group as the specific example of the group represented by the general formula [II]. Among these groups, the naphthyl group is preferable.

Specific examples of the group represented by the general formula [III-I'] include an anthracenyl group, an anthracenyl group having an alkyl group having 1 to 6 carbon atoms, an anthracenyl group having an alkoxy group having 1 to 6 carbon atoms, and an anthracenyl group having a fluoro group. Specific examples of the anthracenyl group having a fluoro group include a fluoroanthracenyl group, a difluoroanthracenyl group, a trifluoroanthracenyl group, a tetrafluoroanthracenyl group, and a perfluoroanthracenyl group. In addition, specific examples of the anthracenyl group having an alkyl group having 1 to 6 carbon atoms and the anthracenyl group having an alkoxy group having 1 to 6 carbon atoms include the same group as the specific example of the group represented by the general formula [III]. Among these groups, the anthracenyl group is preferable.

As the groups represented by the general formulae [I-I'] to [III-I'], the groups represented by the general formulae [I-I'] and [II-I'] are preferable, and among these, the group represented by the general formula [I-I'] is more preferable.

As R^{2'} in the general formula [2'], the alkyl group having 1 to 10 carbon atoms; the fluoroalkyl group having 1 to 10 carbon atoms; and the aryl group having 6 to 14 carbon atoms, which may have an alkyl group having 1 to 6 carbon atoms, an alkoxy group having 1 to 6 carbon atoms, or a fluoro group are preferable.

Preferred specific examples of the compound represented by the general formula [2'] include compounds represented by the following formulae.

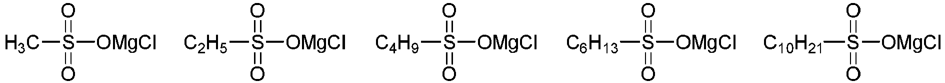

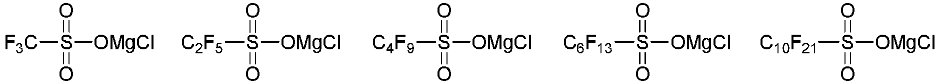

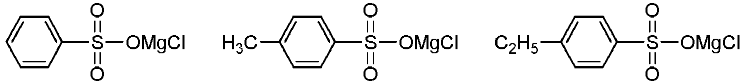

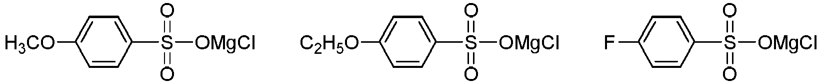

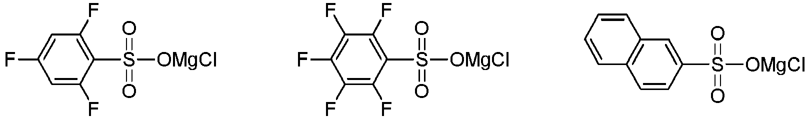

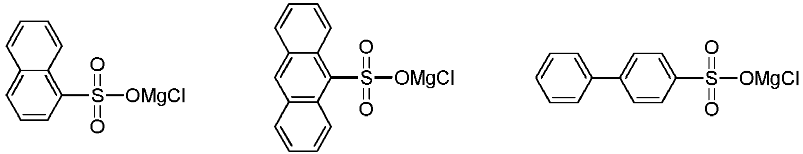

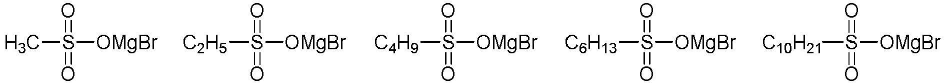

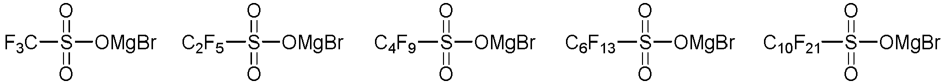

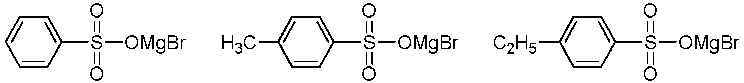

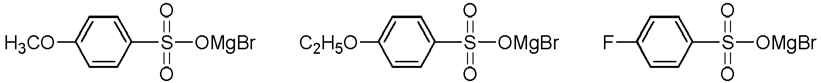

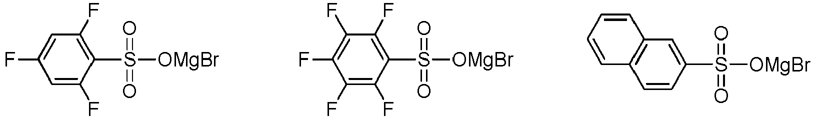

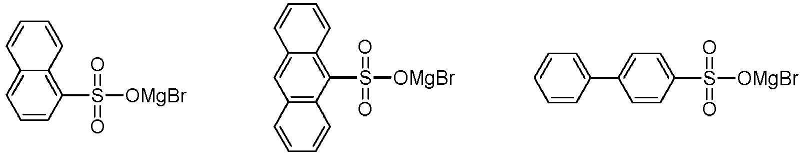

Preferred specific examples of the compound represented by the general formula [3] include a compound represented by the general formula [3']. 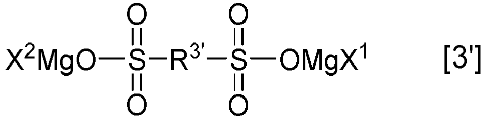 (In the general formula [3'], R^{3'} represents an alkylene group having 1 to 10 carbon atoms; a fluoroalkylene group having 1 to 10 carbon atoms; an arylene group having 6 to 14 carbon atoms which may have an alkyl group having 1 to 6 carbon atoms, an alkoxy group having 1 to 6 carbon atoms or a fluoro group; or a biphenylene group, and X¹ and X² are each the same as described above.)

Specific examples of the alkylene group having 1 to 10 carbon atoms, represented by R^{3'} in the general formula [3'], include the same groups as the specific examples of the alkylene group having 1 to 10 carbon atoms, represented by R³ in the general formula [3], and preferred specific examples and the like thereof are also the same.

The fluoroalkylene group having 1 to 10 carbon atoms, represented by R^{3'} in the general formula [3'], represents a group in which one or a plurality of fluoro groups are bonded to the alkylene group. Among these fluoroalkylene groups, a perfluoroalkylene group having 1 to 10 carbon atoms is preferable, a perfluoroalkylene group having 1 to 6 carbon atoms is more preferable, and a perfluoroalkylene group having 1 to 4 carbon atoms is still more preferable. In addition, the fluoroalkylene group may be linear, branched, or cyclic. Specific examples of such a fluoroalkylene group include the same groups as specific examples of the fluoroalkylene group having 1 to 10 carbon atoms in the haloalkylene group having 1 to 10 carbon atoms, represented by R³ in the general formula [3], and preferred specific examples and the like thereof are also the same.

Specific examples of the arylene group having 6 to 14 carbon atoms in the "arylene group having 6 to 14 carbon atoms, which may have an alkyl group having 1 to 6 carbon atoms, an alkoxy group having 1 to 6 carbon atoms, or a fluoro group", represented by R^{3'} in the general formula [3'] include the same groups as the specific examples of the arylene group in the "arylene group having 6 to 14 carbon atoms, which may have an alkyl group having 1 to 6 carbon atoms, an alkoxy group having 1 to 6 carbon atoms, or a halogeno group", represented by R³ in the general formula [3], and preferred specific examples and the like thereof are also the same.

Specific examples of the alkyl group having 1 to 6 carbon atoms and the alkoxy group having 1 to 6 carbon atoms in the "arylene group having 6 to 14 carbon atoms, which may have an alkyl group having 1 to 6 carbon atoms, an alkoxy group having 1 to 6 carbon atoms, or a fluoro group", represented by R^{3'} in the general formula [3'] include the same groups as the specific examples of the alkyl group and the alkoxy group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the "arylene group having 6 to 14 carbon atoms, which may have an alkyl group having 1 to 6 carbon atoms, an alkoxy group having 1 to 6 carbon atoms, or a fluoro group", represented by R^{3'} in the general formula [3'] include groups represented by the general formulae [I-II'] to [III-II']. 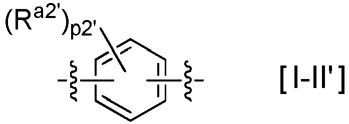 (In the general formula [I-II'], p2' pieces of R^{a2'}'s each independently represent an alkyl group having 1 to 6 carbon atoms, an alkoxy group having 1 to 6 carbon atoms, or a fluoro group, and p2' represents an integer of 0 to 4.) 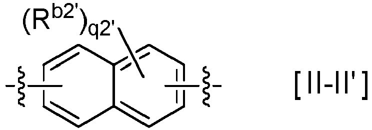 (In the general formula [II-II'], q2' pieces of R^{b2'}'s each independently represent an alkyl group having 1 to 6 carbon atoms, an alkoxy group having 1 to 6 carbon atoms, or a fluoro group, and q2' represents an integer of 0 to 6.) 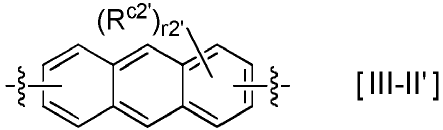 (In the general formula [III-II'], r2' pieces of R^{c2'}'s each independently represent an alkyl group having 1 to 6 carbon atoms, an alkoxy group having 1 to 6 carbon atoms, or a fluoro group, and r2' represents an integer of 0 to 8.)

Specific examples and preferred specific examples of the alkyl group having 1 to 6 carbon atoms and the alkoxy group having 1 to 6 carbon atoms, represented by R^{a2'}, R^{b2'}, and R^{c2'} in the general formulae [I-II'] to [III-II'], include the same groups as the specific examples of the alkyl group having 1 to 6 carbon atoms and the alkoxy group having 1 to 6 carbon atoms in the "arylene group having 6 to 14 carbon atoms, which may have an alkyl group having 1 to 6 carbon atoms and an alkoxy group having 1 to 6 carbon atoms, or a halogeno group", represented by R³ in the general formula [3].

p2' in the general formula [I-II'] represents an integer of 0 to 4, and among these, p2' is preferably an integer of 0 to 2, more preferably an integer of 0 or 1, and still more preferably 0.

q2' in the general formula [II-II'] represents an integer of 0 to 6, and among these, q2' is preferably an integer of 0 to 4, more preferably an integer of 0 to 2, and still more preferably 0.

r2' in the general formula [III-II'] represents an integer of 0 to 8, and among these, r2' is preferably an integer of 0 to 4, more preferably an integer of 0 to 2, and still more preferably 0.

A case where p2', q2', and r2' in the general formulae [I-II'] to [III-II'] are "0" indicates that any of an alkyl group having 1 to 6 carbon atoms, an alkoxy group having 1 to 6 carbon atoms, and a fluoro group is not contained.

In a case where p2', q2', and r2' in the general formulae [I-II'] to [III-II'] are integers of 2 or more, the plurality of R^{a2'}'s, R^{b2'}'s, and R^{c2'}'s may be the same groups or different groups.

In the general formula [I-II'], the two bonds may be bonded to any of the carbon atoms constituting the benzene ring.

In the general formula [II-II'], the two bonds may be bonded to any of the carbon atoms constituting the naphthalene ring.

In the general formula [III-II'], the two bonds may be bonded to any of the carbon atoms constituting the anthracene ring.

p2' pieces of R^{a2'}'s in the general formula [I-II'] may be bonded to any of carbon atoms on the phenylene group.

q2' pieces of R^{b2'}'s in the general formula [II-II'] may be bonded to any of carbon atoms on the naphthylene group.

r2' pieces of R^{c2'}'s in the general formula [III-II'] may be bonded to any of carbon atoms on the anthracenylene group.

Specific examples of the group represented by the general formula [I-II'] include a phenylene group, a phenylene group having an alkyl group having 1 to 6 carbon atoms, a phenylene group having an alkoxy group having 1 to 6 carbon atoms, and a phenylene group having a fluoro group. Specific examples of the phenylene group having a fluoro group include a fluorophenylene group, a difluorophenylene group, a trifluorophenylene group, and a tetrafluorophenylene group. In addition, specific examples of the phenylene group having an alkyl group having 1 to 6 carbon atoms and the phenylene group having an alkoxy group having 1 to 6 carbon atoms include the same groups of the specific example of the group represented by the general formula [I-II]. Among these groups, the phenylene group is preferable.

Specific examples of the group represented by the general formula [II-II'] include a naphthylene group, a naphthylene group having an alkyl group having 1 to 6 carbon atoms, a naphthylene group having an alkoxy group having 1 to 6 carbon atoms, and a naphthylene group having a fluoro group. Specific examples of the naphthylene group having a fluoro group include a fluoronaphthylene group, a difluoronaphthylene group, a trifluoronaphthylene group, a tetrafluoronaphthylene group, and a perfluoronaphthylene group. In addition, specific examples of the naphthylene group having an alkyl group having 1 to 6 carbon atoms and the naphthylene group having an alkoxy group having 1 to 6 carbon atoms include the same groups as the specific examples of the group represented by the general formula [II-II]. Among these groups, the naphthylene group is preferable.

Specific examples of the group represented by the general formula [III-II'] include an anthracenylene group, an anthracenylene group having an alkyl group having 1 to 6 carbon atoms, an anthracenylene group having an alkoxy group having 1 to 6 carbon atoms, and an anthracenylene group having a fluoro group. Specific examples of the anthracenylene group having a fluoro group include a fluoroanthracenylene group, a difluoroanthracenylene group, a trifluoroanthracenylene group, a tetrafluoroanthracenylene group, and a perfluoroanthracenylene group. In addition, specific examples of the anthracenylene group having an alkyl group having 1 to 6 carbon atoms and the anthracenylene group having an alkoxy group having 1 to 6 carbon atoms include the same groups as the specific examples of the group represented by the general formula [III-II]. Among these groups, the anthracenylene group is preferable.

As the groups represented by the general formulae [I-II'] to [III-II'], the groups represented by the general formulae [I-II'] and [II-II'] are preferable, and among these, the group represented by the general formula [I-II'] is more preferable.

As R^{3'} in the general formula [3'], the alkylene group having 1 to 10 carbon atoms; the fluoroalkylene group having 1 to 10 carbon atoms; the arylene group having 6 to 14 carbon atoms, which may have an alkyl group having 1 to 6 carbon atoms, an alkoxy group having 1 to 6 carbon atoms, or a fluoro group are preferable, and among these, the alkylene group having 1 to 10 carbon atoms is more preferable.

Preferred specific examples of the compound represented by the general formula [3'] include compounds represented by the following formulae.

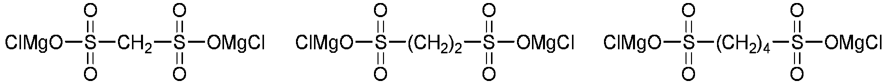

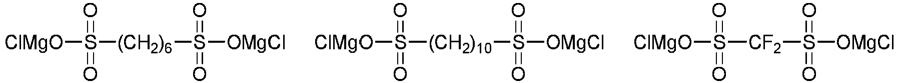

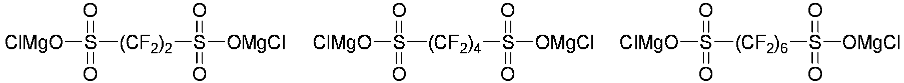

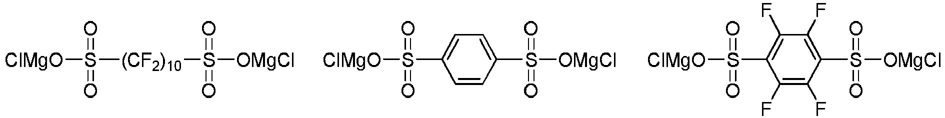

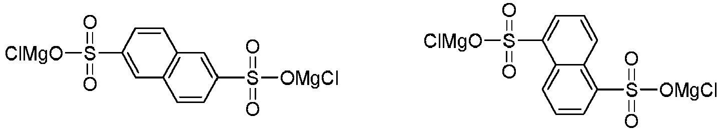

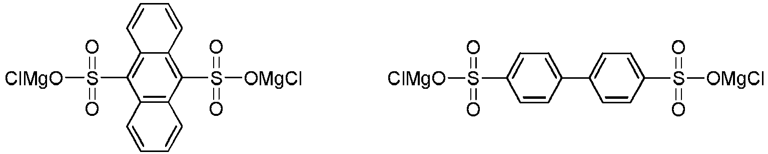

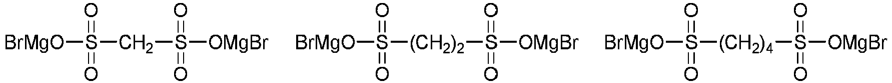

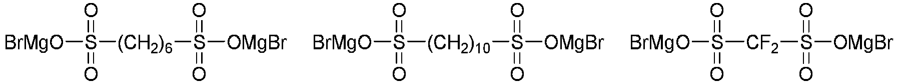

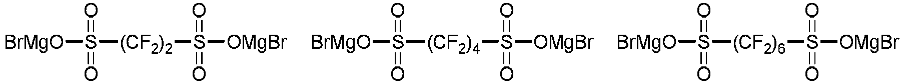

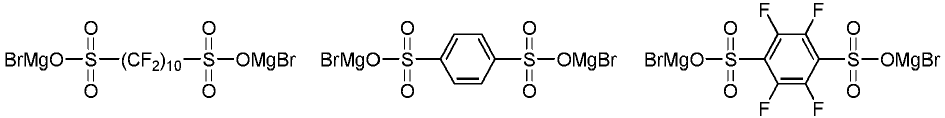

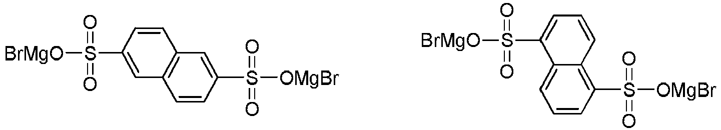

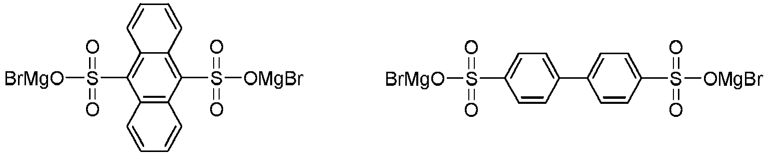

More preferred specific examples of the compound represented by the general formula [1] include compounds represented by the following formulae.

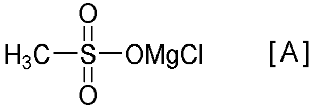

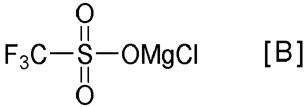

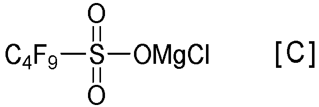

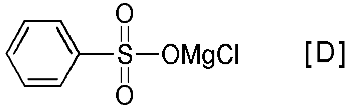

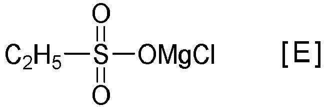

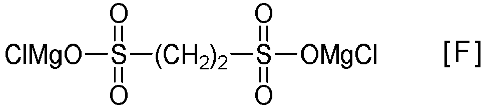

The compound represented by the general formula [1] forms a ligand in some cases. Specifically, the compound represented by the general formula [1] may form a ligand with another compound represented by the general formula [1], or may form a ligand with a solvent according to the present invention, which will be described later, in some cases. For example, it is presumed that in a case where the compound represented by the general formula [1] forms a ligand with various solvents, the following ligands are formed. In addition, with regard to the compound represented by the general formula [1], a plurality of the ligands may be aggregated to form an aggregate in some cases.

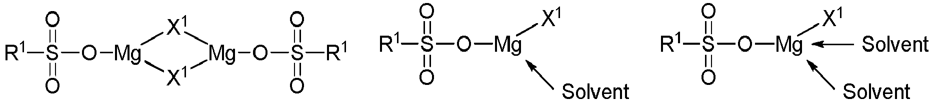

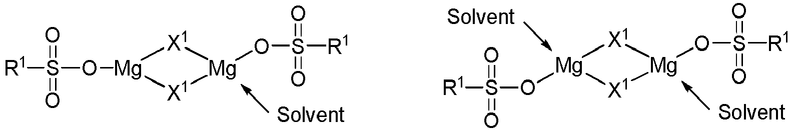

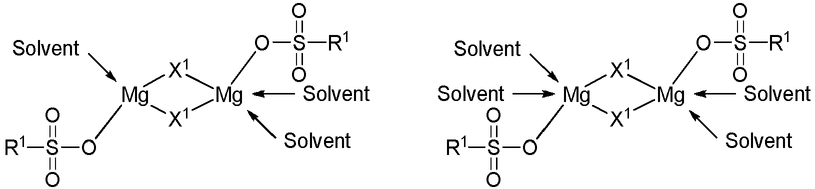

### - Method for Producing Compound Represented by General Formula [1] -

The compound represented by the general formula [1] which has been appropriately synthesized may be used, and can also be produced by the following method and the like.

The compound represented by the general formula [1] can be produced, for example, by reacting a sulfonic acid represented by the general formula [1A] with a Grignard reagent. 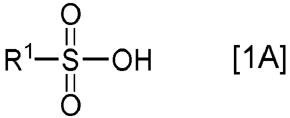 (In the general formula [1A], R¹ is the same as described above.)

Specific examples of the sulfonic acid represented by the general formula [1A] include an alkanesulfonic acid having 1 to 10 carbon atoms, such as methanesulfonic acid, ethanesulfonic acid, butanesulfonic acid, hexanesulfonic acid, and decanesulfonic acid; a fluoroalkanesulfonic acid having 1 to 10 carbon atoms, such as trifluoromethanesulfonic acid, pentafluoroethanesulfonic acid, nonafluorobutanesulfonic acid, perfluorohexanesulfonic acid, and perfluorodecanesulfonic acid; an arylsulfonic acid having 6 to 14 carbon atoms, which may have an alkyl group having 1 to 6 carbon atoms, an alkoxy group having 1 to 6 carbon atoms, or a halogeno group, such as benzenesulfonic acid, toluenesulfonic acid, ethylbenzenesulfonic acid, methoxybenzenesulfonic acid, ethoxybenzenesulfonic acid, fluorobenzenesulfonic acid, trifluorobenzenesulfonic acid, perfluorobenzenesulfonic acid, naphthalenesulfonic acid, and anthracenesulfonic acid; for example, biphenylsulfonic acid; an alkanedisulfonic acid having 1 to 10 carbon atoms, such as methanedisulfonic acid, ethanedisulfonic acid, butanedisulfonic acid, hexanedisulfonic acid, and decandisulfonic acid; a fluoroalkanedisulfonic acid having 1 to 10 carbon atoms, such as difluoromethanedisulfonic acid, tetrafluoroethanedisulfonic acid, octafluorobutanedisulfonic acid, perfluorohexanedisulfonic acid, and perfluorodecanedisulfonic acid; an aryldisulfonic acid having 6 to 14 carbon atoms, which may have an alkyl group having 1 to 6 carbon atoms, an alkoxy group having 1 to 6 carbon atoms, or a halogeno group, such as benzenedisulfonic acid, tetrafluorobenzenedisulfonic acid, naphthalenedisulfonic acid, and anthracendisulfonic acid; and for example, biphenyldisulfonic acid. As the sulfonic acid, a commercially available sulfonic acid or a sulfonic acid which has been appropriately synthesized by a known method may be used.

Examples of the Grignard reagent include a compound represented by R^{d}MgX¹ (R^{d} represents an alkyl group having 1 to 6 carbon atoms; or an aryl group having 6 to 14 carbon atoms, which may have an alkyl group having 1 to 6 carbon atoms, an alkoxy group having 1 to 6 carbon atoms, or a fluoro group, and X¹ is the same as described above); or a compound represented by R^{e}MgX² (R^{e} represents an alkyl group having 1 to 6 carbon atoms; or an aryl group having 6 to 14 carbon atoms, which may have an alkyl group having 1 to 6 carbon atoms, an alkoxy group having 1 to 6 carbon atoms, or a fluoro group, and X² is the same as described above).

Examples of the alkyl group having 1 to 6 carbon atoms, represented by R^{d} in the compound represented by R^{d}MgX¹ and R^{e} in the compound represented by R^{e}MgX², may be linear, branched, or cyclic. Specific examples of such an alkyl group include the same groups as the specific examples of the alkyl group having 1 to 6 carbon atoms, which is a preferred alkyl group in the "alkyl group having 1 to 10 carbon atoms, which may have a group represented by -SO₃MgX²", represented by R¹ in the general formula [1].

Specific examples of the alkyl group having 1 to 6 carbon atoms, the alkoxy group having 1 to 6 carbon atoms, and the aryl group having 6 to 14 carbon atoms, in the "aryl group having 6 to 14 carbon atoms, which may have an alkyl group having 1 to 6 carbon atoms, an alkoxy group having 1 to 6 carbon atoms, or a fluoro group", represented by R^{d} in the compound represented by R^{d}MgX¹ and R^{e} in the compound represented by R^{e}MgX², include the same groups as the specific examples of the alkyl group, the alkoxy group, and the aryl group in the "aryl group having 6 to 14 carbon atoms, which may have an alkyl group having 1 to 6 carbon atoms, an alkoxy group having 1 to 6 carbon atoms, a halogeno group, or a group represented by -SO₃MgX²", represented by R¹ in the general formula [1], and preferred specific examples and the like thereof are also the same.

Specific examples of the Grignard reagent include an alkylmagnesium chloride having 1 to 6 carbon atoms, such as methylmagnesium chloride, ethylmagnesium chloride, propylmagnesium chloride, butylmagnesium chloride, pentylmagnesium chloride, and hexylmagnesium chloride; an alkylmagnesium bromide having 1 to 6 carbon atoms, such as methylmagnesium bromide, ethylmagnesium bromide, propylmagnesium bromide, butylmagnesium bromide, pentylmagnesium bromide, and hexylmagnesium bromide; an arylmagnesium chloride having 6 to 14 carbon atoms, which may an alkyl group having 1 to 6 carbon atoms, an alkoxy group having 1 to 6 carbon atoms, or a fluoro group, such as phenylmagnesium chloride, tolylmagnesium chloride, ethylphenylmagnesium chloride, methoxyphenylmagnesium chloride, ethoxyphenylmagnesium chloride, fluorophenylmagnesium chloride, trifluorophenylmagnesium chloride, perfluorophenylmagnesium chloride, naphthylmagnesium chloride, and anthracenylmagnesium chloride; and an arylmagnesium bromide having 6 to 14 carbon atoms, which may an alkyl group having 1 to 6 carbon atoms, an alkoxy group having 1 to 6 carbon atoms, or a fluoro group, such as phenylmagnesium bromide, tolylmagnesium bromide, ethylphenylmagnesium bromide, methoxyphenylmagnesium bromide, ethoxyphenylmagnesium bromide, fluorophenylmagnesium bromide, trifluorophenylmagnesium bromide, perfluorophenylmagnesium bromide, naphthylmagnesium bromide, and anthracenylmagnesium bromide. Among these Grignard reagents, ethylmagnesium chloride, ethylmagnesium bromide, propylmagnesium chloride, propylmagnesium bromide, phenylmagnesium chloride, and phenylmagnesium bromide are preferable, and among these, ethylmagnesium chloride, isopropylmagnesium chloride, and phenylmagnesium chloride are more preferable. As the Grignard reagent, one kind of Grignard reagent may be used alone, or two or more kinds of Grignard reagents may be used in combination. In addition, as the Grignard reagent, a commercially available Grignard reagent or a Grignard reagent which has been appropriately synthesized by a known method may be used.

The amount of the Grignard reagent to be used in the reaction between the sulfonic acid represented by the general formula [1A] and the Grignard reagent may be determined as appropriate, depending on the number of sulfonic acid groups in the structural formula of the sulfonic acid represented by the general formula [1A]. For example, in a case where the number of sulfonic acid groups in the structural formula of the sulfonic acid represented by the general formula [1A] is 1, the amount of the Grignard reagent to be used is usually 0.8 to 1.2 mol, preferably 0.9 to 1.1 mol, and more preferably 0.95 to 1.05 mol with respect to 1 mol of the sulfonic acid represented by the general formula [1A]. For example, in a case where the number of sulfonic acid groups in the structural formula of the sulfonic acid represented by the general formula [1A] is 2, the amount of the Grignard reagent to be used is usually 1.6 to 2.4 mol, preferably 1.8 to 2.2 mol, and more preferably 1.9 to 2.1 mol with respect to 1 mol of the sulfonic acid represented by the general formula [1A]. For example, in a case where the number of sulfonic acid groups in the structural formula of the sulfonic acid represented by the general formula [1A] is 3, the amount of the Grignard reagent to be used is usually 2.4 to 3.6 mol, preferably 2.7 to 3.3 mol, and more preferably 2.85 to 3.15 mol with respect to 1 mol of the sulfonic acid represented by the general formula [1A]. For example, in a case where the number of sulfonic acid groups in the structural formula of the sulfonic acid represented by the general formula [1A] is 4, the amount of the Grignard reagent to be used is usually 3.2 to 4.8 mol, preferably 3.6 to 4.4 mol, and more preferably 3.8 to 4.2 mol with respect to 1 mol of the sulfonic acid represented by the general formula [1A].

The reaction between the sulfonic acid represented by the general formula [1A] and the Grignard reagent may be carried out in the absence of a solvent or in the presence of a solvent. Among these, the reaction is preferably carried out in the presence of a solvent. The solvent used in the reaction is not particularly limited as long as it does not inhibit the reaction between the sulfonic acid represented by the general formula [1A] and the Grignard reagent, and among these, the solvent is preferably a solvent capable of dissolving at least one of the sulfonic acid represented by the general formula [1A] or the Grignard reagent, and more preferably a solvent capable of dissolving both. Specific examples of such a solvent include aliphatic hydrocarbon-based solvents such as n-hexane, cyclohexane, n-heptane, n-octane, and isooctane; aromatic hydrocarbon-based solvents such as benzene, toluene, and xylene; and ether-based solvents such as diethyl ether, diisopropyl ether, methyl-tert-butyl ether, cyclopentyl methyl ether, ethylene glycol dimethyl ether (1,2-dimethoxyethane), diethylene glycol dimethyl ether (diglyme), triethylene glycol dimethyl ether (triglyme), tetraethylene glycol dimethyl ether (tetraglyme), tetrahydrofuran (THF), 2-methyltetrahydrofuran, and 1,4-dioxane. Among these solvents, the ether-based solvents are preferable, and diethyl ether and tetrahydrofuran (THF) are more preferable. As the solvent, one kind of solvent may be used alone, or two or more kinds of solvents may be used in combination. In addition, as the solvent, a commercially available solvent may be used.

The amount of the solvent to be used for the reaction between the sulfonic acid represented by the general formula [1A] and the Grignard reagent is usually 0.1 to 200 L, and preferably 1 to 100 L with respect to 1 mol of the sulfonic acid represented by the general formula [1A].

The reaction between the sulfonic acid represented by the general formula [1A] and the Grignard reagent is preferably carried out in an atmosphere of an inert gas such as a nitrogen gas and an argon gas, and among these, the reaction is more preferably carried out in an atmosphere of the argon gas.

The reaction between the sulfonic acid represented by the general formula [1A] and the Grignard reagent is desirably carried out in a temperature range of usually - 100°C to 30°C, and preferably -80°C to 0°C.

The reaction between the sulfonic acid represented by the general formula [1A] and the Grignard reagent is usually carried out for 5 seconds to 5 hours, and preferably carried out for 1 minute to 3 hours.

A reaction product obtained by the reaction between the sulfonic acid represented by the general formula [1A] and the Grignard reagent may be washed with a solvent such as hexane, heptane, and diisopropyl ether, as necessary, after concentration and drying.

Specifically, the compound represented by the general formula [1] can be produced by the following method. That is, the compound represented by the general formula [1A] is dissolved in an appropriate solvent such as diethyl ether and tetrahydrofuran (THF) in an argon gas atmosphere. Then, a diethyl ether solution, a tetrahydrofuran solution, or the like in which a compound represented by R^{d}MgX¹ (Grignard reagent) such as ethylmagnesium chloride, phenylmagnesium chloride, isopropylmagnesium chloride, ethylmagnesium bromide, phenylmagnesium bromide, and isopropylmagnesium bromide is dissolved, is added dropwise, or a solution in which a compound represented by R^{d}MgX¹ (Grignard reagent) is dissolved and a solution in which a compound represented by R^{e}MgX² such as ethylmagnesium chloride, phenylmagnesium chloride, isopropylmagnesium chloride, ethylmagnesium bromide, phenylmagnesium bromide, and isopropyl bromide is dissolved are both added dropwise, with the number of moles of the Grignard reagent being determined depending on the number of moles of the compound represented by the general formula [1A] and the number of sulfonic acid groups; and the reaction is carried out in a temperature range of -100°C to 80°C for 5 seconds to 5 hours. After completion of the reaction, the reaction solution is subjected to an operation for obtaining a solid, such as concentration and drying, as necessary, and a solid thus obtained is washed with a solvent such as diisopropyl ether, and dried, whereby the compound represented by the general formula [1] can be obtained.

### - Lewis Acid According to Present Invention -

The Lewis acid according to the electrolyte solution of the embodiment of the present invention (hereinafter sometimes simply referred to as the Lewis acid according to the present invention) is a Lewis acid which contains beryllium (Be), boron (B), aluminum (Al), silicon (Si), tin (Sn), titanium (Ti), chromium (Cr), iron (Fe), or cobalt (Co) as an element. Specific examples of the Lewis acid include a beryllium compound such as beryllium (II) fluoride, beryllium (II) chloride, and beryllium (II) bromide; a boron compound such as boron (III) fluoride, boron (III) chloride, boron (III) bromide, triphenoxyborane, phenyldichloroborane, and triphenylborane; an aluminum compound such as aluminum (III) chloride, aluminum (III) bromide, aluminum (III) iodide, dimethylaluminum chloride, diethylaluminum chloride, methylaluminum dichloride, ethylaluminum dichloride, trimethyl aluminum, trimethyl aluminum, and triphenyl aluminum; a silyl compound such as trimethylsilyl triflate, trimethylsilyl iodide, tertbutyldimethylsilyl triflate, and triisopropylsilyl triflate; a tin compound such as tin (II) chloride, tin (IV) chloride, tin (IV) bromide, and tin (II) triflate; a titanium compound such as titanium (IV) fluoride, titanium (IV) chloride, titanium (IV) bromide, and titanium (IV) iodide; a chromium compound such as chromium (II) fluoride, chromium (III) fluoride, chromium (II) chloride, chromium (III) chloride, chromium (II) bromide, chromium (III) bromide, chromium (II) iodide, and chromium (III) iodide; an iron compound such as iron (II) fluoride, iron (II) chloride, iron (III) chloride, iron (II) bromide, and iron (II) iodide; or a cobalt compound such as cobalt (II) fluoride, cobalt (II) chloride, cobalt (II) bromide, and cobalt (II) iodide. Among these Lewis acids, the boron compound and the aluminum compound are preferable, and the aluminum compound is more preferable. Preferred specific examples of the boron compound include boron (III) chloride. Preferred specific examples of the aluminum compound include aluminum chloride (III), methylaluminum dichloride, dimethylaluminum chloride, and triphenylaluminum. Among these preferred compounds, aluminum chloride (III) is particularly preferable. As the Lewis acid, one kind of Lewis acid may be used alone, or two or more kinds of Lewis acids may be used in combination. In addition, as the Lewis acid, a commercially available Lewis acid may be used.

### - Compound Represented by General Formula [4] -

The compound represented by the general formula [4] according to the electrolyte solution of the embodiment of the present invention (hereinafter sometimes simply referred to as the compound represented by the general formula [4] according to the present invention) is represented by the following general formula.

Mg [N(SO₂R⁴)₂]_{b} [4]

(in the general formula [4], four R⁴'s each independently represent an alkyl group having 1 to 6 carbon atoms, a perfluoroalkyl group having 1 to 6 carbon atoms, a phenyl group, or a perfluorophenyl group).

As the alkyl group having 1 to 6 carbon atoms, represented by R⁴ in the general formula [4], an alkyl group having 1 to 3 carbon atoms is preferable, and an alkyl group having 1 carbon atom is more preferable. In addition, the alkyl group may be linear, branched, or cyclic, and among these, the alkyl group is preferably linear. Specific examples of such an alkyl group include a methyl group, an ethyl group, an n-propyl group, an isopropyl group, a cyclopropyl group, an n-butyl group, an isobutyl group, a sec-butyl group, a tert-butyl group, a cyclobutyl group, an n-pentyl group, an isopentyl group, a sec-pentyl group, a tert-pentyl group, a neopentyl group, a 2-methylbutyl group, a 1,2-dimethylpropyl group, a 1-ethylpropyl group, a cyclopentyl group, an n-hexyl group, an isohexyl group, a sec-hexyl group, a tert-hexyl group, a neohexyl group, a 2-methylpentyl group, a 1,2-dimethylbutyl group, 2,3-dimethylbutyl group, a 1-ethylbutyl group, and a cyclohexyl group. Among these alkyl groups, the linear, branched, or cyclic alkyl group having 1 to 3 carbon atoms is preferable, the linear alkyl group having 1 to 3 carbon atoms is more preferable, and the methyl group is still more preferable.

As the perfluoroalkyl group having 1 to 6 carbon atoms, represented by R⁴ in the general formula [4], a perfluoroalkyl group having 1 to 3 carbon atoms is preferable, and a perfluoroalkyl group having one carbon atom is more preferable. In addition, the perfluoroalkyl group may be linear, branched, or cyclic, and among these, the perfluoroalkyl group is preferably linear. Specific examples of such a perfluoroalkyl group include a trifluoromethyl group, a pentafluoroethyl group, a heptafluoro-n-propyl group, a heptafluoroisopropyl group, a pentafluorocyclopropyl group, a nonafluoro-n-butyl group, a nonafluoroisobutyl group, a nonafluoro-sec-butyl group, a nonafluoro-tert-butyl group, a heptafluorocyclobutyl group, a perfluoro-n-pentyl group, a perfluoroisopentyl group, a perfluoro-sec-pentyl group, a perfluoro-tert-pentyl group, a perfluoroneopentyl group, a perfluoro-2-methylbutyl group, a perfluoro-1,2-dimethylpropyl group, a perfluoro-1-ethylpropyl group, a perfluorocyclopentyl group, a perfluoro-n-hexyl group, a perfluoroisohexyl group, a perfluoro-sec-hexyl group, a perfluoro-tert-hexyl group, a perfluoroneohexyl group, a perfluoro-2-methylpentyl group, a perfluoro-1,2-dimethylbutyl group, a perfluoro-2,3-dimethylbutyl group, a perfluoro-1-ethylbutyl group, and a perfluorocyclohexyl group. Among these perfluoroalkyl groups, the linear, branched, or cyclic perfluoroalkyl group having 1 to 3 carbon atoms are preferable, the linear perfluoroalkyl group having 1 to 3 carbon atoms are more preferable, and the trifluoromethyl group is still more preferable.

Four R⁴'s in the general formula [4] may be the same groups or different groups, and among these, the four R⁴'s are preferably the same groups.

As R⁴ in the general formula [4], an alkyl group having 1 to 6 carbon atoms and a perfluoroalkyl group having 1 to 6 carbon atoms is preferable, and among these, the perfluoroalkyl group having 1 to 6 carbon atoms is more preferable.

Specific examples of the compound represented by the general formula [4] include magnesium bis(alkanesulfonyl)imide having 2 to 12 carbon atoms, such as magnesium bis(methanesulfonyl)imide, magnesium bis(ethanesulfonyl)imide, magnesium bis(n-propanesulfonyl)imide, magnesium bis(isopropanesulfonyl)imide, magnesium bis(n-butanesulfonyl)imide, magnesium bis(isobutanesulfonyl)imide, magnesium bis(sec-butanesulfonyl)imide, magnesium bis(tert-butanesulfonyl)imide, magnesium bis(cyclobutanesulfonyl)imide, magnesium bis(n-pentanesulfonyl)imide, magnesium bis(isopentanesulfonyl)imide, magnesium bis(sec-pentanesulfonyl)imide, magnesium bis(tert-pentanesulfonyl)imide, magnesium bis(neopentanesulfonyl)imide, magnesium bis(2-methylbutanesulfonyl)imide, magnesium bis(1,2-dimethylpropanesulfonyl)imide, magnesium bis(1-ethylpropanesulfonyl)imide, magnesium bis(cyclopentanesulfonyl)imide, magnesium bis(n-hexanesulfonyl)imide, magnesium bis(isohexanesulfonyl)imide, magnesium bis(sec-hexanesulfonyl)imide, magnesium bis(tert-hexanesulfonyl)imide, magnesium bis(neohexanesulfonyl)imide, magnesium bis(2-methylpentanesulfonyl)imide, magnesium bis(1,2-dimethylbutanesulfonyl)imide, magnesium bis(2,3-dimethylbutanesulfonyl)imide, magnesium bis(1-ethylbutanesulfonyl)imide, and magnesium bis(cyclohexanesulfonyl)imide; magnesium bis(perfluoroalkanesulfonyl)imide having 2 to 12 carbon atoms, such as magnesium bis(trifluoromethanesulfonyl)imide, magnesium bis(pentafluoroethanesulfonyl)imide, magnesium bis(heptafluoro-n-propanesulfonyl)imide, magnesium bis(heptafluoroisopropanesulfonyl)imide, magnesium bis(nonafluoro-n-butanesulfonyl)imide, magnesium bis(nonafluoroisobutanesulfonyl)imide, magnesium bis(nonafluoro-sec-butanesulfonyl)imide, magnesium bis(nonafluoro-tert-butanesulfonyl)imide, magnesium bis(heptafluorocyclobutanesulfonyl)imide, magnesium bis(perfluoro-n-pentanesulfonyl)imide, magnesium bis(perfluoroisopentanesulfonyl)imide, magnesium bis(perfluoro-sec-pentanesulfonyl)imide, magnesium bis(perfluoro-tert-pentanesulfonyl)imide, magnesium bis(perfluoroneopentanesulfonyl)imide, magnesium bis(perfluoro-2-methylbutanesulfonyl)imide, magnesium bis(perfluoro-1,2-dimethylpropanesulfonyl)imide, magnesium bis(perfluoro-1-ethylpropanesulfonyl)imide, magnesium bis(perfluorocyclopentanesulfonyl)imide, magnesium bis(perfluoro-n-hexanesulfonyl)imide, magnesium bis(perfluoroisohexanesulfonyl)imide, magnesium bis(perfluoro-sec-hexanesulfonyl)imide, magnesium bis(perfluoro-tert-hexanesulfonyl)imide, magnesium bis(perfluoroneohexanesulfonyl)imide, magnesium bis(perfluoro-2-methylpentanesulfonyl)imide, magnesium bis(perfluoro-1,2-dimethylbutanesulfonyl)imide, magnesium bis(perfluoro-2,3-dimethylbutanesulfonyl)imide, magnesium bis(perfluoro-1-ethylbutanesulfonyl)imide, and magnesium bis(perfluorocyclohexanesulfonyl)imide; magnesium bis(phenylsulfonyl)imide; and magnesium bis(perfluorophenylsulfonyl)imide.

Among these compounds, the magnesium bis(alkanesulfonyl)imide having 2 to 12 carbon atoms and the magnesium bis(perfluoroalkanesulfonyl)imide having 2 to 12 carbon atoms are preferable, and among these, the magnesium bis(alkanesulfonyl)imide having 2 to 6 carbon atoms, such as magnesium bis(methanesulfonyl)imide and magnesium bis(ethanesulfonyl)imide, and the magnesium bis(n-propanesulfonyl)imide; and the magnesium bis(perfluoroalkanesulfonyl)imide having 2 to 6 carbon atoms, such as magnesium bis(trifluoromethanesulfonyl)imide, the magnesium bis(pentafluoroethanesulfonyl)imide, and the magnesium bis(heptafluoro-n-propanesulfonyl)imide are more preferable, the magnesium bis(trifluoromethanesulfonyl)imide and the magnesium bis(pentafluoroethanesulfonyl)imide are still more preferable, and the magnesium bis(trifluoromethanesulfonyl)imide is particularly preferable.

### - Solvent According to Present Invention -

As the solvent according to the electrolyte solution of the embodiment of the present invention (hereinafter sometimes simply referred to as the solvent according to the present invention), a solvent capable of dissolving the compound represented by the general formula [1] according to the present invention and/or the Lewis acid according to the present invention or the compound represented by the general formula [4] according to the present invention is preferable. Examples of such a solvent include an ether-based solvent, a halogenated hydrocarbon-based solvent, a carbonate-based solvent, a nitrile-based solvent, and a sulfone-based solvent. As the solvent, one kind of solvent may be used alone, or two or more kinds of solvents may be used in combination. In addition, as the solvent, a commercially available solvent may be used.

Specific examples of the ether-based solvent include diethyl ether, diisopropyl ether, methyl-tert-butyl ether, cyclopentyl methyl ether, ethylene glycol dimethyl ether (1,2-dimethoxyethane), diethylene glycol dimethyl ether (diglyme), triethylene glycol dimethyl ether (triglyme), tetraethylene glycol dimethyl ether (tetraglyme), tetrahydrofuran (THF), 2-methyltetrahydrofuran, and 1,4-dioxane. Specific examples of the halogenated hydrocarbon-based solvent include dichloromethane, trichloromethane (chloroform), tetrachloromethane (carbon tetrachloride), and 1,2-dichloroethane. Specific examples of the carbonate-based solvent include dimethyl carbonate, diethyl carbonate, ethyl methyl carbonate, and propylene carbonate. Specific examples of the nitrile-based solvent include acetonitrile, propionitrile, butyronitrile, succinonitrile, pimeronitrile, and methoxypropionitrile. Specific examples of the sulfone-based solvent include sulfolane, dimethylsulfone, ethyl methylsulfone, methyl-n-propylsulfone, methyl isopropylsulfone, n-butyl-methylsulfone, isobutyl methylsulfone, sec-butyl methylsulfone, and tert-butyl methylsulfone, diethylsulfone, ethyl-n-propylsulfone, ethyl isopropylsulfone, n-butyl ethylsulfone, isobutyl ethylsulfone, sec-butyl ethylsulfone, tert-butyl ethylsulfone, di-n-propylsulfone, diisopropylsulfone, n-butyl-n-propylsulfone, and di-n-butylsulfone.

Among these solvents according to the present invention, the ether-based solvent and the sulfone-based solvent are preferable, and the ether-based solvent is more preferable. Preferred specific examples of the ether-based solvent include ethylene glycol dimethyl ether (1,2-dimethoxyethane), diethylene glycol dimethyl ether (diglyme), triethylene glycol dimethyl ether (triglyme), tetraethylene glycol dimethyl ether (tetraglyme), tetrahydrofuran (THF), and 1,4-dioxane. Preferred specific examples of the sulfone-based solvent include sulfolane. Among these preferred solvents, diethylene glycol dimethyl ether (diglyme), triethylene glycol dimethyl ether (triglyme), tetraethylene glycol dimethyl ether (tetraglyme), tetrahydrofuran (THF), and 1,4-dioxane are more preferable, triethylene glycol dimethyl ether (triglyme) is still more preferable, and tetrahydrofuran (THF) are particularly preferable.

In a case where two or more kinds of solvents according to the present invention are used in combination, it is preferable to use two or more kinds of ether-based solvents in combination. Specific examples of such a combination include a combination of triethylene glycol dimethyl ether (triglyme) and tetrahydrofuran (THF).

### - Electrolyte Solution of Embodiment of Present Invention -

The electrolyte solution of the embodiment of the present invention is a solution obtained by mixing the compound represented by the general formula [1] of the embodiment of the present invention and the Lewis acid according to the present invention or the compound represented by the general formula [4] according to the present invention in the solvent according to the present invention.

The concentration of the compound represented by the general formula [1] in the electrolyte solution of the embodiment of the present invention is usually 0.01 to 5 mol/L, preferably 0.05 to 3 mol/L, and more preferably 0.1 to 1 mol/L.

The content of the Lewis acid or the compound represented by the general formula [4] in the electrolyte solution of the embodiment of the present invention may be determined as appropriate, depending on the total number of the -OMgX¹ groups and the -OMgX² groups in the structural formula of the compound represented by the general formula [1]. For example, in a case where the total number of the -OMgX¹ groups and the -OMgX² groups in the structural formula of the compound represented by the general formula [1] is 1, the content of the Lewis acid or the compound represented by the general formula [4] is usually 0.1 to 6 mol, preferably 0.3 to 3 mol, and more preferably 0.5 to 2.5 mol with respect to 1 mol of the compound represented by the general formula [1]. For example, in a case where the total number of the - OMgX¹ groups and the -OMgX² groups in the structural formula of the compound represented by the general formula [1] is 2, the content of the Lewis acid or the compound represented by the general formula [4] is usually 0.2 to 12 mol, preferably 0.6 to 6 mol, and more preferably 1 to 5 mol with respect to 1 mol of the compound represented by the general formula [1]. For example, in a case where the total number of the -OMgX¹ groups and the -OMgX² groups in the structural formula of the compound represented by the general formula [1] is 3, the content of the Lewis acid or the compound represented by the general formula [4] is usually 0.3 to 18 mol, preferably 0.9 to 9 mol, and more preferably 1.5 to 7.5 mol with respect to 1 mol of the compound represented by the general formula [1]. For example, in a case where the total number of the -OMgX¹ groups and the -OMgX² groups in the structural formula of the compound represented by the general formula [1] is 4, the content of the Lewis acid or the compound represented by the general formula [4] is usually 0.4 to 24 mol, preferably 1.2 to 12 mol, and more preferably 2 to 10 mol with respect to 1 mol of the compound represented by the general formula [1].

The electrolyte solution of the embodiment of the present invention may contain additives such as a film forming agent, an overcharge inhibitor, an oxygen scavenger, a dehydrating agent, and a flame retardant, coordinating additives such as a crown ether, and ionic additives such as lithium chloride and tetrabutylammonium chloride, which are usually used in the field, in addition to the constituent components.

The electrolyte solution of the embodiment of the present invention can be used in a magnesium battery such as a primary battery and a secondary battery, and in a case where the electrolyte solution is used in a magnesium secondary battery, it exhibits high oxidative stability and can be used stably and repeatedly.

The electrolyte solution of the embodiment of the present invention is a solution produced by mixing or dissolving the compound represented by the general formula [1] according to the present invention and the Lewis acid according to the present invention or the compound represented by the general formula [4] according to the present invention in the solvent according to the present invention. That is, the electrolyte solution can be produced using the compound represented by the general formula [1], and the above-mentioned number of moles of the Lewis acid according to the present invention or the compound represented by the general formula [4] according to the present invention, depending on the number of moles of the compound represented by the general formula [1] according to the present invention and the total number of - OMgX¹ groups and -OMgX² groups, in which the compound represented by the general formula [1], and the Lewis acid or the compound represented by the general formula [4] are added to the solvent according to the present invention, and mixed or dissolved therein so that the concentration range is as described above. It should be noted that the solvent (mixed solution) may be heated or cooled so that the temperature range is -78°C to 300°C as necessary during mixing or dissolution, and among these, it is preferable that the solvent (mixed solution) may be heated or cooled so that the temperature range is 0°C to 150°C. In addition, after the operation of mixing or dissolution, a filtration treatment may be performed as necessary.

The electrolyte solution of the embodiment of the present invention only needs to contain the compound represented by the general formula [1] according to the present invention, the Lewis acid according to the present invention or the compound represented by the general formula [4] according to the present invention, and the solvent according to the present invention, and for example, a ligand formed by the compound represented by the general formula [1] and the solvent according to the present invention is also encompassed within the present invention. That is, the electrolyte solution of the embodiment of the present invention is not limited to a mixture of the compound represented by the general formula [1], the Lewis acid or the compound represented by the general formula [4], and the solvent, but also encompasses an electrolyte solution obtained by mixing a ligand formed of the compound represented by the general formula [1] and the solvent, the Lewis acid or the compound represented by the general formula [4], and the solvent, as necessary.

In a case where the compound represented by the general formula [1] forms a ligand, the amounts of the Lewis acid according to the present invention or the compound represented by the general formula [4] according to the present invention, and the solvent according to the present invention to be used only need to be determined depending on the amount (number of moles) of the compound represented by the general formula [1] constituting the ligand.

### - Electrochemical Device of Embodiment of Present Invention -

The electrochemical device of an embodiment of the present invention has a positive electrode, a negative electrode, and the electrolyte solution of the embodiment of the present invention. It should be noted that in the present invention, any of those involving a magnesium reaction at a negative electrode is encompassed within a "magnesium battery" in the electrolyte solution (electrolyte solution for a magnesium battery) of the embodiment of the present invention. Examples of the reaction of magnesium include dissolution-precipitation of magnesium at an interface between a magnesium metal and an electrolyte solution, a reaction in which magnesium ions are intercalated in a carbon-based material, a reaction in which an element such as bismuth and magnesium is alloyed, and a reaction in which the magnesium ions are occluded·released onto a battery material such as titanium oxide at a low potential such as 1 V or less, for example. Examples of the type of magnesium battery include a primary battery, a secondary battery, an air battery, and an electric double layer capacitor, and among these, the secondary battery is preferable.

Examples of the positive electrode in the electrochemical device of the embodiment of the present invention include a positive electrode containing a current collector, and a current collector coating layer derived from a positive electrode active material, a conductive auxiliary agent, and a binder. That is, examples of the positive electrode include a positive electrode having a current collector, and an active material layer formed of a positive electrode active material, a conductive auxiliary agent, and a binder on a surface of the positive electrode.

As the current collector, known current collectors which are usually used in this field can be used. Specific examples of such a current collector include current collectors which are constituted with conductive materials such as platinum, copper, stainless steel (SUS), hastelloy, aluminum, iron, chromium, nickel, titanium, inconel, molybdenum, graphite, and carbon, and have shapes of a plate, a foil, a mesh, an expanded grid (expanded metal), a punched metal, and the like. The mesh opening, the wire diameter, the number of the meshes, and the like of the current collector are not particularly limited. The thickness of the current collector is not particularly limited, and above all, it is preferably from 1 µm to 300 µm. The size of the current collector is determined in accordance with the applications where a battery is used. In a case of manufacturing a large size electrode to be used in a large size battery, the current collector having a large area is used, and in a case of manufacturing a small size electrode, the current collector having a small area is used.

Specific examples of the positive electrode active material include cobalt, manganese, vanadium, aluminum, iron, silicon, phosphorus, nickel, molybdenum, titanium, tungsten, ruthenium, copper, chromium, lithium, sodium, potassium, rubidium, cesium, beryllium, calcium, strontium, barium, niobium, lanthanoid elements, carbon, sulfur, magnesium, platinum, hafnium, scandium, zirconium, osmium, iridium, gold, mercury, thallium, lead, tin, and antimony; oxides, sulfides, selenides, tellurides, cyanides, halides, borides, silicon oxides, phosphorus oxides, boron oxides, manganese oxides, and sulfates thereof; and double salts thereof. Among these active materials, molybdenum; oxides, sulfides, selenides, tellurides, cyanides, halides, borides, silicon oxides, phosphorus oxides, boron oxides, manganese oxides, and sulfates of molybdenum; or double salts thereof are preferable. It should be noted that the active material may be configured by combination of a plurality of metals and a plurality of compounds, may be doped with a metal such as magnesium and potassium, sulfur, boron, phosphorus, or the like, or may be a hydrate.

Specific examples of the active material mainly composed of the metal as described above include active materials constituted with Mo₆S₈, V₂O₅, MnO₂, Mn₂O₃, Mn₃O₄, RuO₂, TiO₂, Co₃O₄, MoO₃, Co₃O₄, CoO, CoO₂, WO₃, PbO₂, Pb₃O₄, NiFe(CN)₆, CuFe(CN)₆, Ni[Fe(CN)₆]_{0.7}·4.7H₂O, Cu[Fe(CN)₆]_{0.7}·3.6H₂O, MgMo₆S₈, MgVPO₄F, MgFePO₄F, MgMnPO₄F, MgFePO₄, Mg_{0.1}V₂O₅, MgNiO₂, MgCoO₂, MgCo₂O₄, TiNb₂O₇, Mg_{0.5}Hf_{0.5}Sc_{1.0}(MoO₄)₃, MgZrWO₄, MgFe₂O₄, MgMn₂O₄, MgNi₂O₄, MgCr₂O₄, MgCoSiO₄, MgFeSiO₄, MgNiSiO₄, MgMnSiO₄, MgNi_{0.9}Mn_{0.1}SiO₄, MgVSiO₄, MgCuSiO₄, Mg_{1.03}Mn_{0.97}SiO₄, MgMnNiO₄, MgMn_{1.15}Ni_{0.6}Ti_{0.25}O₄, Mg_{0.75}Fe_{0.25}Ca(SiO₃)₂, Mg_{0.59}Co_{0.41}CaSiO, Mg_{0.71}Fe_{1.29}(SiO₃)₂, Mg_{1.88}Fe_{0.12}(SiO₃)₂, MgAg_{0.5}Fe_{0.95}Nb_{0.05}O₄, Mg₂SiO₄, KMnO₄, Mg(MnO₄)₂, NiCI2, CoCl₂, FeCI2, CrCI2, FeF₃, MnF₃, LaF₃, NiS, FeS, CuS, CoS, ZrS₂, WS₂, CoS₂, MoS₂, MnS₂, NbS₂, NbS₃, TiS₂, TiB₂, ZrB₂, MoB₂, VS₂, WSe₂, Cu₂Se, Mo₉Se₁₁, NiSSe, VBO₃, TiBO₃, MnBO₃, CoBO₃, V_{0.5}Fe_{0.5}BO₃, V_{0.5}Ti_{0.5}BO₃, V_{0.5}Ti_{0.3}Fe_{0.2}BO₃, and V₂O₅·MgCl₂·P₂O₅; a salt of a metal cation such as Fe²⁺, Cu²⁺, Ti²⁺, and Cd²⁺ with a polysulfide; boron (B)-doped Mg₂SiO₄; a complex of TiNb₂O₇ and graphene; an active material having an amorphous structure, synthesized from KMnO₄ and hydrochloric acid; a carbon complex of MoS₂ and V₂O₅; V₂O₅ in the form of an aerogel; Mn₃O₄ in the form of a nanowire; MnO₂ containing water of crystallization; porous Mn₃O₄ nanoparticles; TiS₂ in the form of a nanotube; potassium-doped K-αMnO₂; and Prussian blue analogs.

In addition, as the positive electrode active material, for example, sulfur; an organic sulfur compound; a radical compound; an organic compound; a polymer compound; a sulfur-containing polymer compound; a radical polymer; a functional compound; a material forming an electric double layer such as layered carbon, porous carbon, and activated carbon; or the like may be used. Such the material may contain magnesium in an oxidized form or in a form in mixture with sulfur, phosphorus, boron, or the like. In addition, the material may also be in a partially halogenated form.

Specific examples of the active material mainly composed of the organic material as described above include Rubeanic acid, 2,5-dimercapto-1,3,4-thiadiazole (DMcT), trioxotriangulene, 2,2,6,6-tetramethyl-1-piperidinyloxy (TEMPO), 4-hydroxy-2,2,6,6-tetramethylpiperidinyloxy (4-hydroxy-TEMPO), dimethoxybenzoquinone (DMBQ), 9,10-anthraquinone, porphyrin, magnesium porphyrin, phthalocyanine, magnesium phthalocyanine, magnesium anthracene, polyaniline, a polyquinone derivative, a quinone-based polymer, poly(hydroquinoyl-benzoquinonyl sulfide), carbyne polysulfide, poly-2,2'-dithiodianiline (PDTDA), poly(4-methacryloyloxy-2,2,6,6-tetramethylpiperidin-N-oxyl) (PTMA), poly(anthraquinonyl)sulfide, fullerene, S-BUMB18C6, S-UOEE, graphite fluoride, graphite fluoride mixed with copper, and graphene fluoride.

The crystal structure of the positive electrode active material may be any of structures, and may also be an amorphous structure. In addition, the crystal structure may be changed by a physical or chemical treatment or by occluding·releasing ions in an electrolyte solution, and may be a part, not the whole. In addition, the positive electrode active material may have a structure in which the position of a peak is changed in X-ray crystal structure analysis by a change in the crystal structure, and may be the one which is treated as confirmed for a change in XPS.

The particle size of the positive electrode active material is not particularly limited, and among these, it is preferably 10 µm or less, and more preferably 5 µm or less.

As the positive electrode active material, one kind of active material may be used alone, or two or more kinds of active materials may be used in combination.

As the conductive auxiliary agent, known conductive auxiliary agents which are usually used in this field can be used. Specific examples of such a conductive auxiliary agent include carbon blacks such as acetylene black, ketjen black, furnace black, and thermal black, and among these, acetylene black is preferable.

As the binder, known binders which are usually used in this field can be used. Specific examples of such a binder include a binder consisting of polyvinylidene fluoride (PVDF), polytetrafluoroethylene (PTFE), polyamide, polyacrylic acid, and the like.

The contents of the positive electrode active material, the conductive auxiliary agent, and the binder contained in the current collector coating layer (active material layer) may be appropriately set according to the amounts usually used in this field.

As the current collector, the positive electrode active material, the conductive auxiliary agent, and the binder, those that are commercially available or those that have been appropriately synthesized by a known method may be used.

In addition to the positive electrode active material, the conductive auxiliary agent, and the binder, other materials such as a supporting salt, an ion conductive polymer, and a binder polymer may be contained in the current collector coating layer (active material layer). Examples of the supporting salt include Li(C₂F₅SO₂)₂N(LiBETl), LiPF₆, LiBF₄, LiClO₄, LiAsF₆, and LiCF₃SO₃. Examples of the ion conductive polymer include a polyethylene oxide (PEO)-based polymer and a polypropylene oxide (PPO)-based polymer. Specific examples thereof include binder polymers such as polyvinylidene fluoride (PVDF), polytetrafluoroethylene (PTFE), carboxymethyl cellulose (CMC), a styrene-butadiene copolymer (SBR), an acrylonitrile-butadiene copolymer (NBR), polyacrylonitrile (PAN), an ethylene-vinyl alcohol copolymer (EVOH), polyurethane, polyacrylate, polyvinyl ether, and polyimide. The content of the material contained in the current collector coating layer (active material layer) may be appropriately set according to the amount usually used in this field. In addition, as the material, a commercially available material may be used.

A method for producing the positive electrode may be in accordance with a method known per se.

The negative electrode in the electrochemical device of the embodiment of the present invention is not particularly limited as long as it can be reacted with magnesium. Specific examples of such a negative electrode include those consisting of a material capable of occluding·releasing magnesium or magnesium ions such as metallic magnesium and magnesium alloy, and among these, metallic magnesium is preferable.

Specific examples of the magnesium alloy include an Mg-Bi alloy, an Mg-Sb alloy, an Mg-In alloy, an Mg-Zn alloy, an Mg-Zr alloy, an Mg-Sn alloy, an Mg-Cd alloy, an Mg-Co alloy, an Mg-Mn alloy, an Mg-Ga alloy, an Mg-Pb alloy, an Mg-Ni alloy, an Mg-Cu alloy, an Mg-AI alloy, an Mg-Ca alloy, an Mg-Li alloy, an Mg-Bi-Sb alloy, an Mg-AI-Zn alloy, an Mg-Zn-Zr alloy, an Mg-In-Ni alloy, and other alloys containing rare earth elements.

The negative electrode in the electrochemical device of the embodiment of the present invention may contain a current collector, an active material, a conductive auxiliary agent, a binder, a supporting salt, and/or an ion conductive polymer, as necessary, in addition to the material capable of occluding·releasing magnesium ions. Specifically, a material capable of occluding·releasing magnesium ions may be used as the negative electrode itself, and in a case where the negative electrode has a current collector and a negative electrode material layer formed on a surface thereof, a material capable of occluding·releasing magnesium ions, and as necessary, an active material, a conductive auxiliary agent, a binder, a supporting salt, and/or an ion conductive polymer may be used in the negative electrode material layer. Among these, it is preferable that the material capable of occluding·releasing magnesium ions is used as the negative electrode itself. The current collector, the active material, the conductive auxiliary agent, the binder, the supporting salt, and the ion conductive polymer which can be contained in the negative electrode include the same ones as those that can be contained in the positive electrode, and preferred ones thereof are also the same.

The electrochemical device of the embodiment of the present invention may further have a separator, in addition to the positive electrode, the negative electrode, and the electrolyte solution of the embodiment of the present invention. The separator is not particularly limited as long as it electrically insulates a positive electrode and a negative electrode, and allows magnesium ions to pass therethrough. Specific examples of such a separator include glass fiber, porous polyethylene, porous polypropylene, porous polyolefin, and microporous polymers such as those having a multi-layer structure in which porous polyethylene and porous polypropylene are laminated.

### EXAMPLES

Hereinafter, the present invention will be specifically described based on Examples and Comparative Examples, but the present invention is not limited to these Examples.

### Example 1: Preparation of Electrolyte Solution 1

### (1) Synthesis of Magnesium Salt

In an argon gas atmosphere, 2.88 g (30 mmol) of methanesulfonic acid (manufactured by FUJIFILM Wako Pure Chemical Corporation) was added to 45 mL of tetrahydrofuran (THF) (manufactured by FUJIFILM Wako Pure Chemical Corporation). 15 mL (30 mmol) of a THF solution of phenylmagnesium chloride (PhMgCl) having a concentration of 2 M (manufactured by Tokyo Chemical Industry Co., Ltd.) was added dropwise to the obtained THF solution at -60°C to -70°C, and stirred at -60°C for 30 minutes. After elevating the temperature of the reaction solution to room temperature, 40 mL of diisopropyl ether (manufactured by FUJIFILM Wako Pure Chemical Corporation) was added to a solid produced by concentrating the reaction solution under reduced pressure, and the mixture was stirred at room temperature for 1 hour. A white solid thus produced was collected by filtration and dried under reduced pressure at 80°C overnight to obtain a methanesulfonic acid magnesium chloride salt (CH₃S(O)₂OMgCl).

The measurement results of ¹H-NMR of the methanesulfonic acid as a raw material and the methanesulfonic acid magnesium chloride salt as a desired product are shown below, together with the structural formula of the methanesulfonic acid magnesium chloride salt.

Measurement results of ¹H-NMR of methanesulfonic acid:
¹H-NMR (400 MHz, DMSO-d6) δ (ppm): 2.46 (s, 3H, CH₃), 14.66 (s, 1H, OH).

Measurement results of ¹H-NMR of methanesulfonic acid magnesium chloride salt:
¹H-NMR (400 MHz, DMSO-d6) δ (ppm): 2.33 (s, 3H, CH₃).

From the measurement results of ¹H-NMR, it was confirmed that the obtained methanesulfonic acid magnesium chloride salt has a structure different from that of the methanesulfonic acid.

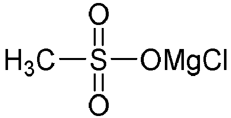

### (2) Preparation of Electrolyte Solution

In an argon gas atmosphere, 0.85 g (3.75 mmol) of the methanesulfonic acid magnesium chloride salt (CH₃S(O)₂OMgCl) obtained in (1) was added to 15 mL of triethylene glycol dimethyl ether (manufactured by FUJIFILM Wako Pure Chemical Corporation), and then 1.00 g (7.50 mmol) of aluminum chloride (AlCl₃) (manufactured by FUJIFILM Wako Pure Chemical Corporation) was added thereto. A solution thus obtained was stirred at 80°C for 4 hours and then cooled to room temperature to obtain an electrolyte solution 1 [methanesulfonic acid magnesium chloride salt-aluminum chloride / triethylene glycol dimethyl ether solution].

### Example 2: Preparation of Electrolyte Solution 2

In an argon gas atmosphere, 0.85 g (3.75 mmol) of a methanesulfonic acid magnesium chloride salt (CH₃S(O)₂OMgCl) obtained in (1) of Example 1 was added to a mixed solvent of 11.75 mL of triethylene glycol dimethyl ether (manufactured by FUJIFILM Wako Pure Chemical Corporation) and 2.25 mL of 1,4-dioxane, and then 1.00 g (7.50 mmol) of aluminum chloride (AlCl₃) (manufactured by FUJIFILM Wako Pure Chemical Corporation) was added thereto. A solution thus obtained was stirred at 80°C for 1 hour and then cooled to room temperature to obtain an electrolyte solution 2 [methanesulfonic acid magnesium chloride salt-aluminum chloride / triethylene glycol dimethyl ether-1,4-dioxane solution].

### Example 3: Preparation of Electrolyte Solution 3

### (1) Synthesis of Magnesium Salt

In an argon gas atmosphere, 7.50 g (50 mmol) of trifluoromethanesulfonic acid (manufactured by FUJIFILM Wako Pure Chemical Corporation) was added to 75 mL of tetrahydrofuran (THF) (manufactured by FUJIFILM Wako Pure Chemical Corporation). 25 mL (50 mmol) of a THF solution of phenylmagnesium chloride (PhMgCl) having a concentration of 2 M (manufactured by Tokyo Chemical Industry Co., Ltd.) was added dropwise to the obtained THF solution at -30°C, and stirred at -30°C for 30 minutes. After elevating the temperature of the reaction solution to room temperature, 60 mL of diisopropyl ether (manufactured by FUJIFILM Wako Pure Chemical Corporation) was added to a solid produced by concentrating the reaction solution under reduced pressure, and the mixture was stirred at room temperature for 1 hour. A white solid thus produced was collected by filtration and dried under reduced pressure at 80°C overnight to obtain a trifluoromethanesulfonic acid magnesium chloride salt (CF₃S(O)₂OMgCl).

The measurement results of ¹H-NMR and ¹⁹F-NMR of the trifluoromethanesulfonic acid as a raw material and the trifluoromethanesulfonic acid magnesium chloride salt as a desired product are shown below, together with the structural formula of the trifluoromethanesulfonic acid magnesium chloride salt.

Measurement results of ¹H-NMR and ¹⁹F-NMR of trifluoromethanesulfonic acid:
¹H-NMR (400 MHz, DMSO-d6) δ (ppm): 13.92-14.04 (m, 1H, OH).
¹⁹F-NMR (400 MHz, DMSO-d6) δ (ppm): -77.68 (s, 3F, CF₃).

Measurement results of ¹H-NMR and ¹⁹F-NMR of trifluoromethanesulfonic acid magnesium chloride salt:
¹H-NMR (400 MHz, DMSO-d6) δ (ppm): No signal caused by the raw material was observed.
¹⁹F-NMR (400 MHz, DMSO-d6) δ (ppm): -77.65 (s, 3F, CF₃).

From the measurement results of ¹H-NMR and ¹⁹F-NMR, it was confirmed that the obtained trifluoromethanesulfonic acid magnesium chloride salt has a structure different from that of the trifluoromethanesulfonic acid.

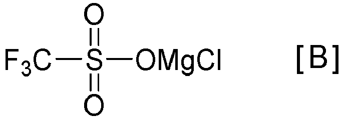

### (2) Preparation of Electrolyte Solution

In an argon gas atmosphere, 1.05 g (3.75 mmol) of the trifluoromethanesulfonic acid magnesium chloride salt (CF₃S(O)₂OMgCl) obtained in (1) was added to 15 mL of triethylene glycol dimethyl ether (manufactured by FUJIFILM Wako Pure Chemical Corporation), and then 0.50 g (3.75 mmol) of aluminum chloride (AlCl₃) (manufactured by FUJIFILM Wako Pure Chemical Corporation) was added thereto. A solution thus obtained was stirred at 80°C for 1 hour and then cooled to room temperature to obtain an electrolyte solution 3 [trifluoromethanesulfonic acid magnesium chloride salt-aluminum chloride / triethylene glycol dimethyl ether solution].

### Example 4: Preparation of Electrolyte Solution 4

In an argon gas atmosphere, 1.05 g (3.75 mmol) of the trifluoromethanesulfonic acid magnesium chloride salt (CF₃S(O)₂OMgCl) obtained in (1) of Example 3 was added to 15 mL of tetrahydrofuran (manufactured by FUJIFILM Wako Pure Chemical Corporation), and then 0.50 g (3.75 mmol) of aluminum chloride (AlCl₃) (manufactured by FUJIFILM Wako Pure Chemical Corporation) was added thereto. A solution thus obtained was stirred at 60°C for 10 minutes and then cooled to room temperature to obtain an electrolyte solution 4 [trifluoromethanesulfonic acid magnesium chloride salt-aluminum chloride / tetrahydrofuran solution].

### Example 5: Preparation of Electrolyte Solution 5

### (1) Synthesis of Magnesium Salt

In an argon gas atmosphere, 10.00 g (33.3 mmol) of nonafluoro-1-butanesulfonic acid (manufactured by Tokyo Chemical Industry Co., Ltd.) was added to 42 mL of diethyl ether (Et₂O) (manufactured by FUJIFILM Wako Pure Chemical Corporation). 33.3 mL (33.3 mmol) of a diethyl ether solution of isopropylmagnesium chloride (i-C₃H₇MgCl) having a concentration of 1 M (manufactured by Tokyo Chemical Industry Co., Ltd.) was added dropwise to the obtained diethyl ether solution at -40°C, and stirred at -20°C for 30 minutes. After elevating the temperature of the reaction solution to room temperature, 40 mL of diisopropyl ether (manufactured by FUJIFILM Wako Pure Chemical Corporation) was added to a solid produced by concentrating the reaction solution under reduced pressure, and the mixture was stirred at room temperature for 1 hour. A white solid thus produced was collected by filtration and dried under reduced pressure at 80°C overnight to obtain a nonafluoro-1-butanesulfonic acid magnesium chloride salt (n-C₄F₉S(O)₂OMgCl).

The measurement results of ¹H-NMR and ¹⁹F-NMR of the nonafluoro-1-butanesulfonic acid as a raw material and the nonafluoro-1-butanesulfonic acid magnesium chloride salt as a desired product are shown below, together with the structural formula of the nonafluoro-1-butanesulfonic acid magnesium chloride salt.

Measurement results of ¹H-NMR and ¹⁹F-NMR of nonafluoro-1-butanesulfonic acid:
¹H-NMR (400 MHz, DMSO-d6) δ (ppm): 12.84 (s, 1H, OH).
¹⁹F-NMR (400 MHz, DMSO-d6) δ (ppm): -80.46 (m, 3F, CF₃), -114.76 (m, 2F, CF₂), - 121.33 (m, 2F, CF₂), -125.65 (m, 2F, CF₂).

Measurement results of ¹H-NMR and ¹⁹F-NMR of nonafluoro-1-butanesulfonic acid magnesium chloride salt:
¹H-NMR (400 MHz, DMSO-d6) δ (ppm): No signal caused by the raw material was observed.
¹⁹F-NMR (400 MHz, DMSO-d6) δ (ppm): -80.32 (m, 3F, CF₃), -114.76 (m, 2F, CF₂), - 121.33 (m, 2F, CF₂), -125.65 (m, 2F, CF₂).

From the measurement results of ¹H-NMR and ¹⁹F-NMR, it was confirmed that the obtained nonafluoro-1-butanesulfonic acid magnesium chloride salt has a structure different from that of the nonafluoro-1-butanesulfonic acid.

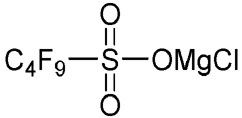

### (2) Preparation of Electrolyte Solution

In an argon gas atmosphere, 1.08 g (2.50 mmol) of the nonafluoro-1-butanesulfonic acid magnesium chloride salt (n-C₄F₉S(O)₂OMgCl) obtained in (1) was added to 10 mL of triethylene glycol dimethyl ether (manufactured by FUJIFILM Wako Pure Chemical Corporation), and then 0.67 g (5.00 mmol) of aluminum chloride (AlCl₃) (manufactured by FUJIFILM Wako Pure Chemical Corporation) was added thereto. A solution thus obtained was stirred at 80°C for 1 hour and then cooled to room temperature to obtain an electrolyte solution 5 [nonafluoro-1-butanesulfonic acid magnesium chloride salt-aluminum chloride / triethylene glycol dimethyl ether solution].

### Example 6: Preparation of Electrolyte Solution 6

### (1) Synthesis of Magnesium Salt

In an argon gas atmosphere, 7.12 g (45.0 mmol) of benzenesulfonic acid (manufactured by Tokyo Chemical Industry Co., Ltd.) was added to 68 mL of tetrahydrofuran (THF) (manufactured by FUJIFILM Wako Pure Chemical Corporation). 22.5 mL (45.0 mmol) of a THF solution of phenylmagnesium chloride (PhMgCl) having a concentration of 2 M (manufactured by Tokyo Chemical Industry Co., Ltd.) was added dropwise to the obtained THF solution at -60°C, and stirred at -60°C for 30 minutes. After elevating the temperature of the reaction solution to room temperature, 60 mL of diisopropyl ether (manufactured by FUJIFILM Wako Pure Chemical Corporation) was added to a solid produced by concentrating the reaction solution under reduced pressure, and the mixture was stirred at room temperature for 1 hour. A white solid thus produced was collected by filtration and dried under reduced pressure at 80°C overnight to obtain a benzenesulfonic acid magnesium chloride salt (C₆H₅S(O)₂OMgCl).

The measurement results of ¹H-NMR of the benzenesulfonic acid as a raw material and the benzenesulfonic acid magnesium chloride salt as a desired product are shown below, together with the structural formula of the benzenesulfonic acid magnesium chloride salt.

Measurement results of ¹H-NMR of benzenesulfonic acid:
¹H-NMR (400 MHz, DMSO-d6) δ (ppm): 13.52 (s, 1H, OH), 7.35-7.40 (m, 3H, Ar), 7.62-7.68 (m, 2H, Ar).

Measurement results of ¹H-NMR of benzenesulfonic acid magnesium chloride salt: ¹H-NMR (400 MHz, DMSO-d6) δ (ppm): 7.25-7.40 (m, 3H, Ar), 7.55-7.65 (m, 2H, Ar).

From the measurement results of ¹H-NMR, it was confirmed that the obtained benzenesulfonic acid magnesium chloride salt has a structure different from that of benzenesulfonic acid.

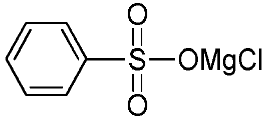

### (2) Preparation of Electrolyte Solution

In an argon gas atmosphere, 1.08 g (3.75 mmol) of the benzenesulfonic acid magnesium chloride salt (C₆H₅S(O)₂OMgCl) obtained in (1) was added to 10 mL of triethylene glycol dimethyl ether (manufactured by FUJIFILM Wako Pure Chemical Corporation), and then 1.00 g (7.50 mmol) of aluminum chloride (AlCl₃) (manufactured by FUJIFILM Wako Pure Chemical Corporation) was added thereto. A solution thus obtained was stirred at 80°C for 4 hours and then cooled to room temperature to obtain an electrolyte solution 6 [benzenesulfonic acid magnesium chloride salt-aluminum chloride / triethylene glycol dimethyl ether solution].

### Example 7: Preparation of Electrolyte Solution 7

### (1) Synthesis of Magnesium Salt

In an argon gas atmosphere, 3.68 g (30.07 mmol) of ethanesulfonic acid (manufactured by Tokyo Chemical Industry Co., Ltd.) was added to 45 mL of tetrahydrofuran (THF) (manufactured by FUJIFILM Wako Pure Chemical Corporation). 15 mL (30 mmol) of a THF solution of phenylmagnesium chloride (PhMgCl) having a concentration of 2 M (manufactured by Tokyo Chemical Industry Co., Ltd.) was added dropwise to the obtained THF solution at -60°C to -70°C, and stirred at -60°C for 30 minutes. After elevating the temperature of the reaction solution to room temperature, 40 mL of diisopropyl ether (manufactured by FUJIFILM Wako Pure Chemical Corporation) was added to a solid produced by concentrating the reaction solution under reduced pressure, and the mixture was stirred at room temperature for 1 hour. A white solid thus produced was collected by filtration and dried under reduced pressure at 80°C overnight to obtain an ethanesulfonic acid magnesium chloride salt (C₂H₅S(O)₂OMgCl).

The measurement results of ¹H-NMR of the ethanesulfonic acid as a raw material and the ethanesulfonic acid magnesium chloride salt as a desired product are shown below, together with the structural formula of the ethanesulfonic acid magnesium chloride salt.

Measurement results of ¹H-NMR of ethanesulfonic acid:
¹H-NMR (400 MHz, DMSO-d6) δ (ppm): 1.13 (t, J = 7.2 Hz, 3H, CH₃), 2.53 (q, J = 7.2 Hz, 2H, CH₂₎), 13.85 (br s, 1H, OH).

Measurement results of ¹H-NMR of ethanesulfonic acid magnesium chloride salt:
¹H-NMR (400 MHz, DMSO-d6) δ (ppm): 1.08 (t, J = 7.2 Hz, 3H, CH₃), 2.40 (q, J = 7.2 Hz, 2H, CH₂).

From the measurement results of ¹H-NMR, it was confirmed that the obtained ethanesulfonic acid magnesium chloride salt has a structure different from that of the ethanesulfonic acid.

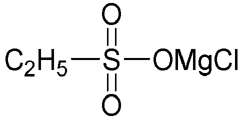

### (2) Preparation of Electrolyte Solution

In an argon gas atmosphere, 1.03 g (5.00 mmol) of the ethanesulfonic acid magnesium chloride salt (C₂H₅S(O)₂OMgCl) obtained in (1) was added to 20 mL of triethylene glycol dimethyl ether (manufactured by FUJIFILM Wako Pure Chemical Corporation), and then 1.33 g (10.00 mmol) of aluminum chloride (AlCl₃) (manufactured by FUJIFILM Wako Pure Chemical Corporation) was added thereto. A solution thus obtained was stirred at 80°C for 3 hours and then cooled to room temperature to obtain an electrolyte solution 7 [ethanesulfonic acid magnesium chloride salt-aluminum chloride / triethylene glycol dimethyl ether solution].

### Example 8: Preparation of Electrolyte Solution 8

### (1) Synthesis of Magnesium Salt

In an argon gas atmosphere, 2.85 g (14.99 mmol) of an ethanedisulfonic anhydride obtained by vacuum-drying an ethanedisulfonic acid dihydrate (manufactured by Tokyo Chemical Industry Co., Ltd.) was added to 45 mL of tetrahydrofuran (THF) (manufactured by FUJIFILM Wako Pure Chemical Corporation) at 145°C for 11 hours. 15 mL (30 mmol) of a THF solution of phenylmagnesium chloride (PhMgCl) having a concentration of 2 M (manufactured by Tokyo Chemical Industry Co., Ltd.) was added dropwise to the obtained THF solution at -60°C to -70°C for 10 minutes, and stirred at -60°C for 30 minutes. After elevating the temperature of the reaction solution to room temperature, 40 mL of diisopropyl ether (manufactured by FUJIFILM Wako Pure Chemical Corporation) was added to a solid produced by concentrating the reaction solution under reduced pressure, and the mixture was stirred at room temperature for 1 hour. The resulting white solid was collected by filtration and dried under reduced pressure at 80°C overnight to obtain an ethanedisulfonic acid magnesium chloride salt (ClMgO(O)₂SC₂H₄S(O)₂OMgCl).

The measurement results of ¹H-NMR of the ethanedisulfonic anhydride as a raw material and the ethanedisulfonic acid magnesium chloride salt as a desired product are shown below, together with the structural formula of the ethanesulfonic acid magnesium chloride salt.

¹H-NMR measurement results of ethanedisulfonic anhydride:
¹H-NMR (400 MHz, DMSO-d6) δ (ppm): 2.80 (s, 4H, CH₂), 14.44 (br s, 2H, OH).

¹H-NMR measurement results of ethanedisulfonic acid magnesium chloride salt:
¹H-NMR (400 MHz, DMSO-d6) δ (ppm): 2.77 (s, 4H, CH₂).

From the measurement results of ¹H-NMR, it was confirmed that the obtained ethanedisulfonic acid magnesium chloride salt has a structure different from that of ethanedisulfonic anhydride.

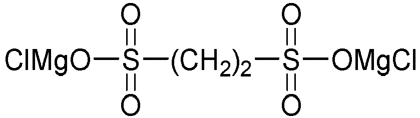

### (2) Preparation of Electrolyte Solution

In an argon gas atmosphere, 1.01 g (2.50 mmol) of the ethanedisulfonic acid magnesium chloride salt (ClMgO(O)₂SC₂H₄S(O)₂OMgCl) obtained in (1) was added to 10 mL of triethylene glycol dimethyl ether (manufactured by FUJIFILM Wako Pure Chemical Corporation), and then 1.33 g (10.00 mmol) of aluminum chloride (AlCl₃) (manufactured by FUJIFILM Wako Pure Chemical Corporation) was added thereto. A solution thus obtained was stirred at 80°C for 3 hours and then cooled to room temperature to obtain an electrolyte solution 8 [ethanedisulfonic acid magnesium chloride salt-aluminum chloride / triethylene glycol dimethyl ether solution].

### Comparative Example 1: Preparation of Comparative Electrolyte Solution 1

In an argon gas atmosphere, 0.93 g (10.30 mmol) of n-butanethiol (manufactured by FUJIFILM Wako Pure Chemical Corporation) was added to 5.15 mL of tetrahydrofuran (THF) (manufactured by FUJIFILM Wako Pure Chemical Corporation). 5.15 mL (10.30 mmol) of a THF solution of ethylmagnesium chloride (EtMgCl) having a concentration of 2 M (manufactured by Tokyo Chemical Industry Co., Ltd.) was added dropwise to the obtained THF solution at -60°C, and stirred at -50°C for 2 hours. After warming the reaction solution to room temperature, a solution obtained by dissolving 1.37 g (10.30 mmol) of aluminum chloride (AlCl₃) (manufactured by FUJIFILM Wako Pure Chemical Corporation) in 30.90 mL of tetrahydrofuran (THF) (manufactured by FUJIFILM Wako Pure Chemical Corporation) was added dropwise to the solution, and then the mixture was stirred at room temperature overnight to obtain a comparative electrolyte solution 1 [n-C₄H₉SMgCl-aluminum chloride / tetrahydrofuran solution].

### Comparative Example 2: Preparation of Comparative Electrolyte Solution 2

In an argon gas atmosphere, 0.98 g (10.90 mmol) of n-butanethiol (manufactured by FUJIFILM Wako Pure Chemical Corporation) was added to 5.45 mL of tetrahydrofuran (THF) (manufactured by FUJIFILM Wako Pure Chemical Corporation). 5.45 mL (10.90 mmol) of a THF solution of ethylmagnesium chloride (EtMgCl) having a concentration of 2 M (manufactured by Tokyo Chemical Industry Co., Ltd.) was added dropwise to the obtained THF solution at -60°C, and stirred at -50°C for 2 hours. After warming the reaction solution to room temperature, a solution obtained by dissolving 0.73 g (5.45 mmol) of aluminum chloride (AlCl₃) (manufactured by FUJIFILM Wako Pure Chemical Corporation) in 32.70 mL of tetrahydrofuran (THF) (manufactured by FUJIFILM Wako Pure Chemical Corporation) was added dropwise to the solution, and then the mixture was stirred at room temperature overnight to obtain a comparative electrolyte solution 2 [(n-C₄H₉SMgCl)₂-aluminum chloride / tetrahydrofuran solution].

### Comparative Example 3: Preparation of Comparative Electrolyte Solution 3

### (1) Synthesis of Magnesium Salt

In an argon gas atmosphere, 3.50 g (24.6 mmol) of benzenesulfinic acid (manufactured by Amel Pharmatech Corporation) was added to 37 mL of tetrahydrofuran (THF) (manufactured by FUJIFILM Wako Pure Chemical Corporation). 12.3 mL (24.6 mmol) of a THF solution of phenylmagnesium chloride (PhMgCl) having a concentration of 2 M (manufactured by Tokyo Chemical Industry Co., Ltd.) was added dropwise to the obtained diethyl ether solution at -70°C, and stirred at -20°C for 30 minutes. After elevating the temperature of the reaction solution to room temperature, 33 mL of diisopropyl ether (manufactured by FUJIFILM Wako Pure Chemical Corporation) was added to a solid produced by concentrating the reaction solution under reduced pressure, and the mixture was stirred at room temperature for 1 hour. A white solid thus produced was collected by filtration and dried under reduced pressure at 80°C overnight to obtain a benzenesulfinic acid magnesium chloride salt (C₆H₅S(O)OMgCl).

The measurement results of ¹H-NMR of the benzenesulfinic acid as a raw material and the benzenesulfinic acid magnesium chloride salt as a desired product are shown below, together with the structural formula of the benzenesulfinic acid magnesium chloride salt.

¹H-NMR measurement results of benzenesulfinic acid:
¹H-NMR (400 MHz, DMSO-d6) δ (ppm): 5.03 (br s, 1H, OH), 7.42-7.52 (m, 3H, Ar), 7.56-7.64 (M, 2H, Ar).

¹H-NMR measurement results of benzenesulfonic acid magnesium chloride salt: ¹H-NMR (400 MHz, DMSO-d6) δ (ppm): 7.25-7.45 (m, 3H, Ar), 7.50-7.65 (m, 2H, Ar).

From the measurement results of ¹H-NMR, it was confirmed that the obtained benzenesulfinic acid magnesium chloride salt has a structure different from that of benzenesulfinic acid.

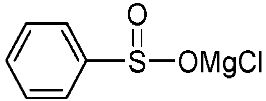

### (2) Preparation of Electrolyte Solution

In an argon gas atmosphere, 0.68 g (2.50 mmol) of the benzenesulfinic acid magnesium chloride salt (C₆H₅S(O)OMgCl) obtained in (1) was added to 10 mL of triethylene glycol dimethyl ether (manufactured by FUJIFILM Wako Pure Chemical Corporation), and then 0.33 g (2.50 mmol) of aluminum chloride (AlCl₃) (manufactured by FUJIFILM Wako Pure Chemical Corporation) was added thereto. A solution thus obtained was stirred at 80°C for 1 hour, cooled to room temperature, and then filtered to obtain a comparative electrolyte solution 3 [benzenesulfinic acid magnesium chloride salt-aluminum chloride / triethylene glycol dimethyl ether solution].

### Comparative Example 4: Preparation of Comparative Electrolyte Solution 4

In an argon gas atmosphere, 0.68 g (2.50 mmol) of the benzenesulfinic acid magnesium chloride salt (C₆H₅S(O)OMgCl) obtained in (1) of Comparative Example 3 was added to 10 mL of triethylene glycol dimethyl ether (manufactured by FUJIFILM Wako Pure Chemical Corporation), and then 0.67 g (5.00 mmol) of aluminum chloride (AlCl₃) (manufactured by FUJIFILM Wako Pure Chemical Corporation) was added thereto. A solution thus obtained was stirred at 80°C for 1 hour, cooled to room temperature, and then filtered to obtain a comparative electrolyte solution 4 [benzenesulfinic acid magnesium chloride salt-aluminum dichloride / triethylene glycol dimethyl ether solution].

### Comparative Example 5: Preparation of Comparative Electrolyte Solution 5

In an argon gas atmosphere, 1.52 g (10.00 mmol) of p-isopropylbenzenethiol (manufactured by FUJIFILM Wako Pure Chemical Corporation) was added to 5.00 mL of tetrahydrofuran (THF) (manufactured by FUJIFILM Wako Pure Chemical Corporation). 5.00 mL (10.00 mmol) of a THF solution of ethylmagnesium chloride (EtMgCl) having a concentration of 2 M (manufactured by Tokyo Chemical Industry Co., Ltd.) was added dropwise to the obtained THF solution at room temperature and the mixture was stirred overnight. A solution obtained by dissolving 0.88 g (6.67 mmol) of aluminum chloride (AlCl₃) (manufactured by FUJIFILM Wako Pure Chemical Corporation) in 10.00 mL of tetrahydrofuran (THF) (manufactured by FUJIFILM Wako Pure Chemical Corporation) was added dropwise to the reaction solution, and then the mixture was stirred at room temperature for 5 hours to obtain a comparative electrolyte solution 5 [p-i-C₃H₇-C₆H₄SMgCl-aluminum chloride / tetrahydrofuran solution].

### Evaluation Example 1: Cyclic Voltammetry (CV) Measurement of Various Electrolyte Solutions

Cyclic voltammetry (CV) measurement was carried out using the electrolyte solutions 1 to 8 and the comparative electrolyte solutions 1 to 5 immediately after the preparation thereof.

The CV measurement was carried out by the following method. That is, using a 3-electrode beaker cell, a platinum electrode (diameter: 3 mm; manufactured by BAS Co., Ltd.) was used as a working electrode, and magnesium rods (diameter: 1.6 mm; manufactured by The Nilaco Corporation) were used as a counter electrode and a reference electrode, respectively. 2 mL of the electrolyte solution was added to the beaker to carry out the measurement at room temperature (25°C) at a scan rate of 5 mV/s within the range of -1.5 to 3.5 V. An electrochemical measurement system (manufactured by BioLogic Co., Ltd.) was used for the measurement. In the measurement, the oxidative stability was determined based on a potential (oxidative decomposition potential) with respect to the magnesium reference electrode in which a current associated with the oxidative decomposition of the electrolyte solution was confirmed.

The results of the oxidative stability (10^{th} cycle) of each electrolyte solution are shown in Table 1.

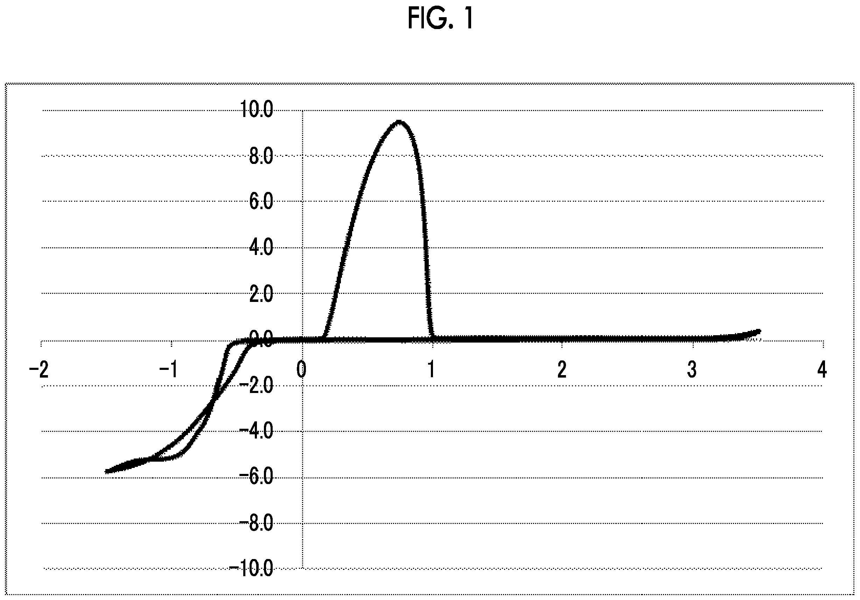

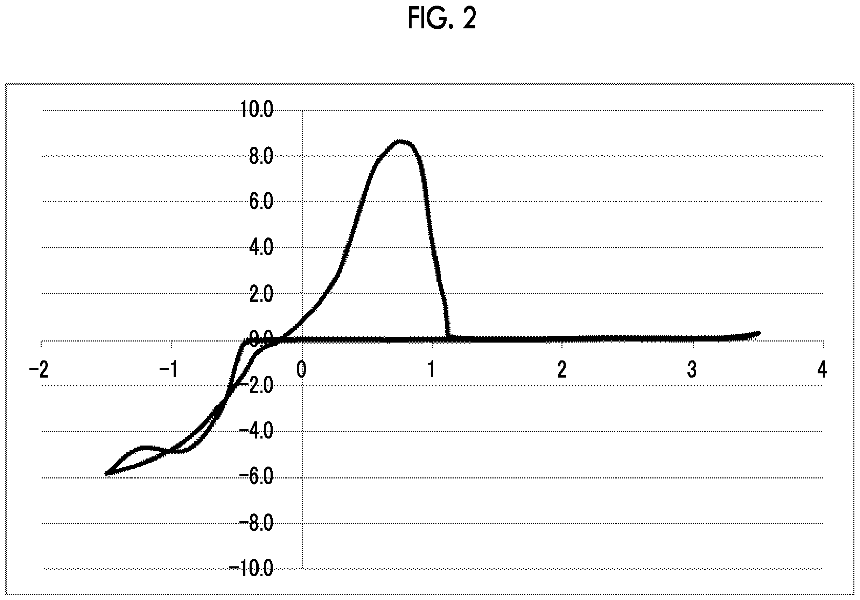

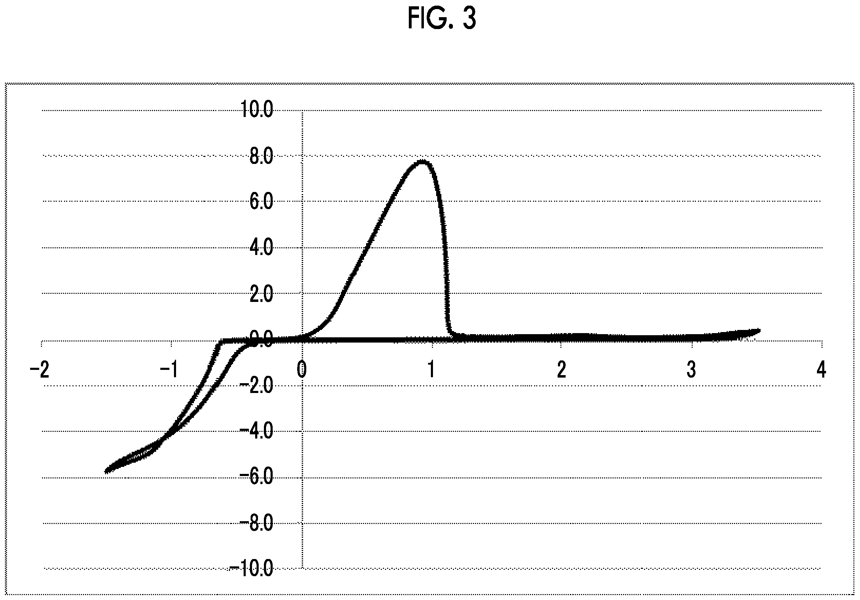

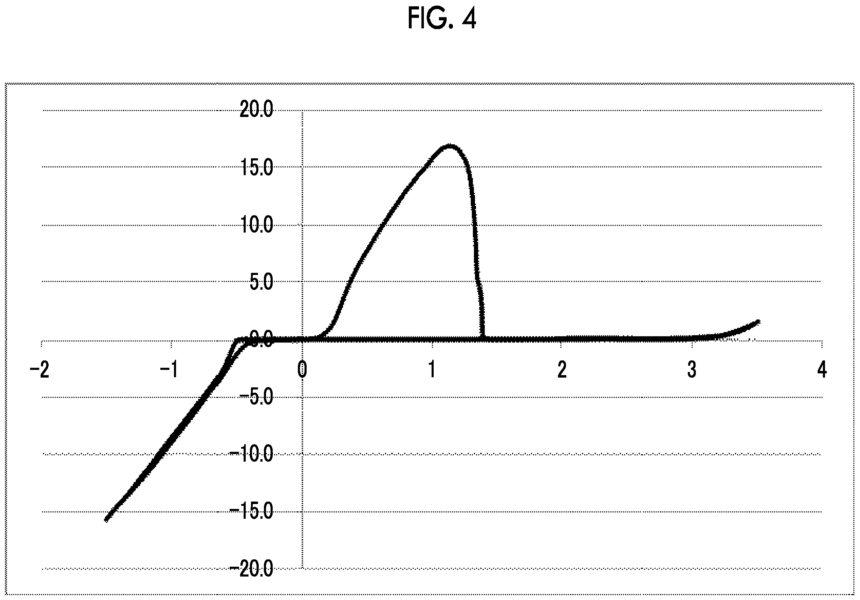

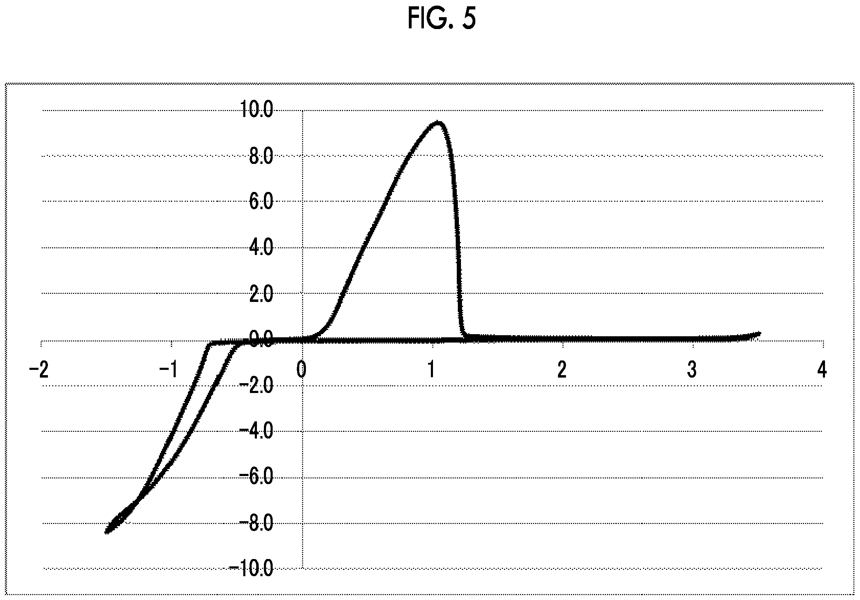

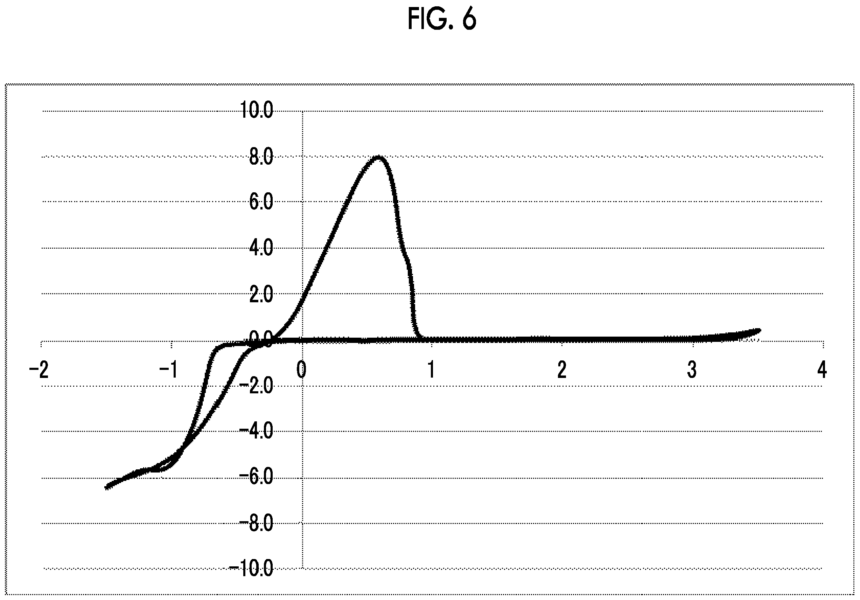

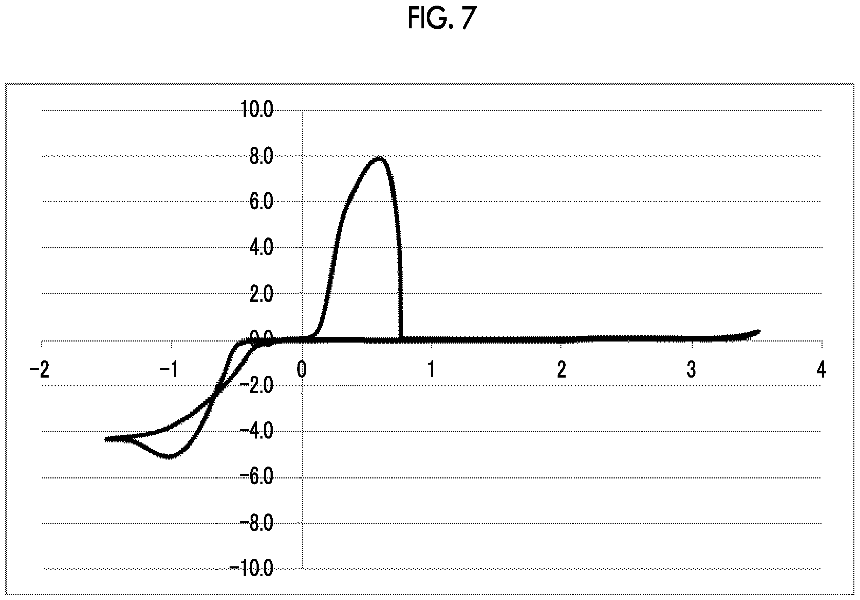

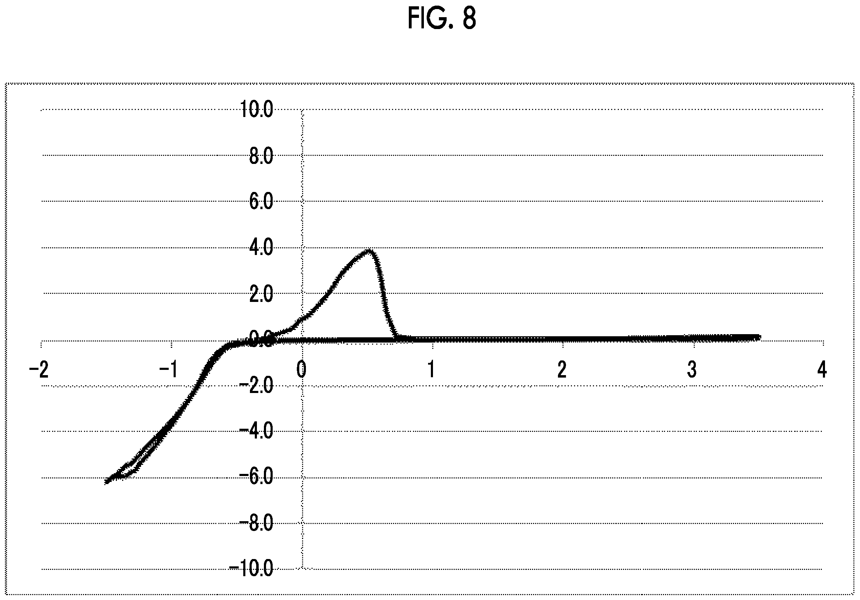

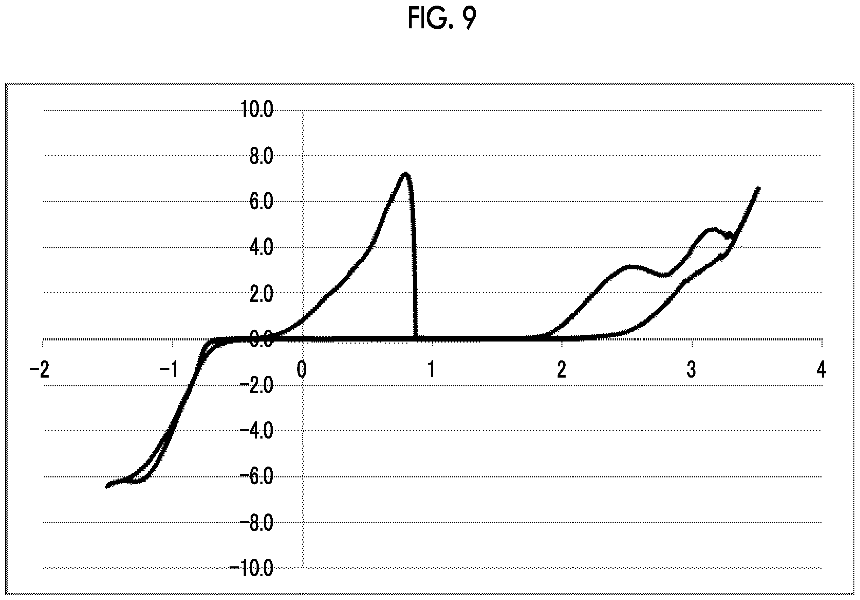

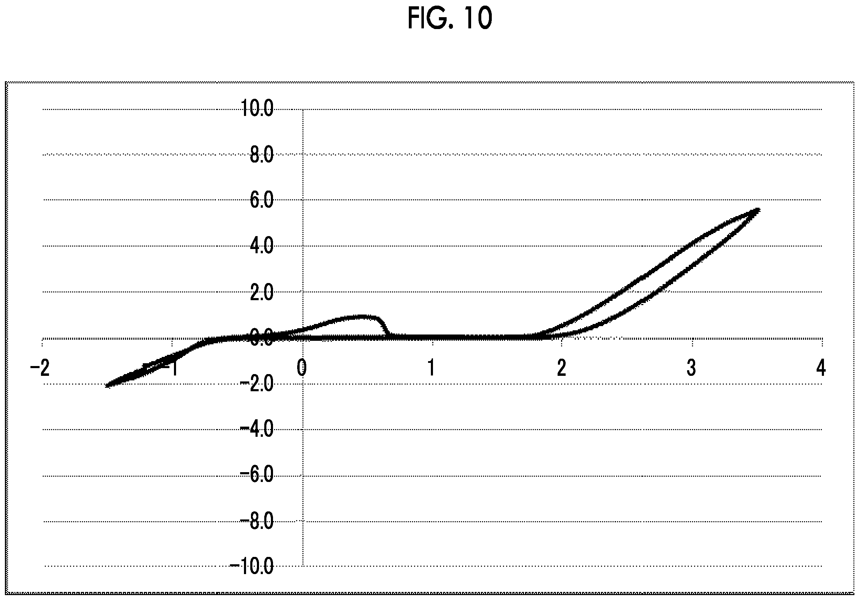

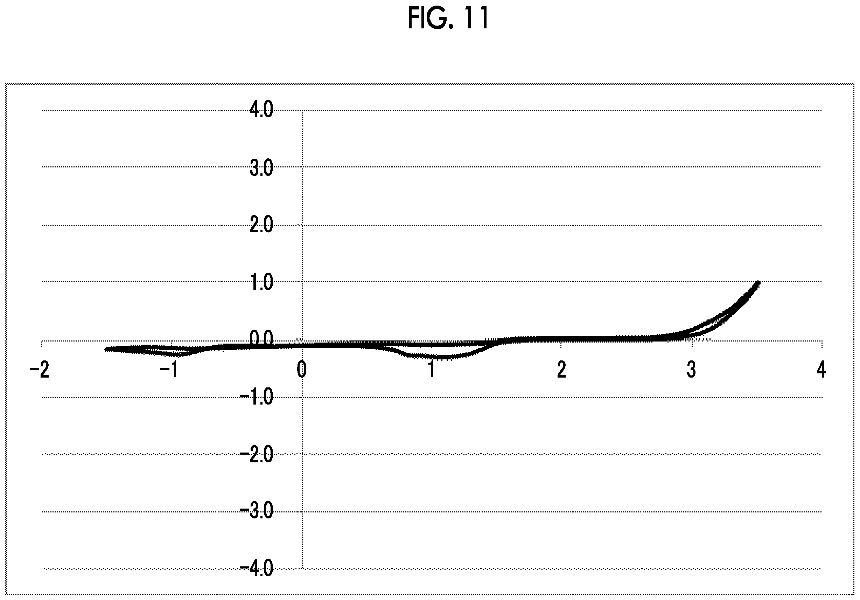

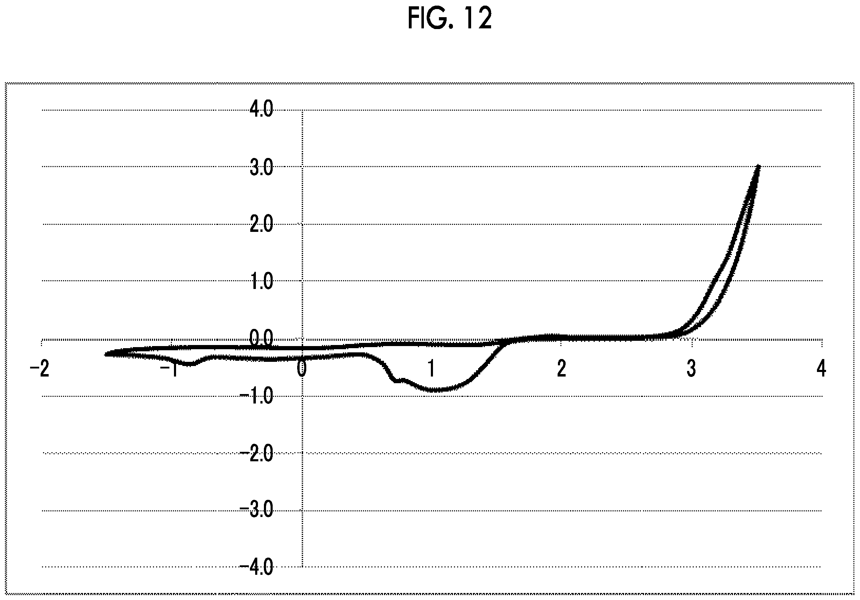

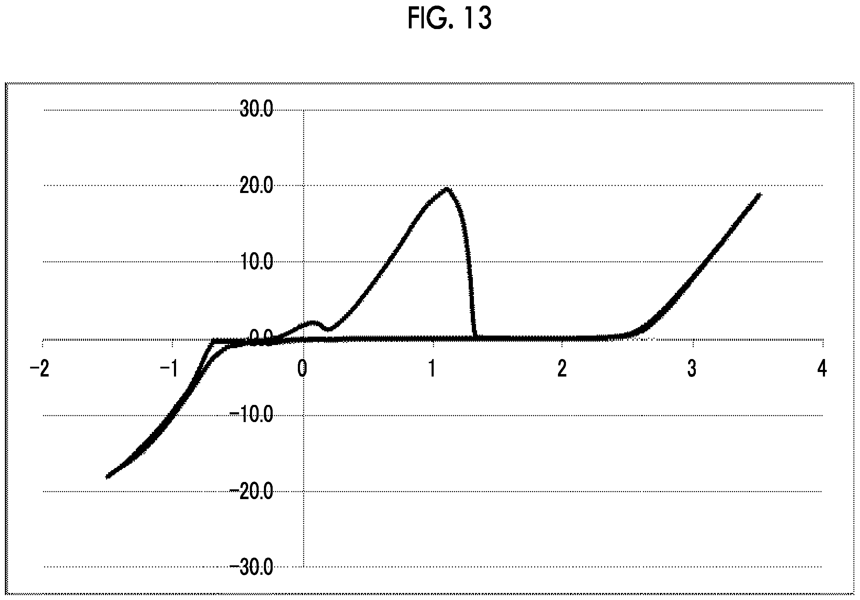

In addition, the results of the 10^{th} cycle of the respective electrolyte solutions are shown in Figs. 1 to 13 (Fig. 1: electrolyte solution 1, Fig. 2: electrolyte solution 2, Fig. 3: electrolyte solution 3, Fig. 4: electrolyte solution 4, Fig. 5: electrolyte solution 5, Fig. 6: electrolyte solution 6, Fig. 7: electrolyte solution 7, Fig. 8: electrolyte solution 8, Fig. 9: comparative electrolyte solution 1, Fig. 10: comparative electrolyte solution 2, Fig. 11: comparative electrolyte solution 3, Fig. 12: comparative electrolyte solution 4, and Fig. 13: comparative electrolyte solution 5). It should be noted that the horizontal axis in the drawings represents a potential of the working electrode with respect to the potential of the reference electrode, and the vertical axis (mA/cm²) represents a current density obtained by dividing a current value observed at each potential with a surface area of the working electrode.

**[Table 1]**

| | Electrolyte solution | Oxidative stability |
|---|---|---|
| Electrolyte solution 1 | CH₃S(O)₂OMgCl-AlCl₃/Triethylene glycol dimethyl ether | +3.2 V |
| Electrolyte solution 2 | CH₃S(O)₂OMgCl-AlCl₃/Triethylene glycol dimethyl ether-1,4-dioxane | +3.2 V |
| Electrolyte solution 3 | CF₃S(O)₂OMgCl-AlCl₃/Triethylene glycol dimethyl ether | +3.0 V |
| Electrolyte solution 4 | CF₃S(O)₂OMgCl-AlCl₃/THF | +3.0 V |
| Electrolyte solution 5 | n-C₄F₉S(O)₂OMgCl-AlCl₃/Triethylene glycol dimethyl ether | +3.2 V |
| Electrolyte solution 6 | C₆H₅S(O)₂OMgCl-AlCl₃/Triethylene glycol dimethyl ether | +3.0 V |
| Electrolyte solution 7 | C₂H₅S(O)₂OMgCl-AlCl₃/Triethylene glycol dimethyl ether | +3.1 V |
| Electrolyte solution 8 | ClMgO(O)₂SC₂H₄S(O)₂OMgCl-AlCl₃/Triethylene glycol dimethyl ether | +3.0 V |
| Comparative electrolyte solution 1 | n-C₄H₉SMgCl-AlCl₃/THF | +1.7 V |
| Comparative electrolyte solution 2 | (n-C₄H₉SMgCl)₂-AlCl₃/THF | +1.8 V |
| Comparative electrolyte solution 3 | C₆H₅S(O)OMgCl-AlCl₃/Triethylene glycol dimethyl ether | +2.8 V |
| Comparative electrolyte solution 4 | C₆H₅S(O)OMgCl-2AlCl₃/Triethylene glycol dimethyl ether | +2.8 V |
| Comparative electrolyte solution 5 | p-*i*-C₃H₇-C₆H₄SMgCl-AlCl₃/THF | +2.3 V |

From the results of Table 1 and Figs. 1 to 13, the oxidative stability of the electrolyte solutions of the present invention (electrolyte solutions 1 to 8) was +3.0 V or more with respect to the magnesium reference electrode. On the other hand, the oxidative stability of the electrolyte solutions formed of a magnesium salt of thiol (comparative electrolyte solutions 1, 2, and 5) and the electrolyte solutions formed of a magnesium salt of sulfinic acid (comparative electrolyte solutions 3 and 4) was +2.8 V or less with respect to the magnesium reference electrode.

From the above, it was found that the electrolyte solution of the embodiment of the present invention containing the compound represented by the general formula [1] has higher oxidative stability than the electrolyte solution formed of a magnesium salt of thiol and an electrolyte solution formed of a magnesium salt of sulfinic acid.

### Evaluation Example 2: Manufacture of Electrochemical Device and Charge-Discharge Test

A positive electrode was prepared by applying a paste which contain a mixture of Mo₆S₈ prepared according to the method described in the literature (J. Electrochem. Soc., 2014, 161, A593), Denka Black (manufactured by Denka Co., Ltd.) and polyvinylidene fluoride (PVDF) (manufactured by Kureha Co., Ltd.) at a weight ratio of 70:15:15, onto carbon paper (TGP-H-030; manufactured by Toray Industries, Inc.), and vacuum-drying at 80°C for 1 hour and at 120°C for 5 hours. Next, the positive electrode, a separator (GA-100; manufactured by ADVANTEC) and a negative electrode (AZ31, manufactured by Nakagawa Metal Co., Ltd.), processed to a diameter of 16 mm, were laminated in a CR2032 coin cell (SUS316L; manufactured by Hosen Co., Ltd.), and filled with the electrolyte solution 8 prepared in Example 8 to manufacture a magnesium secondary battery.

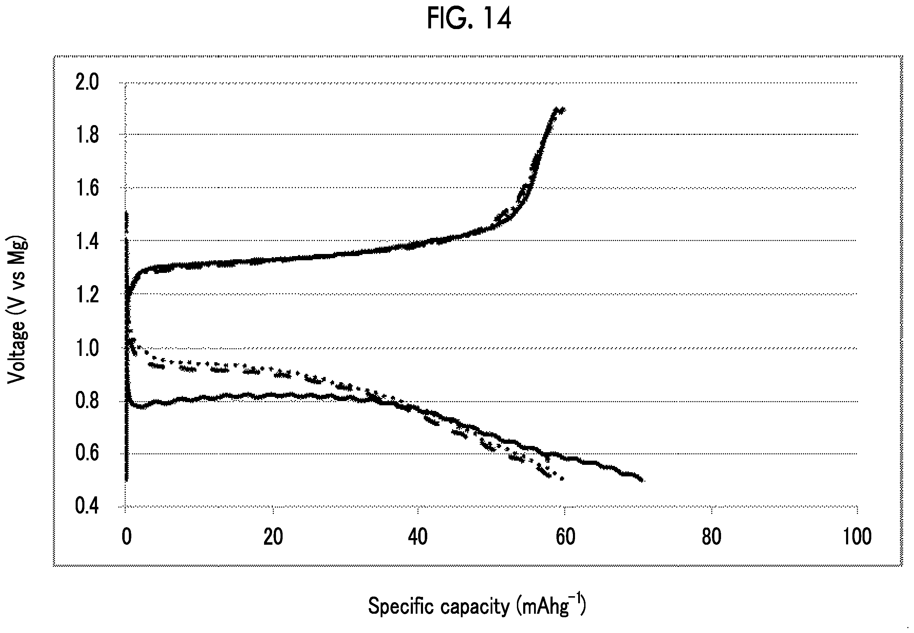

A constant current charge-discharge test was carried out at a cutoff potential of 0.5 to 1.9 V and a C/20 rate at room temperature, and a charge-discharge curve thus obtained is shown in Fig. 14. It should be noted that the horizontal axis (mAh/g) in the drawings represents a discharge capacity at each potential, and the vertical axis represents a potential of the working electrode based on a potential of the reference electrode. In addition, in Fig. 14, the solid line represents the result of the 1^{st} cycle, the wavy line represents the result of the 5^{th} cycle, and the dotted line represents the result of the 10^{th} cycle.

As clearly seen from the results of Fig. 14, it was found that a battery manufactured by using the electrolyte solution of the embodiment of the present invention operates as a secondary battery device.

### INDUSTRIAL APPLICABILITY

Since the electrolyte solution for a magnesium battery of the present invention has a high oxidative decomposition potential, it is useful as, for example, an electrolyte solution for a magnesium secondary battery.

## Claims

1. An electrolyte solution for a magnesium battery comprising a mixture of a compound represented by the general formula [1], a Lewis acid or a compound represented by the general formula [4], and a solvent: 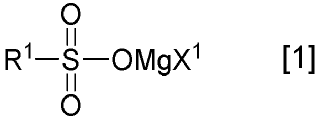 in the general formula [1], X¹ represents a halogeno group, and R¹ represents an alkyl group having 1 to 10 carbon atoms, which may have a group represented by - SO₃MgX² (X² represents a halogeno group); a haloalkyl group having 1 to 10 carbon atoms, which may have a group represented by -SO₃MgX² (X² is the same as described above); an aryl group having 6 to 14 carbon atoms, which may have an alkyl group having 1 to 6 carbon atoms, an alkoxy group having 1 to 6 carbon atoms, a halogeno group, or a group represented by -SO₃MgX² (X² is the same as described above); or a biphenyl group which may have a group represented by -SO₃MgX² (X² is the same as described above);
Mg [N(SO₂R⁴)₂]₂ [4]
in the general formula [4], four R⁴'s each independently represent an alkyl group having 1 to 6 carbon atoms, a perfluoroalkyl group having 1 to 6 carbon atoms, a phenyl group, or a perfluorophenyl group.

2. The electrolyte solution according to claim 1, wherein the electrolyte solution for a magnesium battery comprises a mixture of the compound represented by the general formula [1], the Lewis acid, and the solvent.

3. The electrolyte solution according to claim 1, wherein the compound represented by the general formula [1] is a compound represented by the general formula [2] or the general formula [3]: 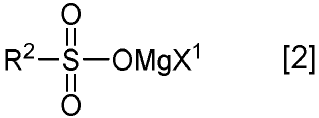 in the general formula [2], R² represents an alkyl group having 1 to 10 carbon atoms; a haloalkyl group having 1 to 10 carbon atoms; an aryl group having 6 to 14 carbon atoms, which may have an alkyl group having 1 to 6 carbon atoms, an alkoxy group having 1 to 6 carbon atoms, or a halogeno group; or a biphenyl group, and X¹ is the same as described above; 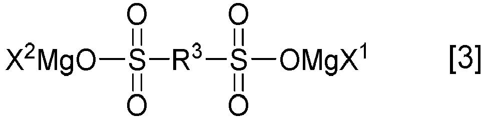 in the general formula [3], R³ represents an alkylene group having 1 to 10 carbon atoms; a haloalkylene group having 1 to 10 carbon atoms; an arylene group having 6 to 14 carbon atoms, which may have an alkyl group having 1 to 6 carbon atoms, an alkoxy group having 1 to 6 carbon atoms, or a halogeno group; or a biphenylene group, and X¹ and X² are each the same as described above.

4. The electrolyte solution according to claim 1, wherein the compound represented by the general formula [1] is a compound represented by the general formula [2']: 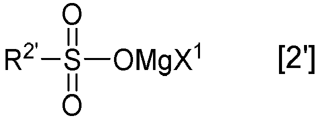 in the general formula [2'], R^{2'} represents an alkyl group having 1 to 10 carbon atoms; a fluoroalkyl group having 1 to 10 carbon atoms; an aryl group having 6 to 14 carbon atoms, which may have an alkyl group having 1 to 6 carbon atoms, an alkoxy group having 1 to 6 carbon atoms, or a fluoro group; or a biphenyl group, and X¹ is the same as described above.

5. The electrolyte solution according to claim 1, wherein the Lewis acid is a Lewis acid containing beryllium, boron, aluminum, silicon, titanium, chromium, iron, cobalt, or tin as an element.

6. The electrolyte solution according to claim 1, wherein the Lewis acid is a Lewis acid containing aluminum as an element.

7. The electrolyte solution according to claim 1, wherein the Lewis acid is aluminum chloride.

8. The electrolyte solution according to claim 1, wherein X¹ and X² in the compound represented by the general formula [1] are each a chloro group or a bromo group.

9. The electrolyte solution according to claim 1, wherein X¹ and X² in the compound represented by the general formula [1] are a chloro group.

10. The electrolyte solution according to claim 1, wherein the solvent is an ether-based solvent, a carbonate-based solvent, a halogenated hydrocarbon-based solvent, a nitrile-based solvent, or a sulfone-based solvent.

11. The electrolyte solution according to claim 1, wherein the solvent is an ether-based solvent.

12. An electrochemical device comprising: the electrolyte solution according to claim 1; a positive electrode; and a negative electrode.

13. A compound represented by the general formula [1]: 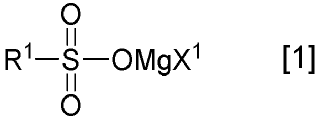 in the general formula [1], X¹ represents a halogeno group, and R¹ represents an alkyl group having 1 to 10 carbon atoms, which may have a group represented by - SO₃MgX² (X² represents a halogeno group); a haloalkyl group having 1 to 10 carbon atoms, which may have a group represented by -SO₃MgX² (X² is the same as described above); an aryl group having 6 to 14 carbon atoms, which may have an alkyl group having 1 to 6 carbon atoms, an alkoxy group having 1 to 6 carbon atoms, a halogeno group, or a group represented by -SO₃MgX² (X² is the same as described above); or a biphenyl group which may have a group represented by -SO₃MgX² (X² is the same as described above).

14. The compound according to claim 13, wherein the compound represented by the general formula [1] is a compound represented by the general formula [2] or the general formula [3]: 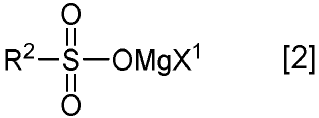 in the general formula [2], R² represents an alkyl group having 1 to 10 carbon atoms; a haloalkyl group having 1 to 10 carbon atoms; an aryl group having 6 to 14 carbon atoms, which may have an alkyl group having 1 to 6 carbon atoms, an alkoxy group having 1 to 6 carbon atoms, or a halogeno group; or a biphenyl group, and X¹ is the same as described above; 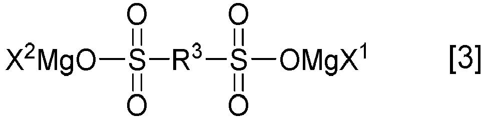 in the general formula [3], R³ represents an alkylene group having 1 to 10 carbon atoms; a haloalkylene group having 1 to 10 carbon atoms; an arylene group having 6 to 14 carbon atoms, which may have an alkyl group having 1 to 6 carbon atoms, an alkoxy group having 1 to 6 carbon atoms, or a halogeno group; or a biphenylene group, and X¹ and X² are each the same as described above.

15. The compound according to claim 13, wherein the compound represented by the general formula [1] is a compound represented by the general formula [2']: 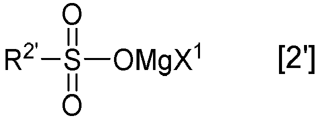 in the general formula [2'], R^{2'} represents an alkyl group having 1 to 10 carbon atoms; a fluoroalkyl group having 1 to 10 carbon atoms; an aryl group having 6 to 14 carbon atoms, which may have an alkyl group having 1 to 6 carbon atoms, an alkoxy group having 1 to 6 carbon atoms, or a fluoro group; or a biphenyl group, and X¹ is the same as described above.
